# EUROPÄISCHE PATENTSCHRIFT

(11) **EP 0 888 334 B1**
(45) Veröffentlichungstag und Bekanntmachung des Hinweises auf die Patenterteilung: **31.07.2002**
(21) Anmeldenummer: 97906099.3
(22) Anmeldetag: 20.02.1997
(51) Int. Cl.: C07D 339/08, C07D 327/06, A01N 43/32

(54) **2-HETAROYLCYCLOHEXAN-1,3-DIONE**
2-HETAROYLCYCLOHEXANE-1,3-DIONES
2-HETAROYLCYCLOHEXANE-1,3-DIONES

(30) Priorität: 24.02.1996 DE 19607105; 29.03.1996 DE 19612687
(43) Veröffentlichungstag der Anmeldung: 07.01.1999
(73) Patentinhaber: BASF AKTIENGESELLSCHAFT, 67056 Ludwigshafen (DE)
(72) Erfinder: OTTEN, Martina, D-67069 Ludwigshafen (DE); VON DEYN, Wolfgang, D-67435 Neustadt (DE); ENGEL, Stefan, D-65510 Idstein (DE); HILL, Regina, Luise, D-67346 Speyer (DE); KARDORFF, Uwe, D-68159 Mannheim (DE); VOSSEN, Marcus, D-68199 Mannheim (DE); PLATH, Peter, D-67227 Frankenthal (DE); WALTER, Helmut, D-67283 Obrigheim (DE); WESTPHALEN, Karl-Otto, D-67346 Speyer (DE); MISSLITZ, Ulf, D-67433 Neustadt (DE)
(86) Internationale Anmeldenummer: EP9700802
(87) Internationale Veröffentlichungsnummer: WO9730986

(56) Entgegenhaltungen:
- US-A- 5 480 858
- CHEMICAL ABSTRACTS, vol. 126, no. 2, 1997 Columbus, Ohio, US; abstract no. 18883v, Seite 542; XP002031682 & JP 08 245 618 A (NIPPON SODA) 24.September 1996

## Beschreibung

Die vorliegende Erfindung betrifft neue Hetaroylderivate mit herbizider Wirkung, Verfahren zur Herstellung der Hetaroylderivate, Mittel, welche diese enthalten sowie die Verwendung dieser Derivate oder diese enthaltende Mittel zur Unkrautbekämpfung.

Aus der Literatur sind herbizidwirksame 2-Hetaroylcylohexadione bekannt, beispielsweise aus WO 948988, WO 9404524 und EP 283261.

Die herbiziden Eigenschaften der bekannten Verbindungen sowie die Verträglichkeit gegenüber Kulturpflanzen können jedoch nur bedingt befriedigen.

Die Aufgabe der vorliegenden Erfindung bestand darin, neue 2-Hetaroylcyclohexandione mit verbesserten Eigenschaften zu finden.

Es wurden Hetaroylderivate der Formel I gefunden, 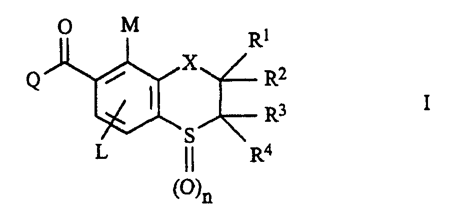 in der die Substituenten folgende Bedeutung haben:
- L, M: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
- X: Sauerstoff oder Schwefel, der mit einem oder zwei Sauerstoffen substituiert sein kann;
- n: null, eins, zwei;
- R¹: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl;
- R²: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl; R² und R³ können eine Bindung bilden;
- R³: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl; R³ und R² können eine Bindung bilden;
- R⁴: Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl;
- Q: ein in 2-Stellung verknüpfter Cyclohexan-1,3-dionring der Formel II 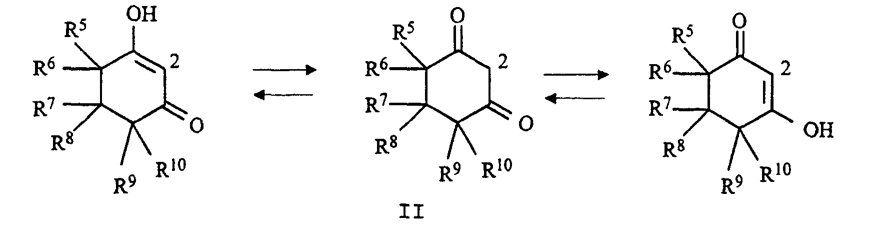
in welcher die Substituenten folgende Bedeutung haben:
- R⁵, R⁶ und R¹⁰: Wasserstoff, C₁-C₄-Alkyl;
- R⁷: Wasserstoff, C₁-C₄-Alkyl, C₃-C₄-Cycloalkyl, wobei diese Gruppen gegebenenfalls einen bis drei der folgenden Substituenten tragen können: Halogen, C₁-C₄-Thioalkyl oder C₁-C₄-Alkoxy; oder
C₁-C₄-Alkoxy; C₁-C₆-Alkoxyalkyl, Tetrahydropyranyl-3, Tetrahydropyranyl-4; oder
- R⁷ und R⁹: können gemeinsam eine Bindung oder einen drei bis sechsgliedrigen carbocyclischen Ring bilden;
- R⁸: Wasserstoff, C₁-C₄-Alkyl;
- R⁹: Wasserstoff, C₁-C₄-Alkyl oder eine Gruppe COOR¹¹;
- R¹¹: C₁-C₄-Alkyl;
sowie landwirtschaftlich brauchbare Salze.

Verbindungen der Formel I erhält man dadurch, daß man Verbindungen der Formel II mit einem Benzoesäurederivat der Formel III umsetzt und zu Hetaroylderivaten der Formel I umlagert.

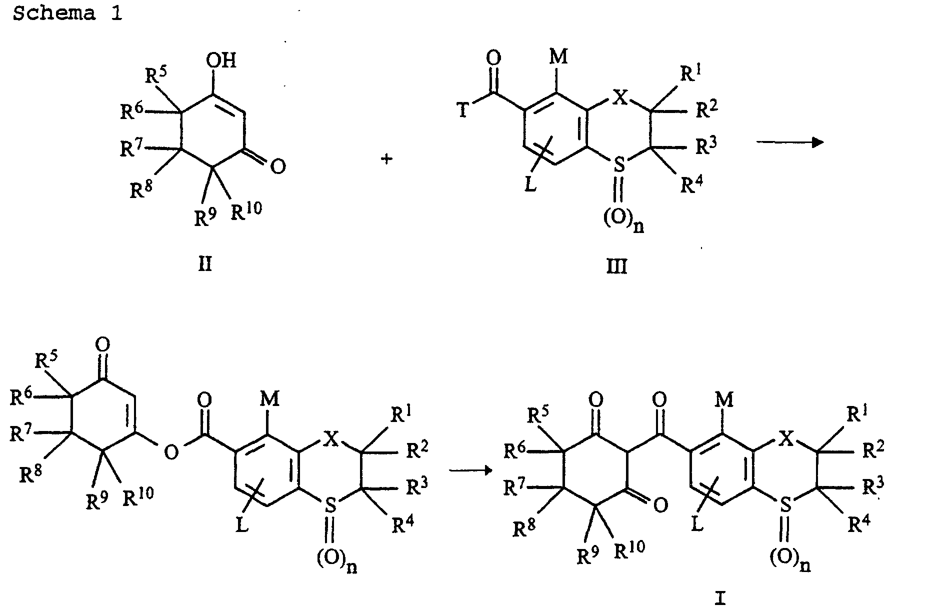

In dem obigen Schema 1 hat T in den genannten Formeln die Bedeutung Halogen oder OH und L, M, X, R¹, R², R³, R⁴ und n die oben angegebene Bedeutung.

Der erste Schritt der Reaktionsabfolge, die Acylierung, erfolgt in allgemein bekannter Weise z.B. durch Zugabe eines Säurechlorids der Formel III (T=Cl) oder einer z.B. mit DCC (Dicyclocarbodiimide) oder ähnlichen literaturbekannten Mitteln z.B. Triphenylphosphin/DEAD = Diethylazodicarboxylat, 2-Pyridindisulfid/Triphenylphosphin aktivierten Carbonsäure III (T=OH) zur Lösung oder Suspension eines Cyclohexandions II gegebenenfalls in Gegenwart einer Hilfsbase. Die Reaktanden und die Hilfsbase werden dabei zweckmäßig in äquimolaren Mengen eingesetzt. Ein geringer Überschuß, z.B. 1,2 bis 1,5 Moläquivalente, bezogen auf II, der Hilfsbase kann u.U. vorteilhaft sein.

Als Hilfsbase eignen sich tertiäre Alkylamine, Pyridin oder Alkalicarbonate. Als Lösungsmittel können z.B. Methylenchlorid, Dioxan, Diethylether, Toluol, Acetonitril oder Essigsäureethylester verwendet werden.

Während der Zugabe des Säurechlorids wird die Reaktionsmischung vorzugsweise auf 0 bis 10°C gekühlt, danach wird bei einer Temperatur von 20 bis 100°C, insbesondere 25 bis 50°C gerührt, bis die Umsetzung beendet ist. Die Aufarbeitung erfolgt in üblicher Weise, z.B. wird das Reaktionsgemisch in Wasser gegossen und das Wertprodukt extrahiert, z.B. mit Methylenchlorid. Nach Trocknen der organischen Phase und Entfernung des Lösungsmittels kann der rohe Enolester ohne weitere Reinigung zur Umlagerung eingesetzt werden. Herstellungsbeispiele für Benzoylenolester von Cyclohexan-1,3-dionen findet man z.B. EP-A 186118 oder US 4 780 127.

Die Umlagerung der Enolester zu den Verbindungen der Formel Ia-Ie erfolgt zweckmäßig bei Temperaturen von 20 bis 40°C in einem Lösungsmittel und in Gegenwart einer Hilfsbase sowie mit Hilfe einer Cyanoverbindung als Katalysator.

Als Lösungsmittel kann z.B. Acetonitril, Methylenchlorid, 1,2-Dichlorethan, Essigsäureethylester oder Toluol verwendet werden. Bevorzugtes Lösungsmittel ist Acetonitril. Als Hilfsbase eignen sich tertiäre Alkylamine, Pyridin oder Alkalicarbonate, die vorzugsweise in äquimolarer Menge oder bis zu einem vierfachen Überschuß, bezogen auf den Benzoylenolester, eingesetzt werden. Bevorzugte Hilfsbase ist Triethylamin in doppelter Menge.

Als Katalysator eignen sich Kaliumcyanid, Acetoncyanhydrin und Trimethylsilylcyanid, vorzugsweise in einer Menge von 1 bis 50 Molprozent, bezogen auf den Enolester. Bevorzugt setzt man Acetoncyanhydrin zu, z.B. in der Menge von 5 bis 15, insbesondere 10 Molprozent. Beispiele zur cyanidkatalysierten Umlagerung von Enolestern findet man z.B. in EP-A 186118 oder US 4 780 127.

Die Aufarbeitung erfolgt in an sich bekannter Weise z. B. wird das Reaktionsgemisch mit verdünnten Mineralsäuren wie 5 %iger Salzsäure oder Schwefelsäure angesäuert und mit einem organischen Lösungsmittel wie Methylenchlorid oder Essigsäureethylester extrahiert. Zur Reinigung wird der Extrakt mit kalter 5 bis 10 %iger Alkalicarbonatlösung extrahiert, wobei das Endprodukt in die wäßrige Phase übergeht. Durch Ansäuern der wäßrigen Lösung wird das Produkt der Formel Ia-Ie ausgefällt oder erneut mit Methylenchlorid oder Essigsäureethylester extrahiert, getrocknet und anschließend vom Lösungsmittel befreit.

Die als Ausgangsmaterial verwendeten 1,3-Diketone der Formel II sind bekannt und können nach an sich bekannten Verfahren hergestellt werden, wie sie beispielsweise in der EP-A 71707, EP-A 142741, EP-A 243313, US 4,249,937 und WO 92/13821 beschrieben sind. Cyclohexandion und Dimedon sind käufliche Verbindungen.

Benzoesäurederivate der Formel III lassen sich folgendermaßen herstellen:

Benzoylhalogenide wie beispielsweise Benzoylchloride der Formel III (T = C1) werden in an sich bekannter Weise durch Umsetzung der Benzoesäuren der Formel III (T = OH) mit Thionylchlorid hergestellt.

Die Benzoesäuren der Formel III (T = OH) können in bekannter Weise durch saure oder basische Hydrolyse aus den entsprechenden Estern der Formel III (T = C₁-C₄-Alkoxy) hergestellt werden.

Die Zwischenprodukte der Formel III lassen sich ausgehend von teilweise literaturbekannten Verbindungen wie substituierten Phenolcarbonsäuren IV oder Thiolcarbonsäuren V darstellen. Nicht bekannte Verbindungen IV oder V lassen sich durch Anwendung von literaturbekannten Reaktionen (Lit.: Houben Weyl, Methoden der Organischen Chemie Band VI, IX und E11) aufbauen.

Die weitere Umsetzung zu den Zwischenprodukten der Formel III läuft über literaturbekannte Verfahren (z.B. Synthesis 1975, 451; J. Org. Chem. 1974, 39-1811; J. Am. Chem. Soc. 1954, 76, 1068; Heterocyclic Compounds, Band: Multi-Sulfur and Sulfur and Oxygen Five an Six-Membered Heterocycles; J. Org. Chem. 1979, 44, 1977). 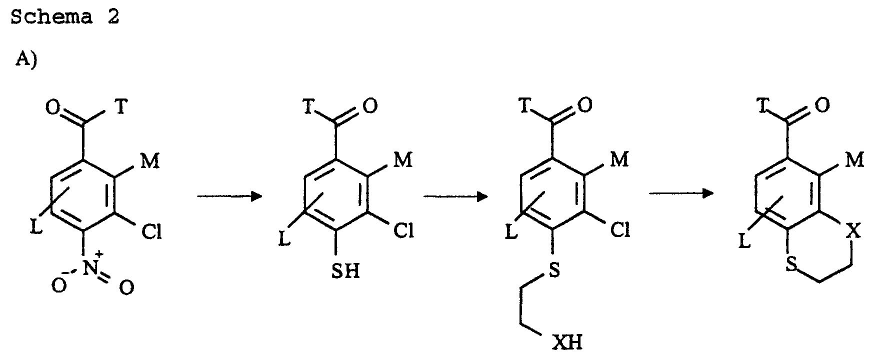 oder 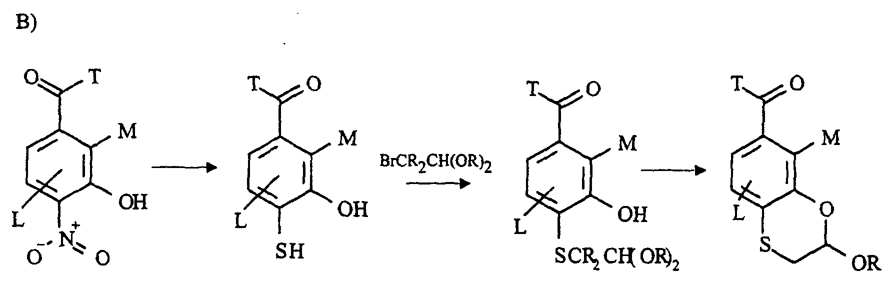 oder 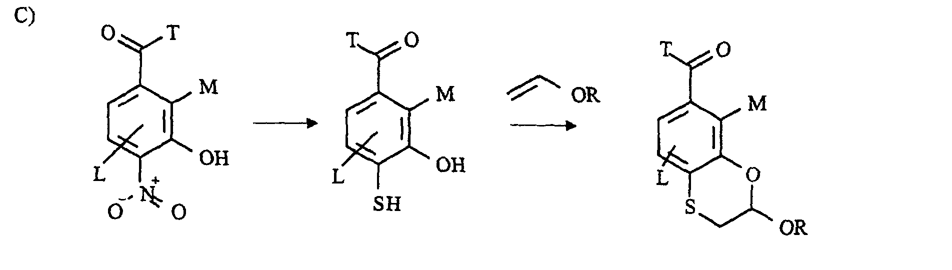 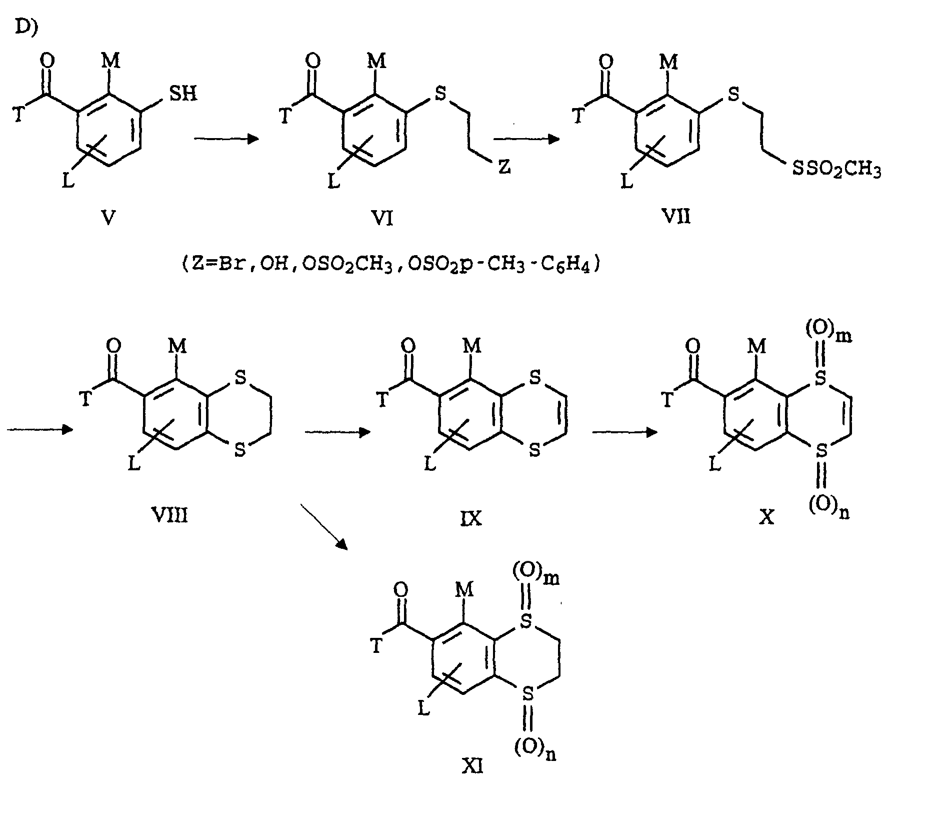 oder

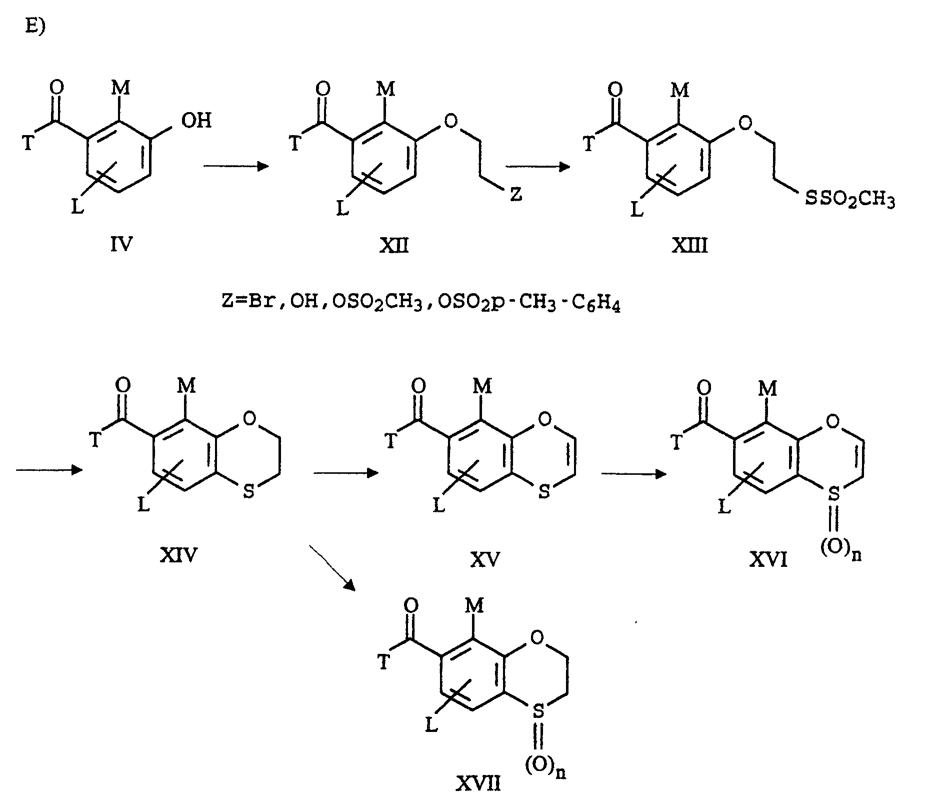

Danach lassen sich 2,3-Dihydrobenz-1,4-oxathiinderivate z.B. wie in Schema 2A dargestellt durch intramolekulare nucleophile Substitution am Aromaten aufbauen (X = O,S; T = OH, C₁-C₄-Alkoxy) (Lit: J. Heterocycl. Chem. 20, 867, 1983) Weg B und C beschreiben den in der Literatur angegebenen Weg zu 2-Alkoxy-2,3-dihydrobenz-1,4-oxathiinderivaten. (Lit: J. Am. Chem. Soc. 76, 1068, 1954; J. Org. Chem. 44, 1977, 1979) Wie in Schema 2 in D und E dargestellt, lassen sich Phenole oder Thiole mit Alkylbromiden in alkalischer Lösung alkylieren. Die Reaktanden und die Base werden dabei zweckmäßigerweise in äquimolaren Mengen eingesetzt. Ein Überschuß an Base kann von Vorteil sein. Als Lösungsmittel werden Alkohole wie Ethanol oder DMF und als Basen Alkoholate wie z.B. Natriumethanolat oder NaH bevorzugt. Die Reaktion kann unter Normaldruck oder bei erhöhtem Druck erfolgen. Bevorzugter Druckbereich ist 1 bis 10 bar. Die Reaktionsmischung wird vorzugsweise bei 20 - 150°C, insbesondere bei 60-80°C, gerührt. Die Aufarbeitung erfolgt beispielsweise so, daß das Reaktionsgemisch auf verdünnte Lauge wie Natronlauge gegossen wird und das Wertprodukt durch Extraktion mit z.B. Essigsäureethylester, getrocknet und vom Lösungsmittel befreit, gewonnen werden kann.

Anschließend kann z.B. ein Austausch von Z mit Kaliummethanthiosulfonat in alkoholischer Lösung erfolgen. Bevorzugte Lösungsmittel sind Ethanol, Methanol und Isopropanol. Die Reaktionsmischung wird dabei vorzugsweise bei 20 - 100°C, insbesondere bei 60 - 80°C, gerührt.

Die Aufarbeitung erfolgt beispielsweise durch Zugabe von Wasser, wobei das Wertprodukt abgesaugt wird oder durch Extraktion mit z.B. Methylenchlorid extrahiert und getrocknet wird.

Die Cyclisierung zum Dihydrobenzoxathiin- oder Dihydrobenzdithiingerüst erfolgt unter Zusatz einer Lewissäure in einem inerten Lösungsmittel. Dabei wird als Lewissäure Aluminiumtrichlorid und als inertes Lösungsmittel Nitromethan oder Methylenchlorid bevorzugt. Das Reaktionsgemisch wird bei einer Temperatur von 20-50°C gehalten. Die Aufarbeitung erfolgt beispielsweise durch Zugabe von verdünnter Mineralsäure wie Salzsäure und das Wertprodukt wird abgesaugt oder durch Extraktion mit Ether extrahiert, getrocknet und vom Lösungsmittel befreit.

Durch Oxidation nach literaturbekannten Methoden und/oder Dehydrierung (Houben Weyl Methoden der Organischen Synthese Band IV/1a und b ) können die Verbindungen weiter funktionalisiert werden.

Die Benzoesäuren der Formel III können auch erhalten werden, indem man die entsprechenden brom- oder iodsubstituierten Verbindungen der Formel XVIII 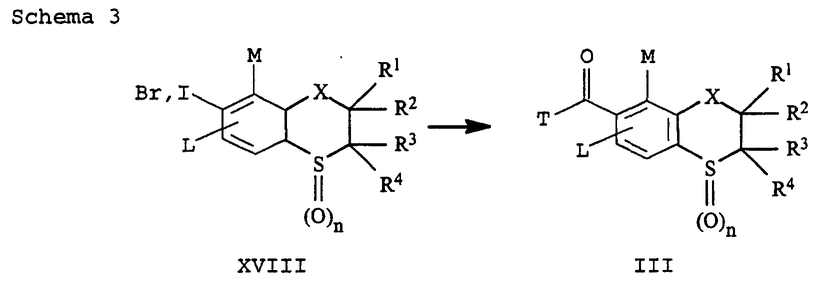 wobei
- T: OH, C₁-C₄-Alkoxy; und
- L, M, X, R¹ bis R⁴ und n: die oben beschriebene Bedeutung haben;
in Gegenwart eines Palladium-, Nickel-, Cobalt- oder Rhodium-Übergangsmetallkatalysators und einer Base mit Kohlenmonoxid und Wasser unter erhöhtem Druck umsetzt.

Die Katalysatoren Nickel, Cobalt, Rhodium und insbesondere Palladium können metallisch oder in Form üblicher Salze wie in Form von Halogenverbindungen, z.B. PdCl₂, RhCl₃·H₂O, Acetaten, z.B. Pd(OAc)₂, Cyaniden usw. in den bekannten Wertigkeitsstufen vorliegen. Ferner können Metallkomplexe mit tertiären Phosphinen, Metallalkylcarbonyle, Metallcarbonyle, z.B. CO₂(CO)₈, Ni(CO)₄, Metallcarbonyl-Komplexe mit tertiären Phosphinen, z.B. (PPh₃)₂Ni(CO)₂, oder mit tertiären Phosphinen komplexierte Übergangsmetallsalze vorliegen. Die letztgenannte Ausführungsform ist insbesondere im Fall von Palladium als Katalysator bevorzugt. Dabei ist die Art der Phosphinliganden breit variabel. Beispielsweise lassen sie sich durch folgende Formeln wiedergeben: 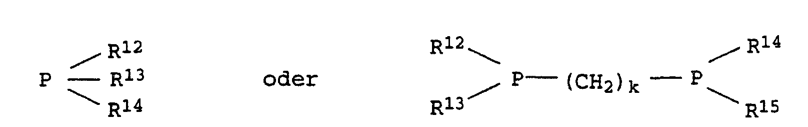 wobei k die Zahlen 1, 2, 3 oder 4 bedeutet und die Reste R¹² bis R¹⁵ für niedermolekulares Alkyl, z.B. C₁-C₆-Alkyl, Aryl, C₁-C₄-Alkylaryl, z.B. Benzyl, Phenethyl oder Aryloxy stehen. Aryl ist z.B. Naphthyl, Anthryl und vorzugsweise gegebenenfalls substituiertes Phenyl, wobei man hinsichtlich der Substituenten nur auf deren Inertheit gegenüber der Carboxylierungsreaktion zu achten hat, ansonsten können sie breit variiert werden und umfassen alle inerten C-organischen Reste wie C₁-C₆-Alkylreste, z.B. Methyl, Carboxylreste wie COOH, COOM (M ist z.B. ein Alkali-, Erdalkalimetall oder Ammoniumsalz), oder C-organische Reste über Sauerstoff gebunden wie C₁-C₆-Alkoxyreste.

Die Herstellung der Phosphinkomplexe kann in an sich bekannter Weise, z.B. wie in den eingangs genannten Dokumenten beschrieben, erfolgen. Beispielsweise geht man von üblichen kommerziell erwerblichen Metallsalzen wie PdCl₂ oder Pd(OCOCH₃)₂ aus und fügt das Phosphin z.B. P(C₆H₅)₃, P(n-C₄H₉)₃, PCH₃(C₆H₅)₂, 1,2-Bis(diphenylphosphino)ethan hinzu.

Die Menge an Phosphin, bezogen auf das Übergangsmetall, beträgt üblicherweise 0 bis 20, insbesondere 0,1 bis 10 Moläquivalente, besonders bevorzugt 1 bis 5 Moläquivalente.

Die Menge an Übergangsmetall ist nicht kritisch. Natürlich wird man aus Kostengründen eher eine geringe Menge, z.B. von 0,1 bis 10 Mol.-%, insbesondere 1 bis 5 Mol.-%, bezogen auf den Ausgangsstoff II bzw. III verwenden.

Zur Herstellung der Benzoesäuren III (T = OH) führt man die Umsetzung mit Kohlenmonoxid und mindestens äquimolaren Mengen an Wasser, bezogen auf die Ausgangsstoffe VI durch. Der Reaktionspartner Wasser kann gleichzeitig auch als Lösungsmittel dienen, d.h. die maximale Menge ist nicht kritisch.

Es kann aber auch je nach Art der Ausgangsstoffe und der verwendeten Katalysatoren von Vorteil sein, anstelle des Reaktionspartners ein anderes inertes Lösungsmittel oder die für die Carboxylierung verwendete Base als Lösungsmittel zu verwenden.

Als inerte Lösungsmittel kommen für Carboxylierungsreaktionen übliche Lösungsmittel wie Kohlenwasserstoffe, z.B. Toluol, Xylol, Hexan, Pentan, Cyclohexan, Ether z.B. Methyl-tert.butylether, Tetrahydrofuran, Dioxan, Dimethoxyethan, substituierte Amide wie Dimethylformamid, persubstituierte Harnstoffe wie Tetra-C₁-C₄-alkylharnstoffe oder Nitrile wie Benzonitril oder Acetonitril in Betracht.

In einer bevorzugten Ausführungsform des Verfahrens verwendet man einen der Reaktionspartner, insbesondere die Base, im Überschuß, so daß kein zusätzliches Lösungsmittel erforderlich ist.

Für das Verfahren geeignete Basen sind alle inerten Basen, die den bei der Umsetzung freiwerdenden Jodwasserstoff bzw. Bromwasserstoff zu binden vermögen. Beispielsweise sind hier tertiäre Amine wie tert.-Alkylamine, z.B. Trialkylamine wie Triethylamin, cyclische Amine wie N-Methylpiperidin oder N,N'-Dimethylpiperazin, Pyridin, Alkali- oder -hydrogencarbonate, oder tetraalkylsubstituierte Harnstoffderivate wie Tetra-C₁-C₄-alkylharnstoff, z.B. Tetramethylharnstoff, zu nennen.

Die Menge an Base ist nicht kritisch, üblicherweise werden 1 bis 10, insbesondere 1 bis 5 Mol verwendet. Bei gleichzeitiger Verwendung der Base als Lösungsmittel, wird die Menge in der Regel so bemessen, daß die Reaktionspartner gelöst sind, wobei man aus Praktikabilitätsgründen unnötig hohe Überschüsse vermeidet, um Kosten zu sparen, kleine Reaktionsgefäße einsetzen zu können und den Reaktionspartnern maximalen Kontakt zu gewährleisten.

Während der Umsetzung wird der Kohlenmonoxiddruck so eingestellt, daß immer ein Überschuß an CO, bezogen auf VI vorliegt. Vorzugsweise liegt der Kohlenmonoxiddruck bei Raumtemperatur bei 1 bis 250 bar, insbesondere 5 bis 150 bar CO.

Die Carbonylierung wird in der Regel bei Temperaturen von 20 bis 250°C, insbesondere bei 30 bis 150°C kontinuierlich oder diskontinuierlich durchgeführt. Bei diskontinuierlichem Betrieb wird zweckmäßigerweise zur Aufrechterhaltung eines konstanten Druckes kontinuierlich Kohlenmonoxid auf das Umsetzungsgemisch aufgepreßt.

Die als Ausgangsverbindungen benutzten Arylhalogenverbindungen XVIII sind bekannt oder können leicht durch geeignete Kombination bekannter Synthesen und nach oben beschriebenen Reaktionsfolgen hergestellt werden.

Im Hinblick auf die bestimmungsgemäße Verwendung der Benzoylderivate der allgemeinen Formel I kommen als Substituenten folgende Reste in Betracht:
L,M Wasserstoff,
C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl oder 1-Ethyl-2-methyl-propyl,
insbesondere Methyl, Ethyl, 1-Methylethyl, 1-Methylpropyl, 2-Methylpropyl, 1,1-Dimethylethyl und 1,1-Dimethylpropyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl,1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3 butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
insbesondere 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butenyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2 propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1, 1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
insbesondere C₁-C₃-Alkoxy wie Methoxy, Ethoxy, i-Propoxy,
wobei diese Gruppen gegebenenfalls durch ein bis fünf Halogenatome wie Fluor, Chlor, Brom und Iod, vorzugsweise Fluor und Chlor oder C₁-C₄-Alkoxy wie vorstehend genannt substituiert sein können.

Bevorzugt sind Hetaroylderivate der Formel Ia, 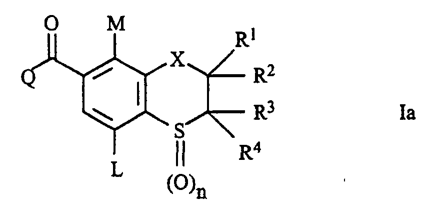 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, X, R¹ bis R⁴ und n die oben angegebenen Bedeutungen haben.

### Weiterhin bevorzugt sind Hetaroylderivate der Formel Ib

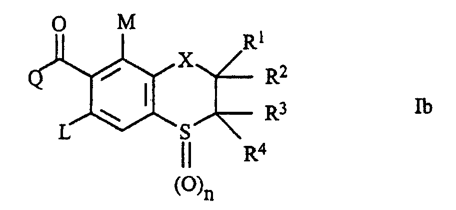

in der L für C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, X, R¹ bis R⁴ und n die oben angegebenen Bedeutungen haben.

Bevorzugt sind auch Hetaroylderivate der Formel I, in der die Reste L und M für Wasserstoff, Methyl, Methoxy, Chlor Cyano, Nitro und Trifluormethyl stehen.

### Weiterhin bevorzugt sind auch Hetaroylderivate der Formel Ic

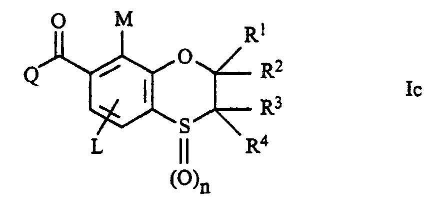

in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, R¹ bis R⁴ und n die oben angegebenen Bedeutungen haben.

Bevorzugt sind auch Hetaroylderivate der Formel Id 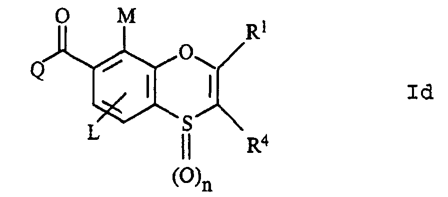 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, R¹, R⁴ und n die oben angegebenen Bedeutungen haben.

### Bevorzugt sind weiterhin Hetaroylderivate der Formel Ie

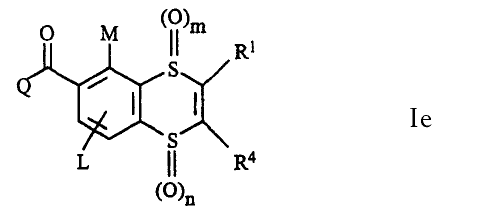

in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro sowie m für null, eins oder zwei steht und Q, R¹, R⁴ und n die oben angegebenen Bedeutungen haben.

### Auch bevorzugt sind Hetaroylderivate der Formel If

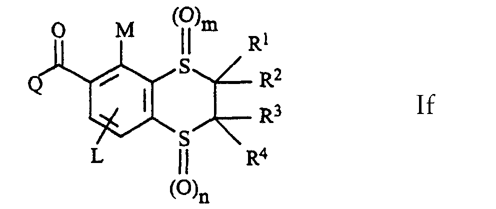

in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro sowie m für null, eins oder zwei steht und Q, R¹ bis R⁴ und n die oben angegebenen Bedeutungen haben.

Bevorzugt sind auch Hetaroylderivate der Formel I bis Id, deren Substituenten aus einer Kombination der bevorzugten Substituenten bestehen.

Besonders bevorzugte Verbindungen der Formel I sind in den nachfolgenden Tabellen 1-6 aufgeführt.

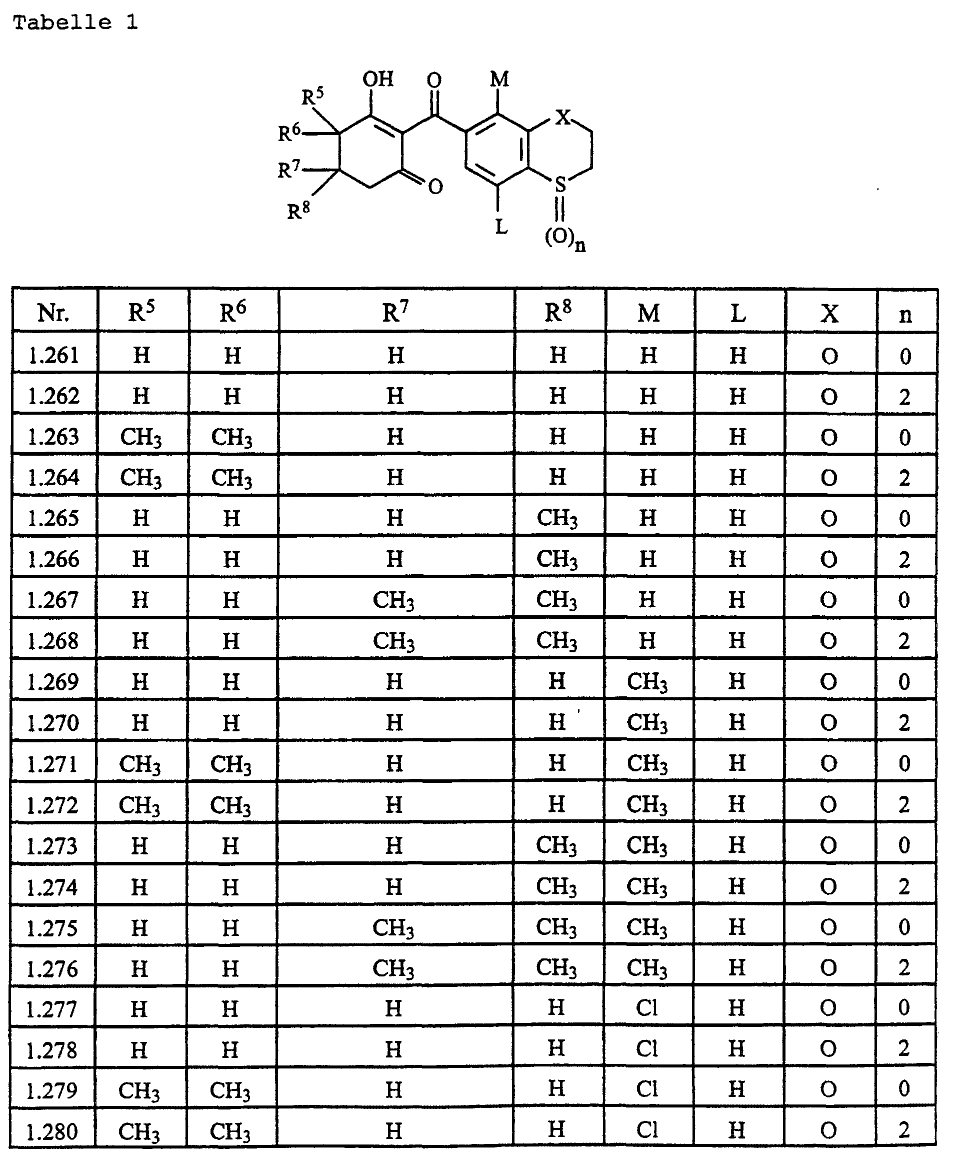

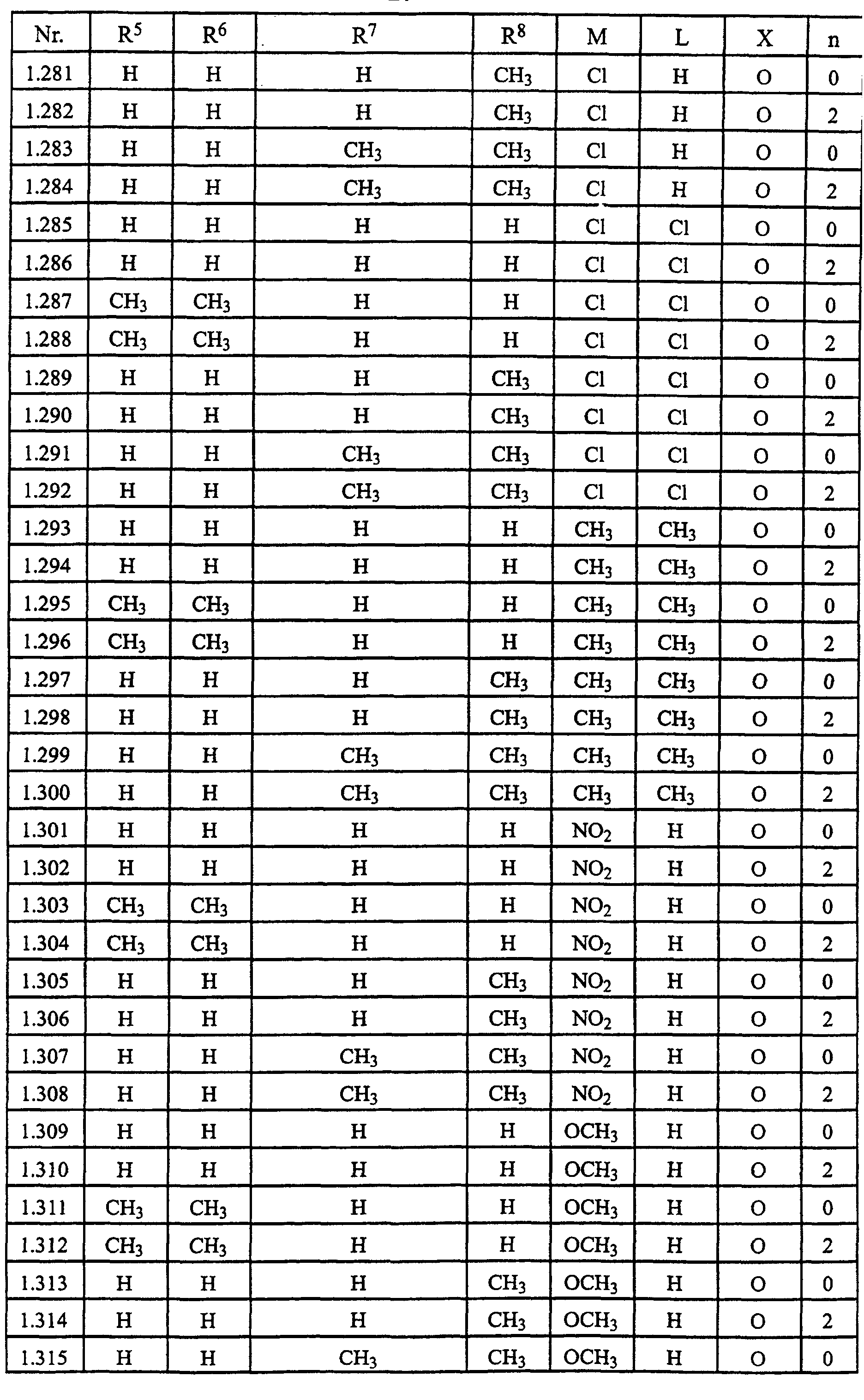

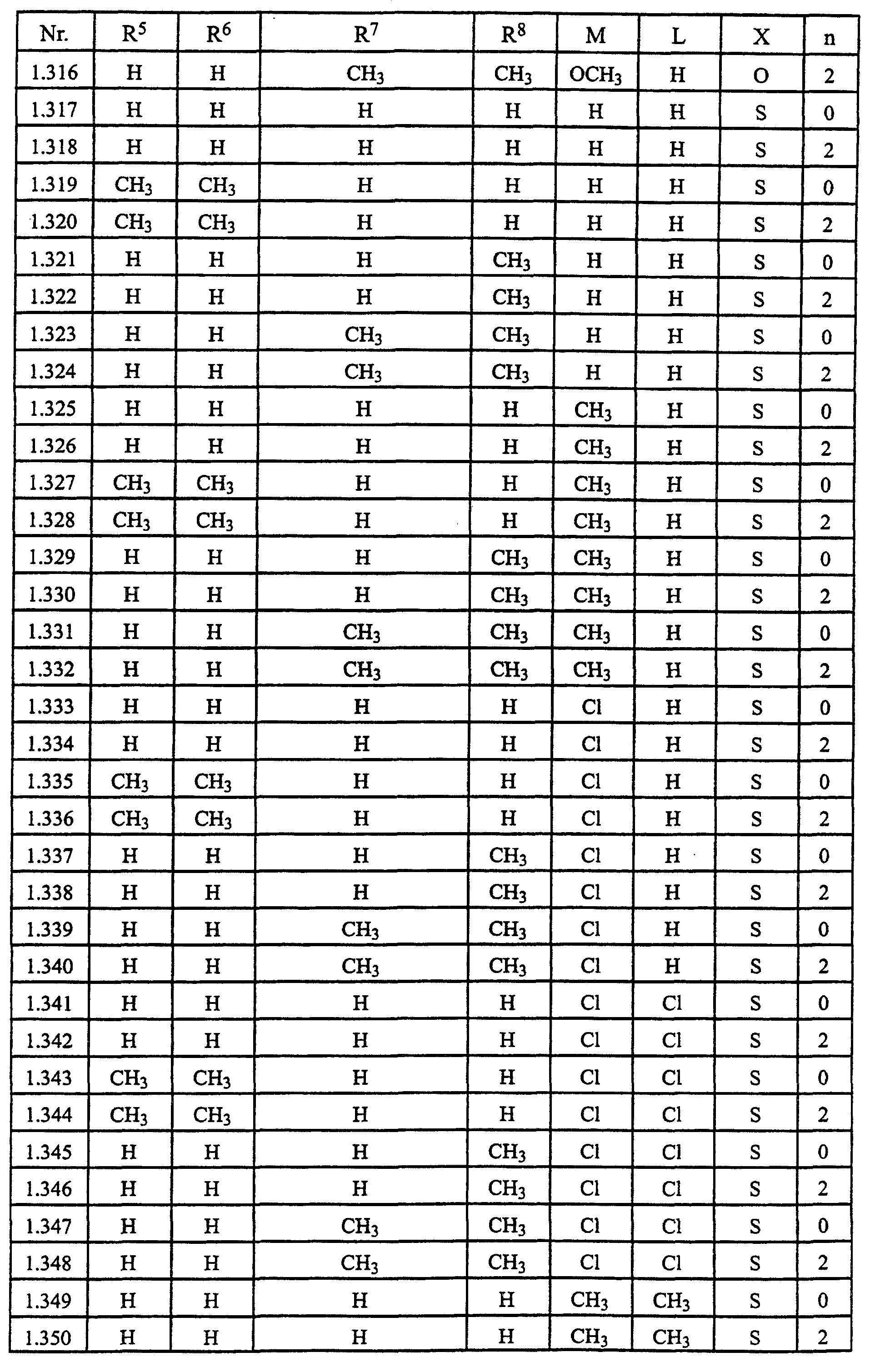

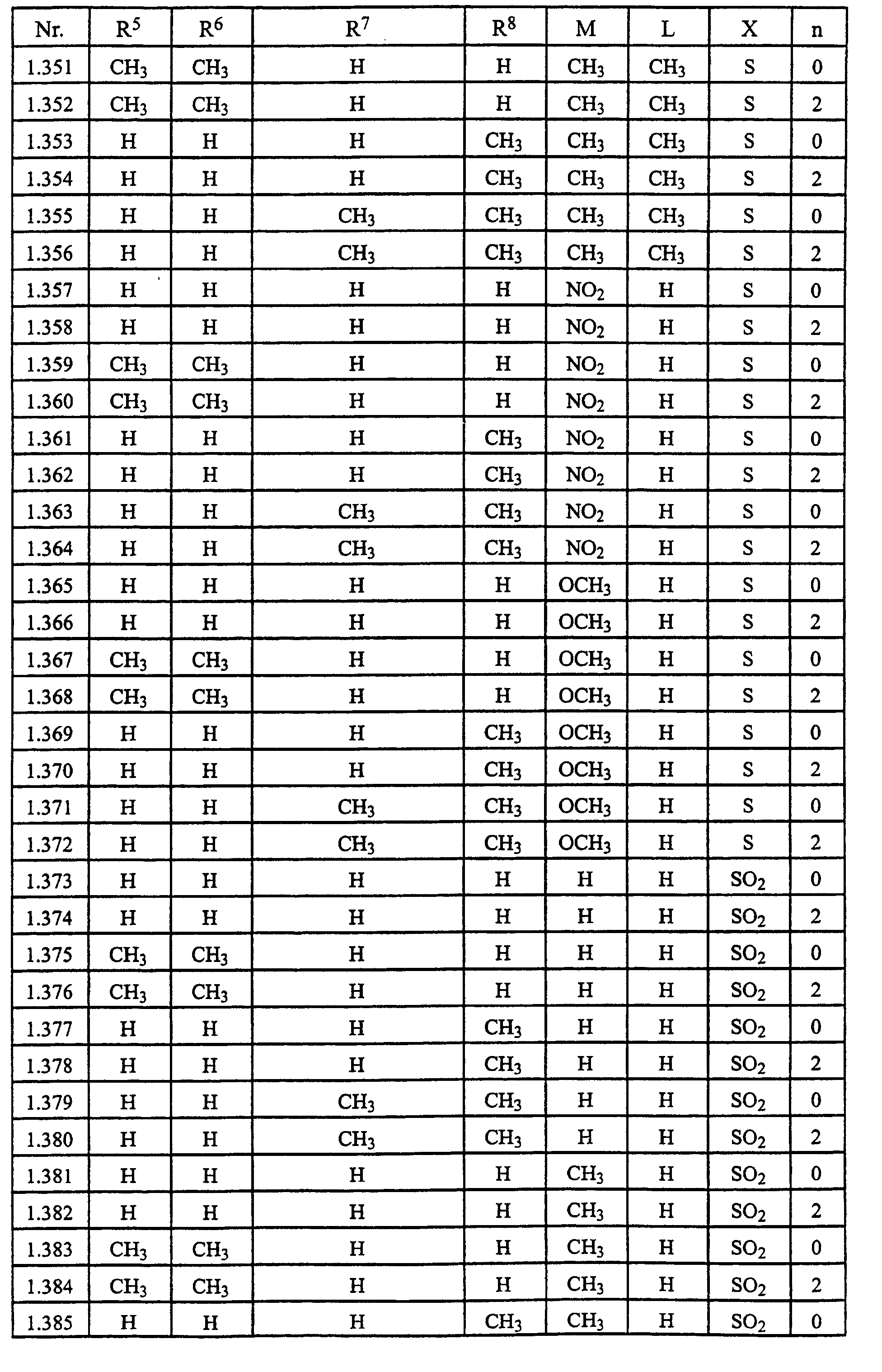

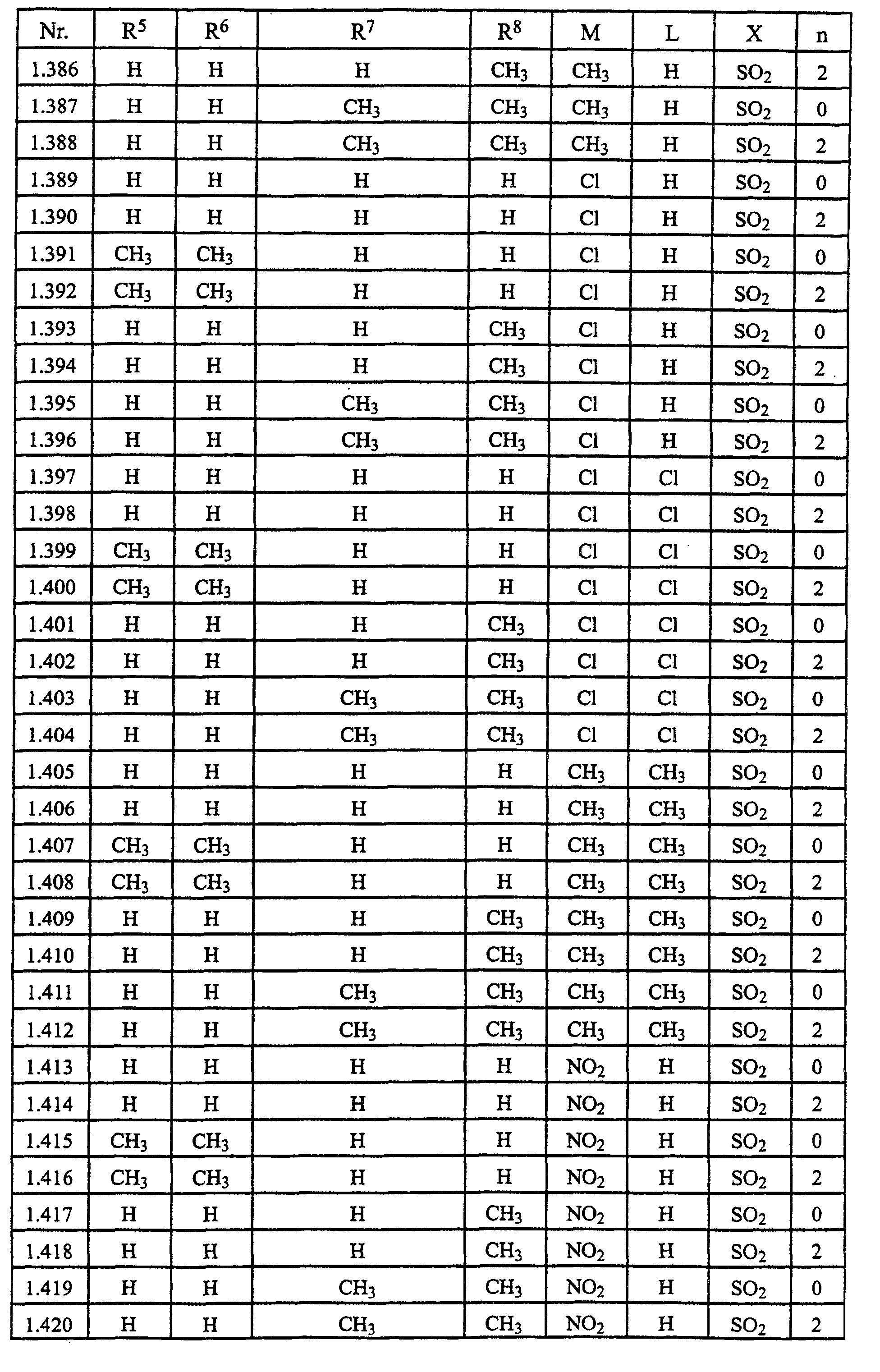

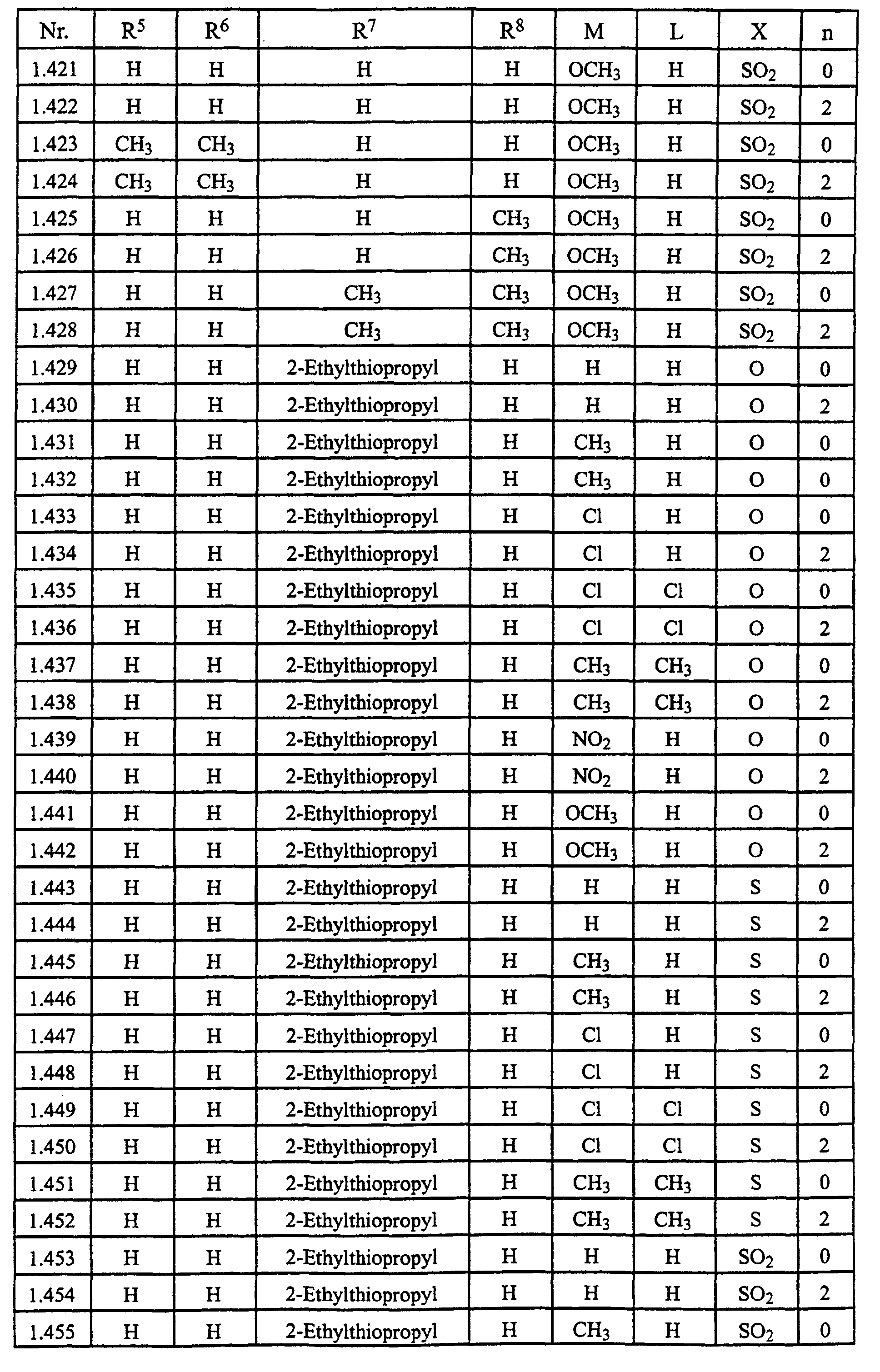

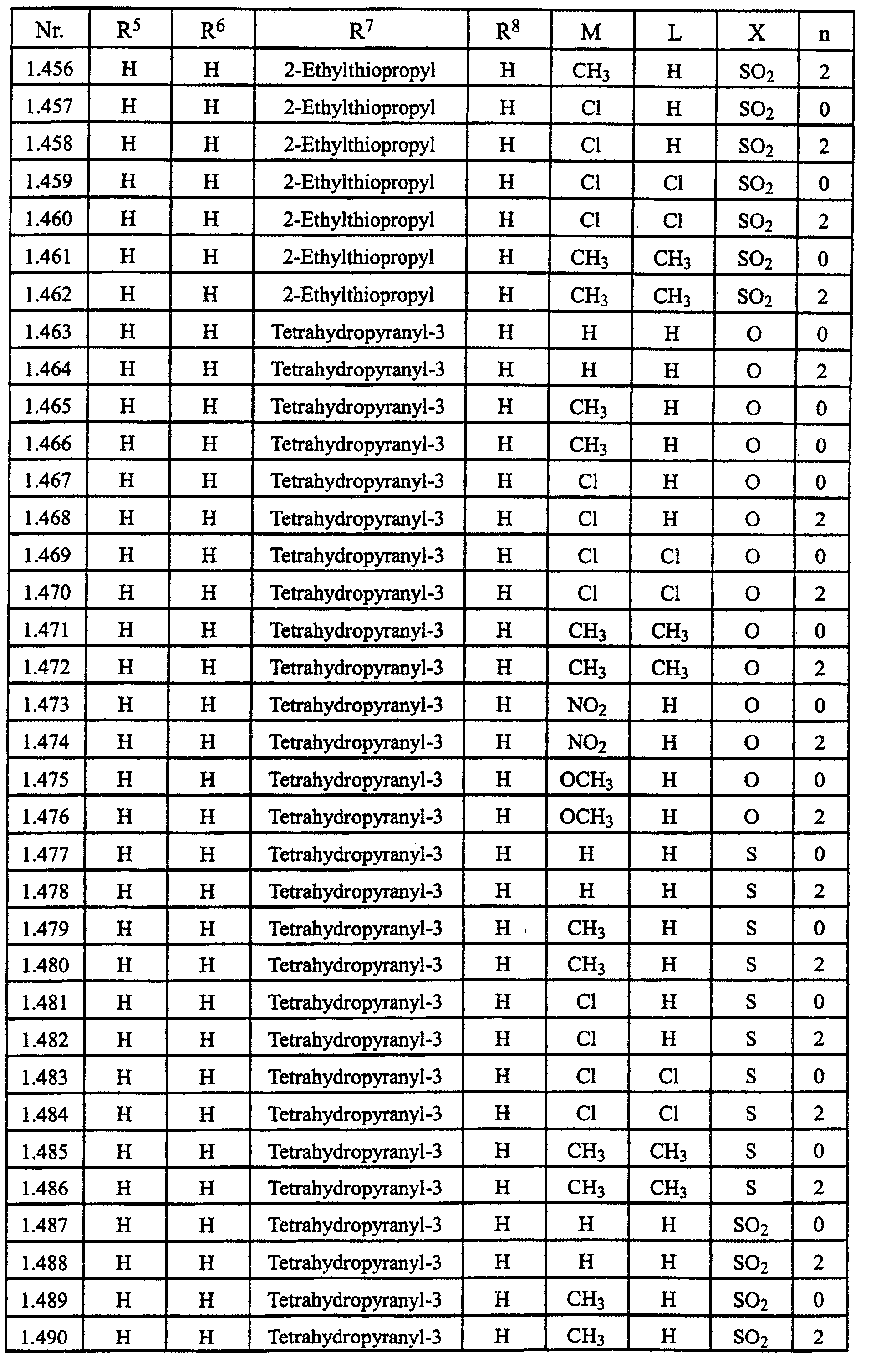

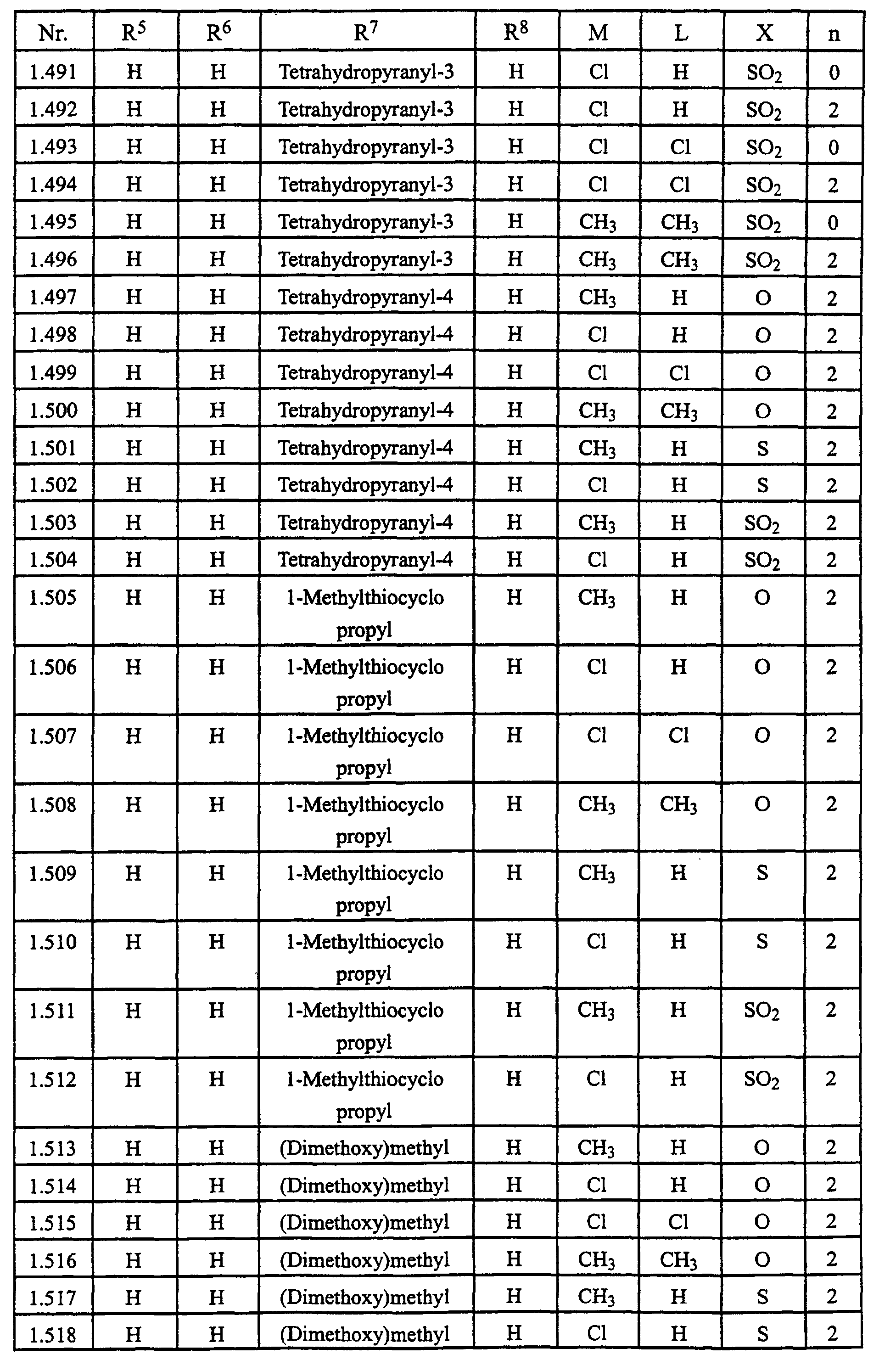

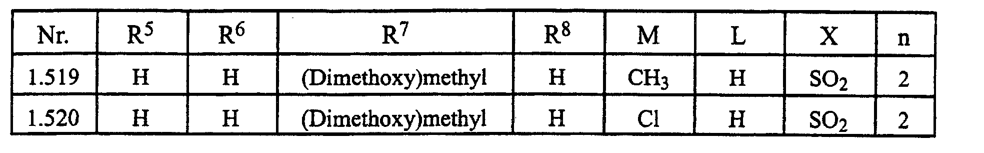

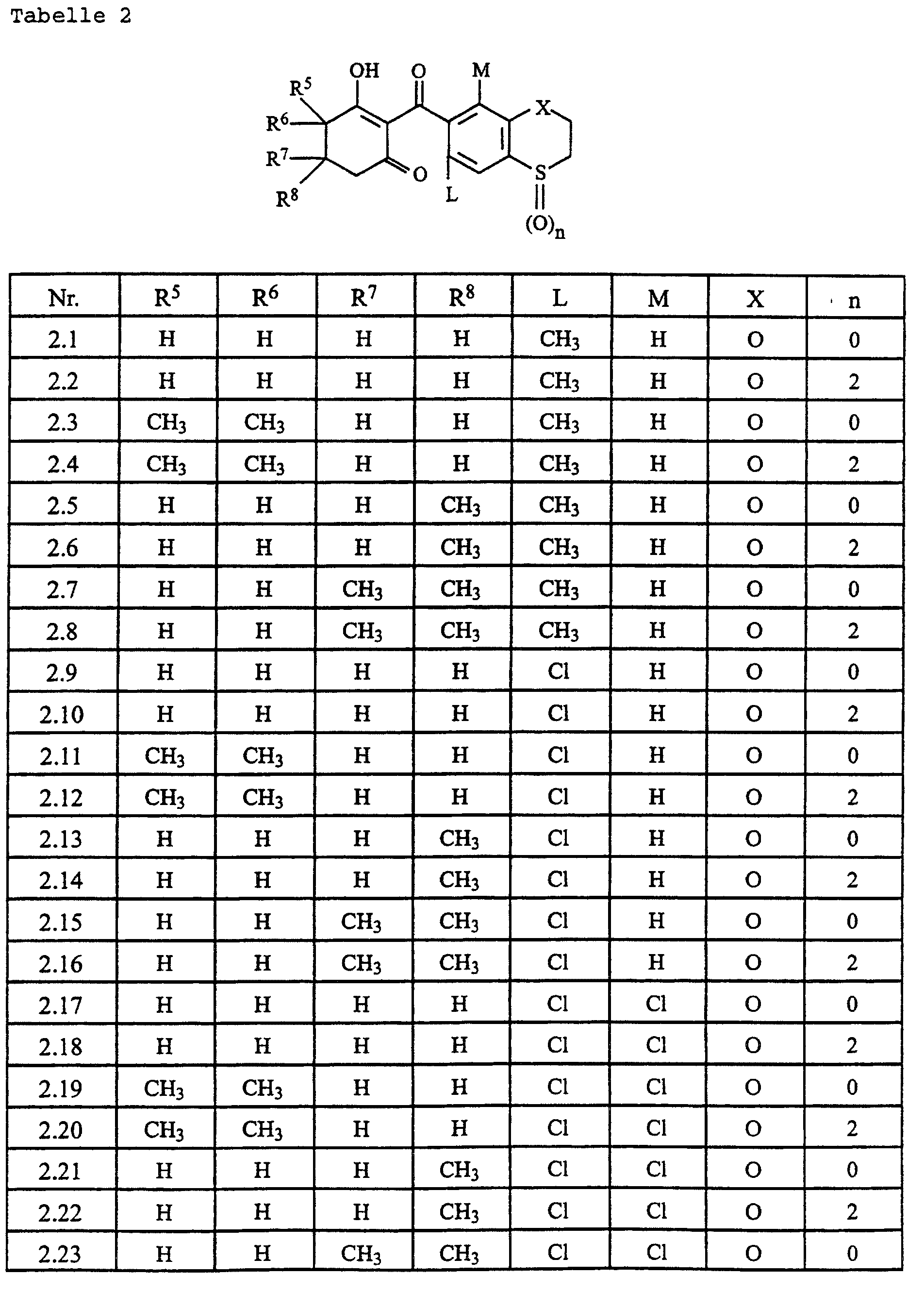

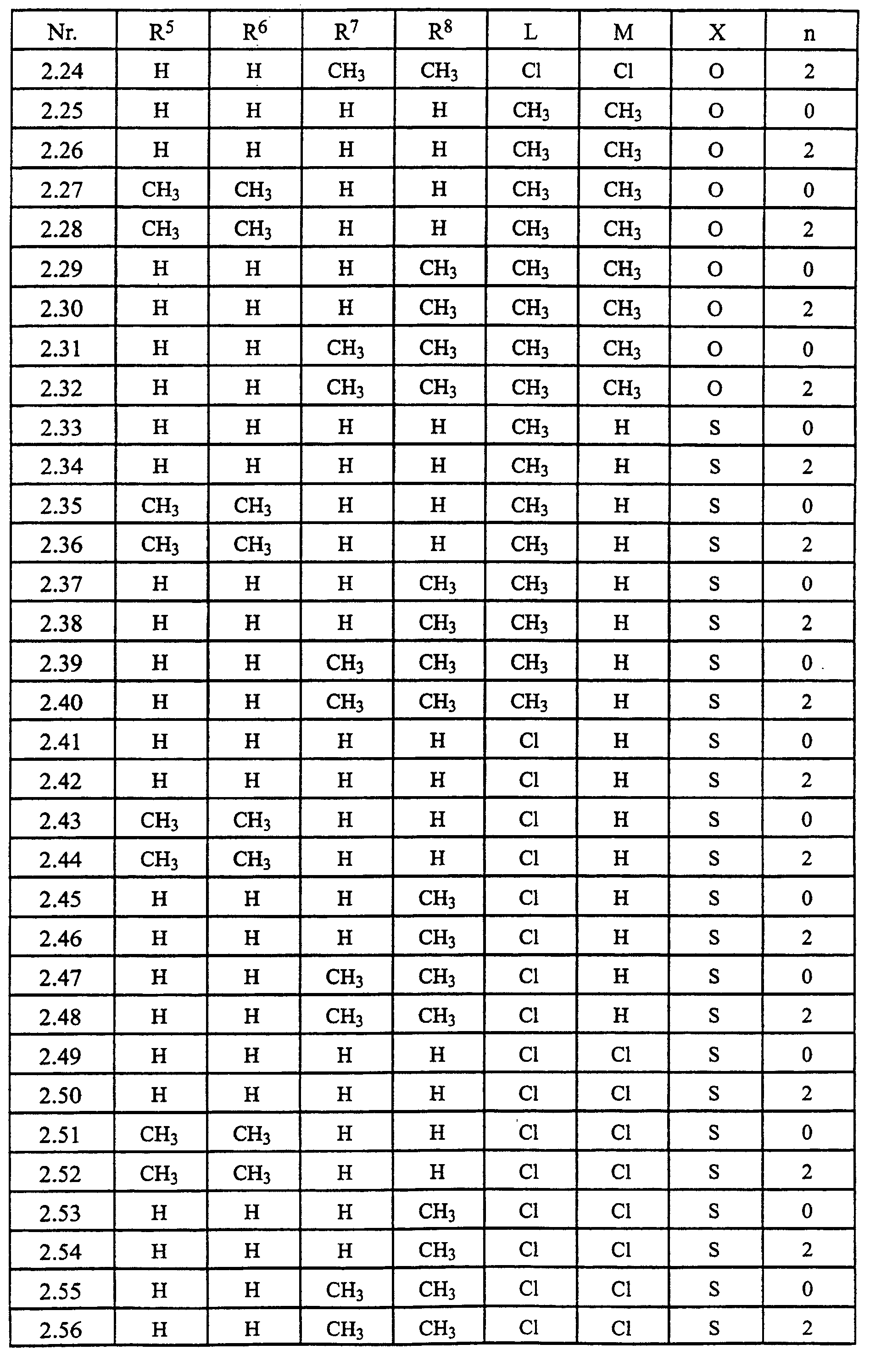

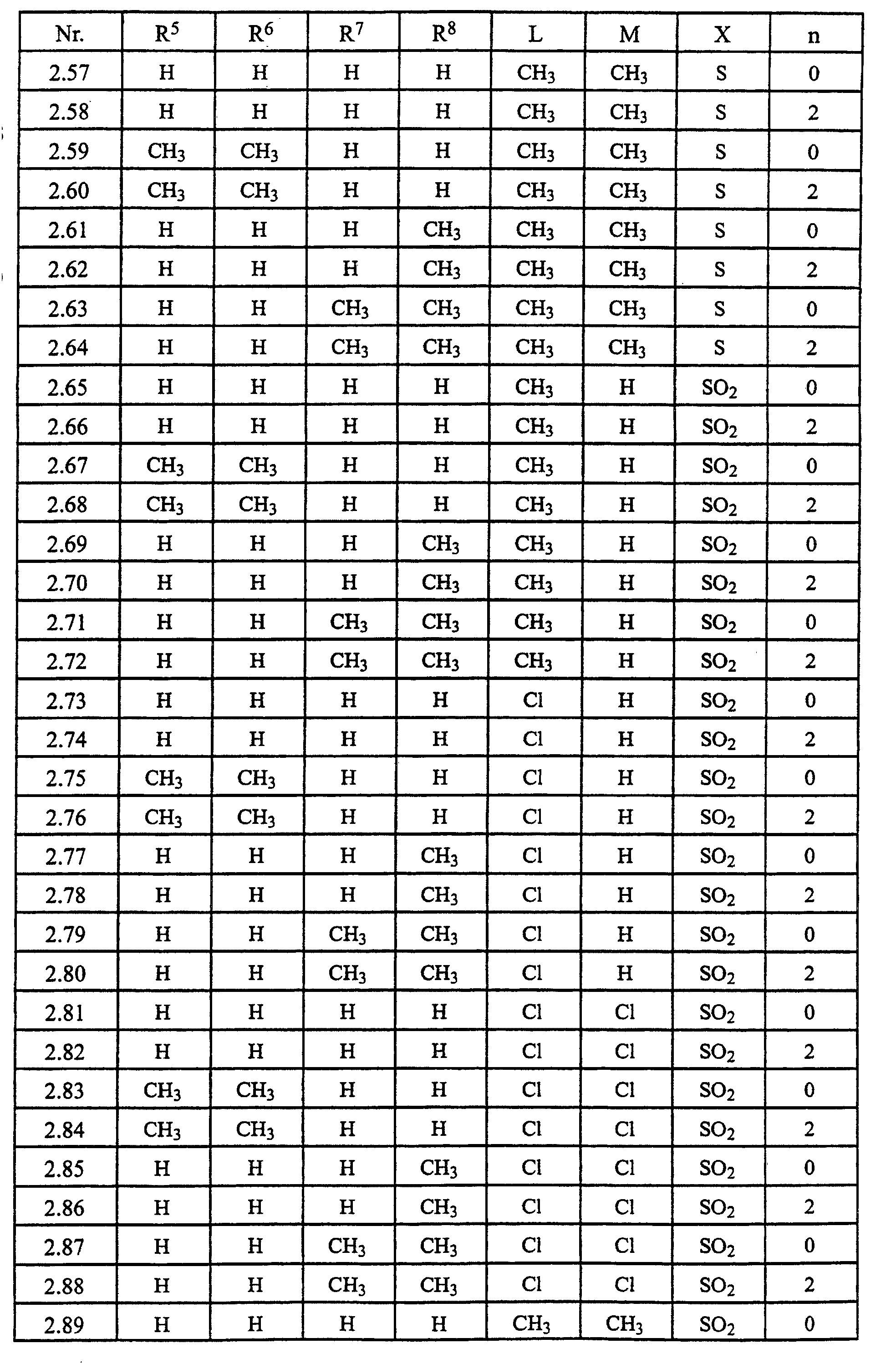

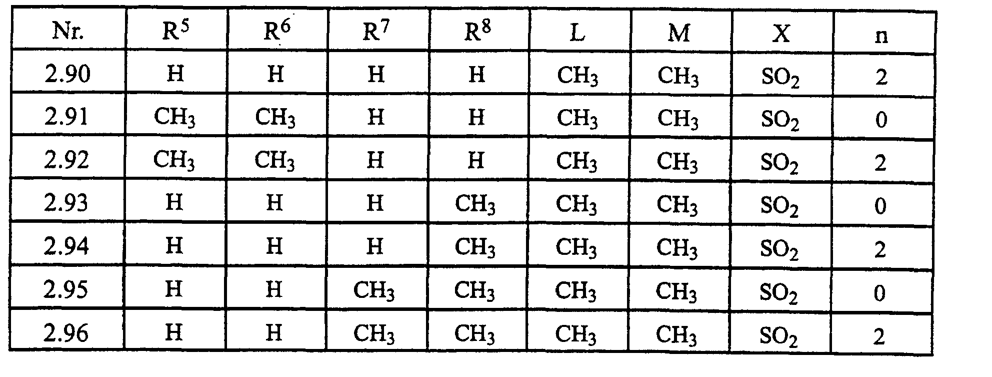

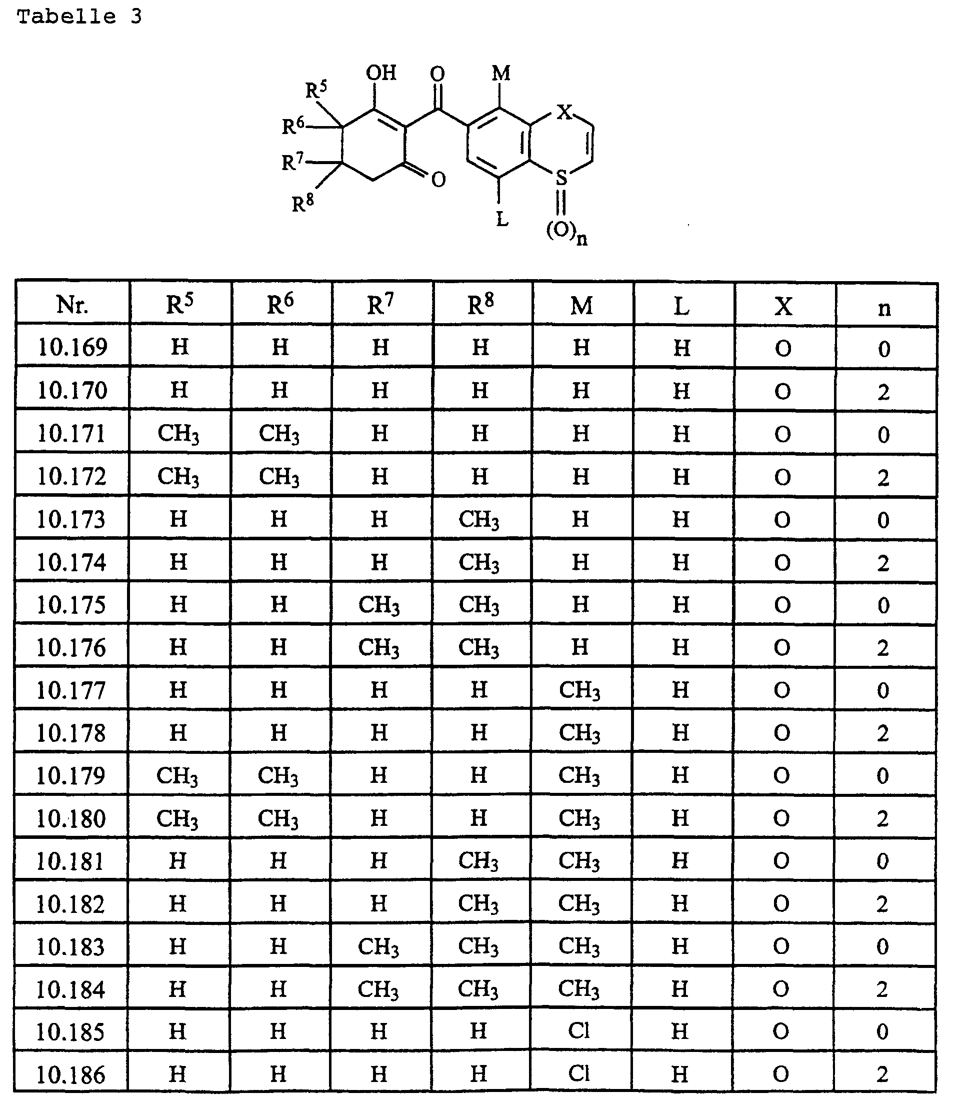

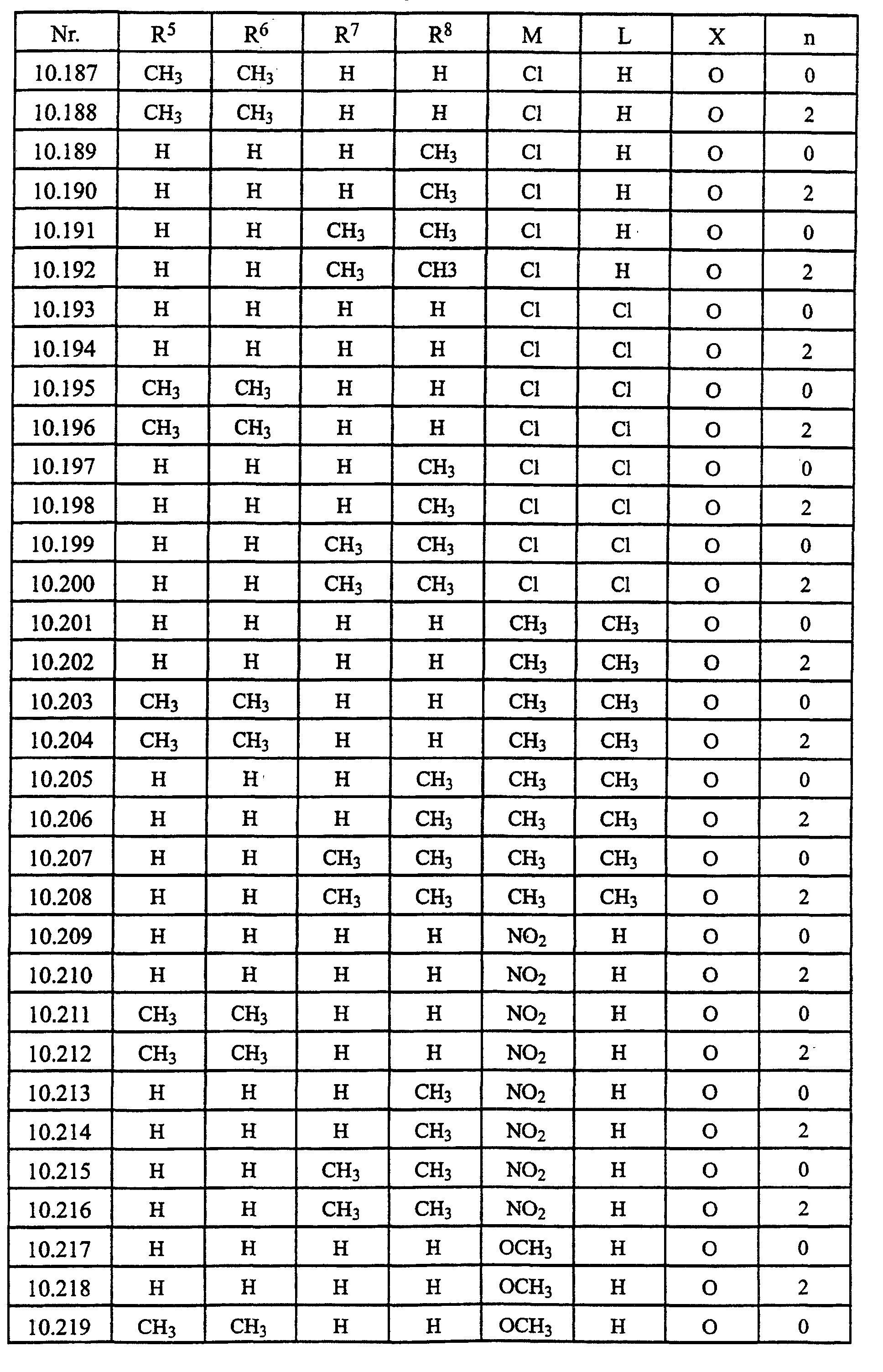

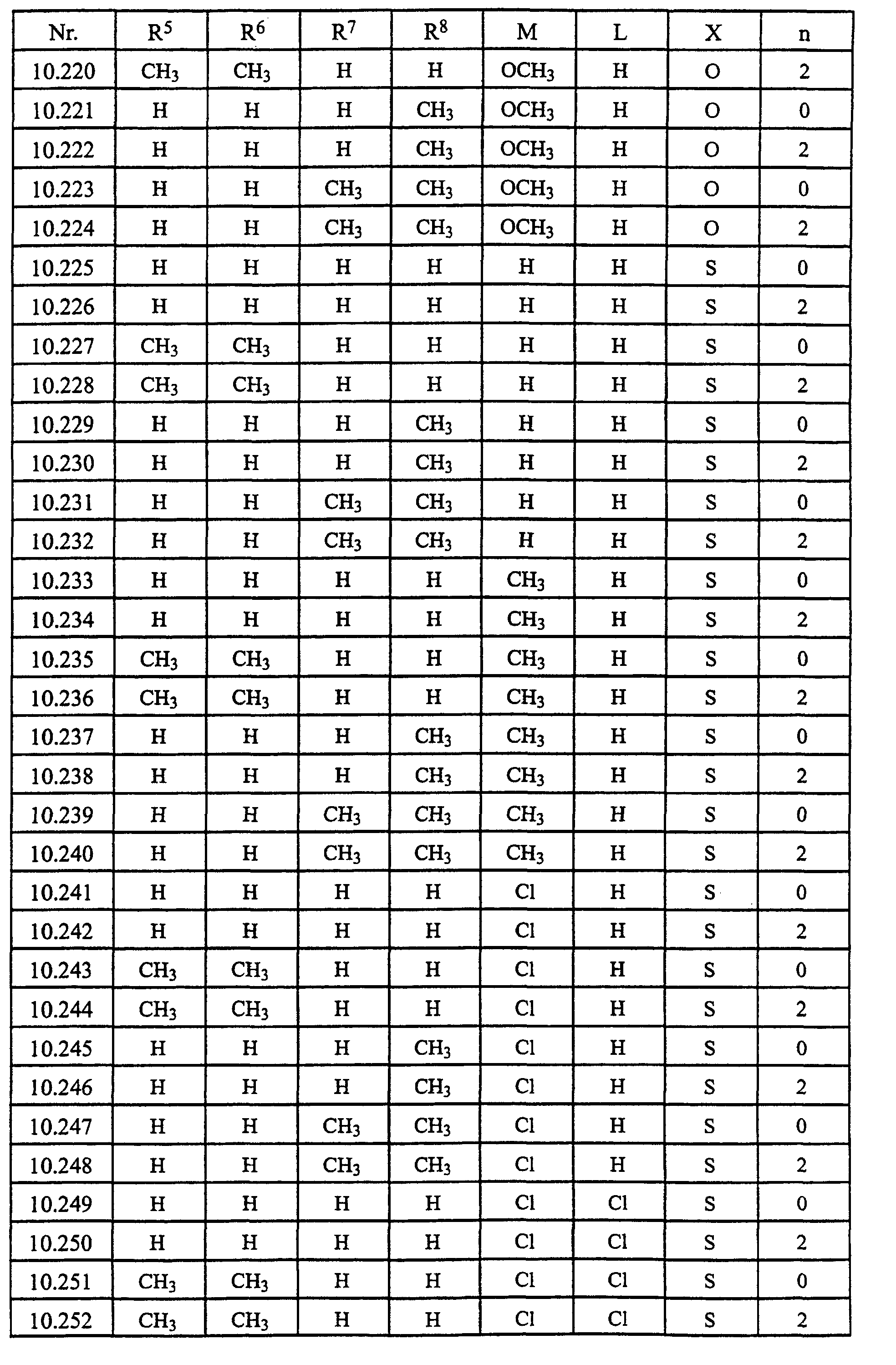

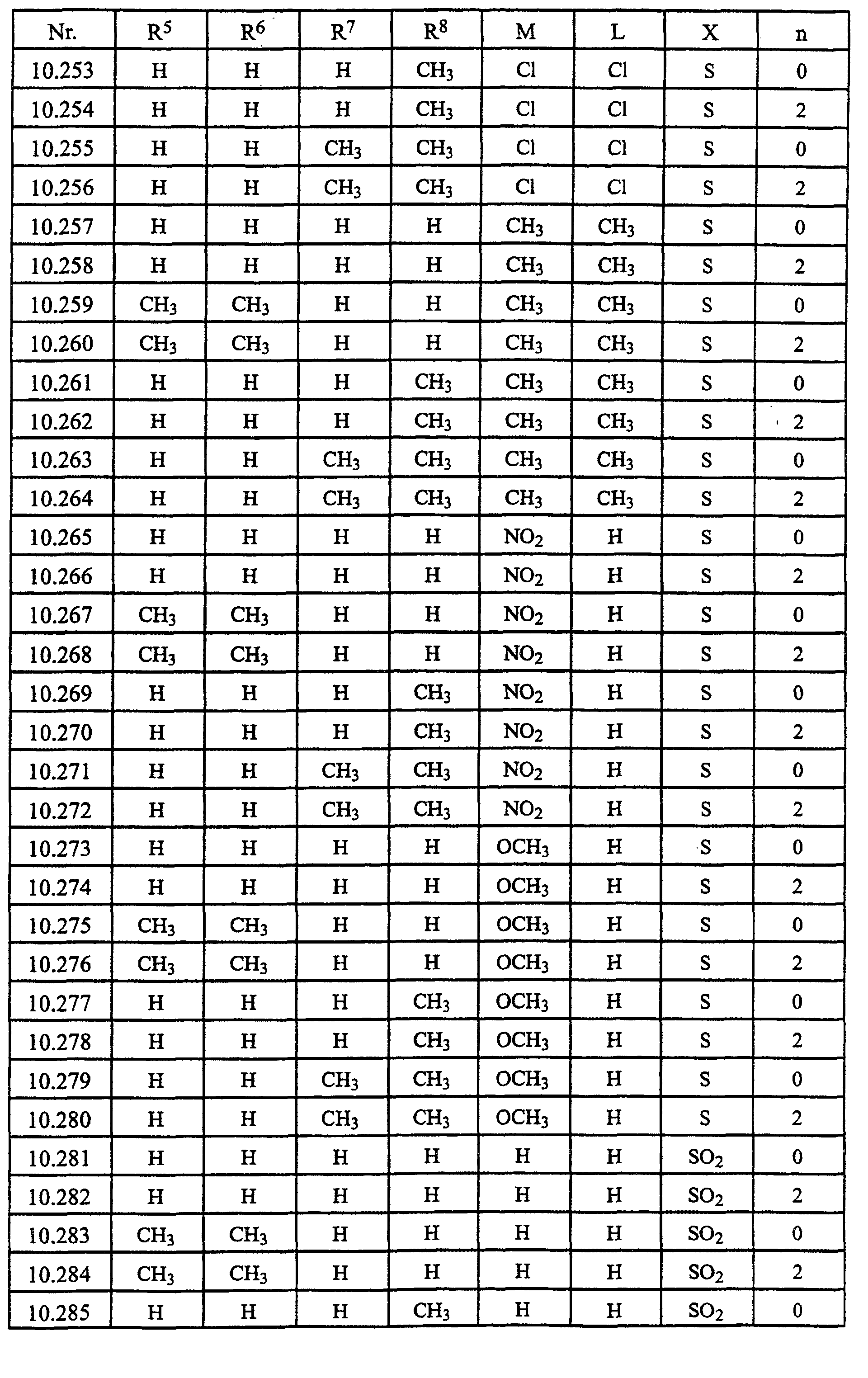

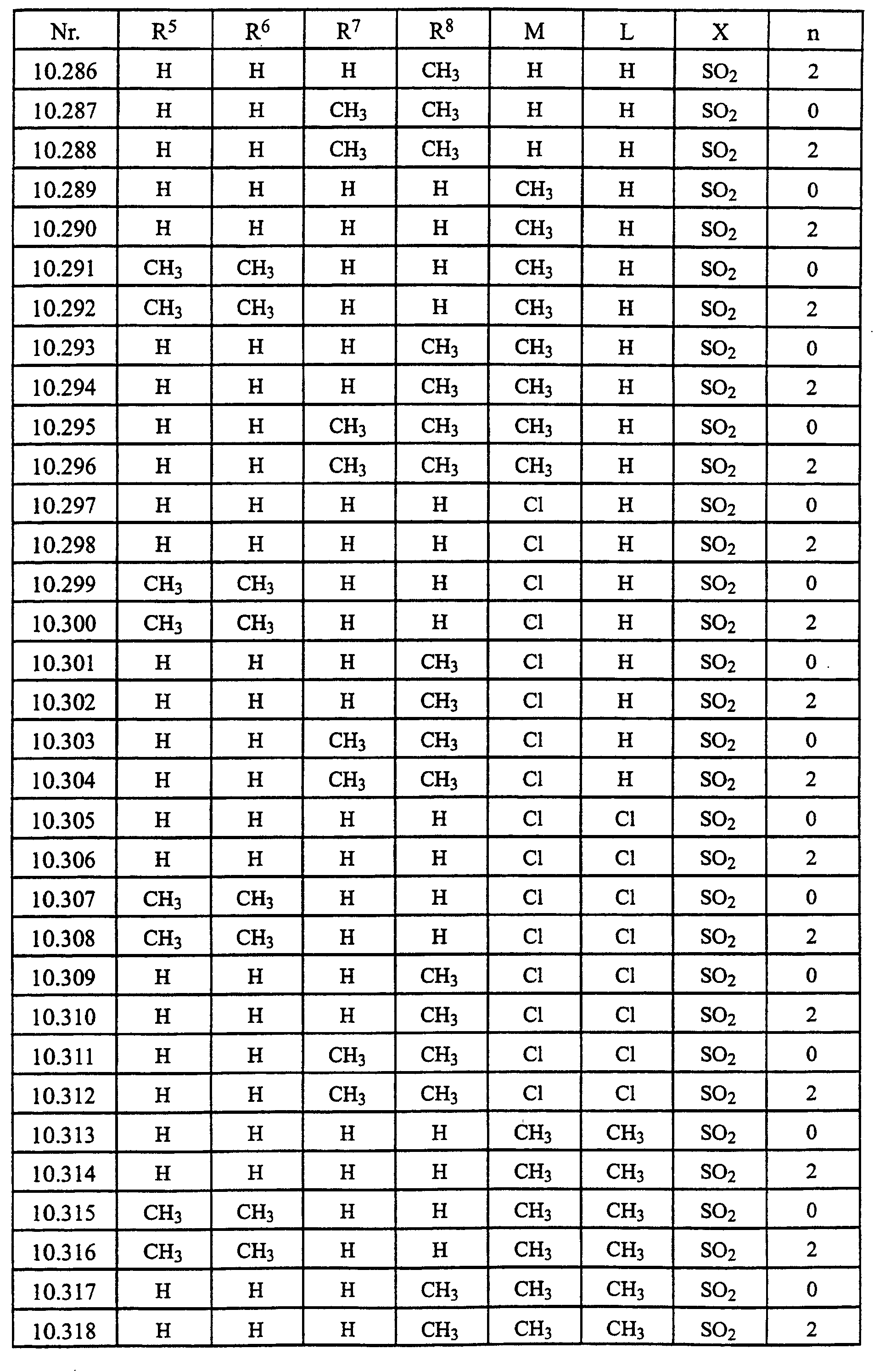

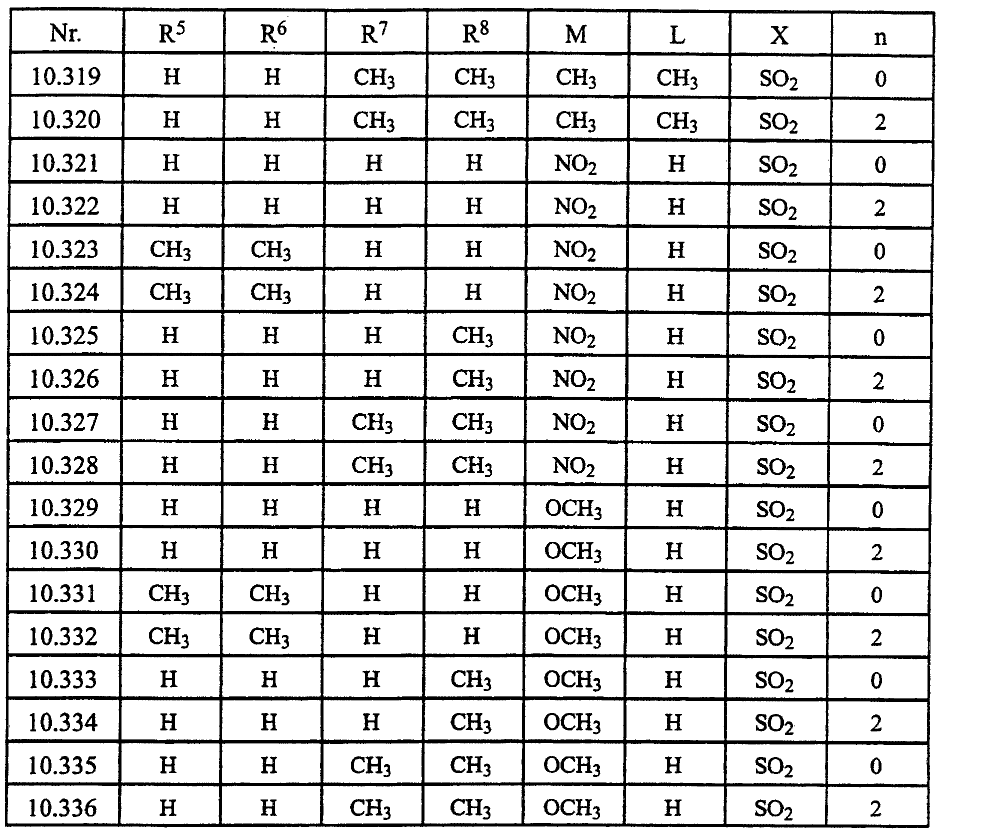

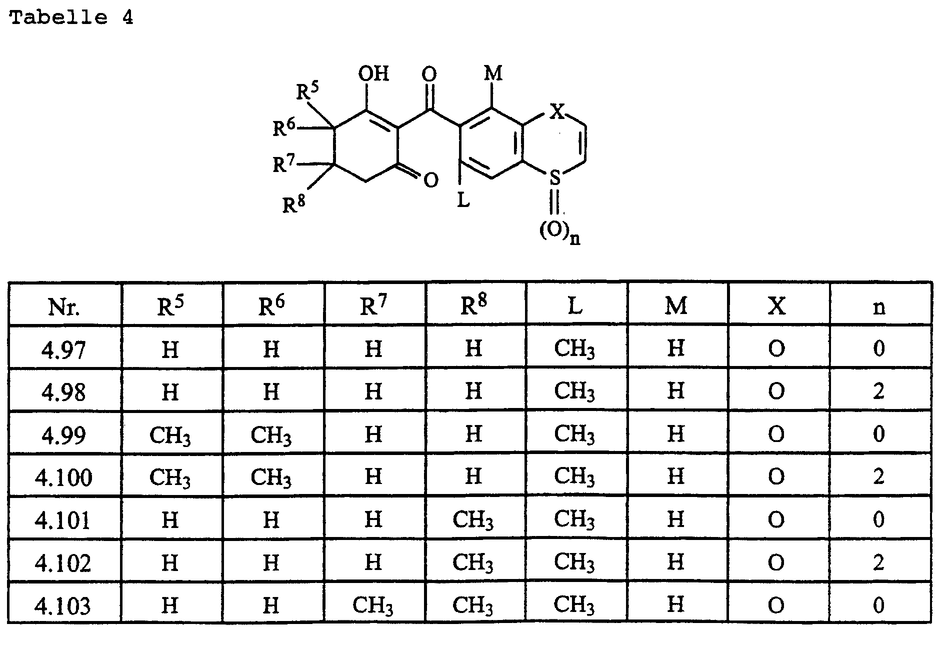

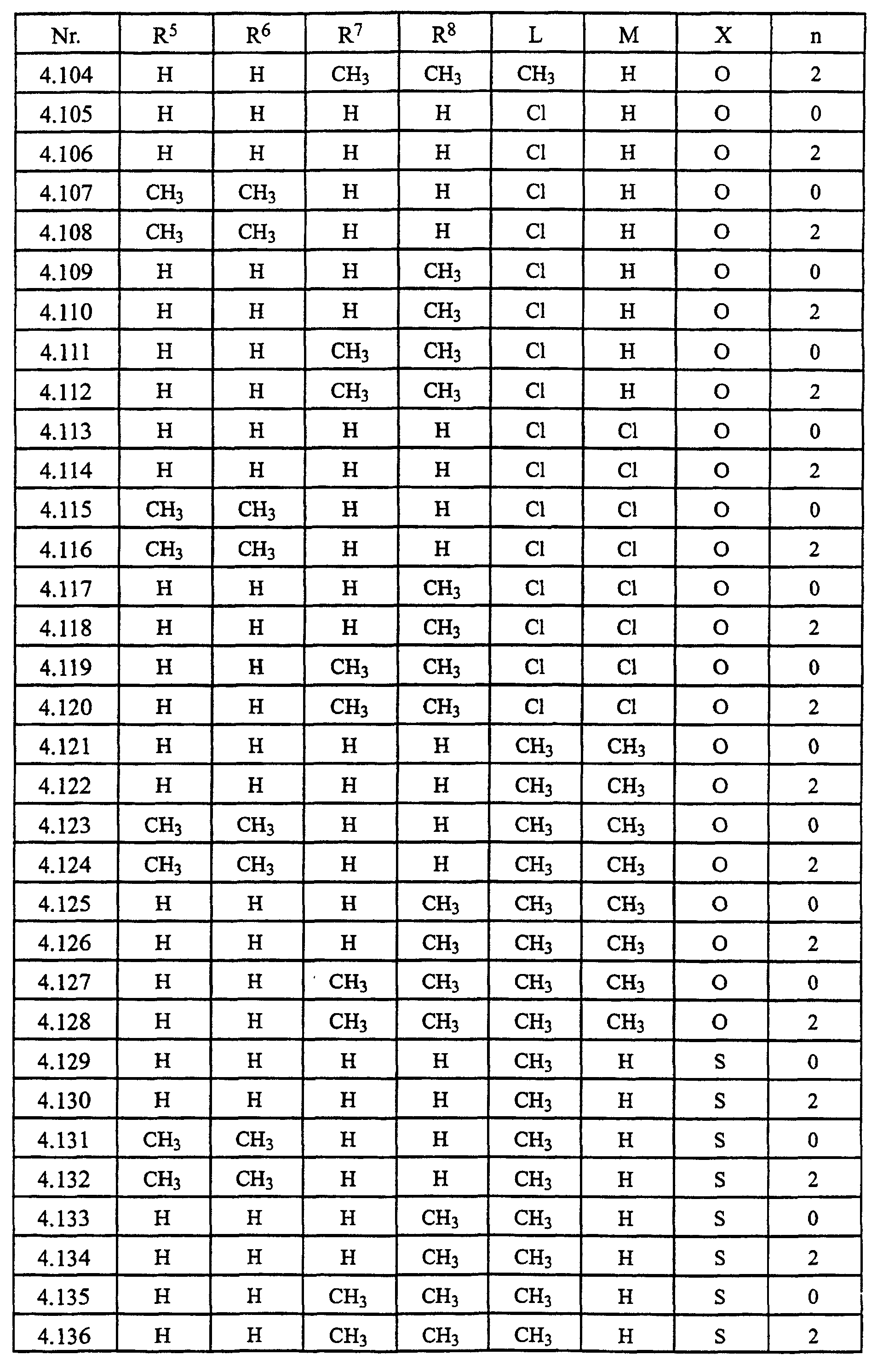

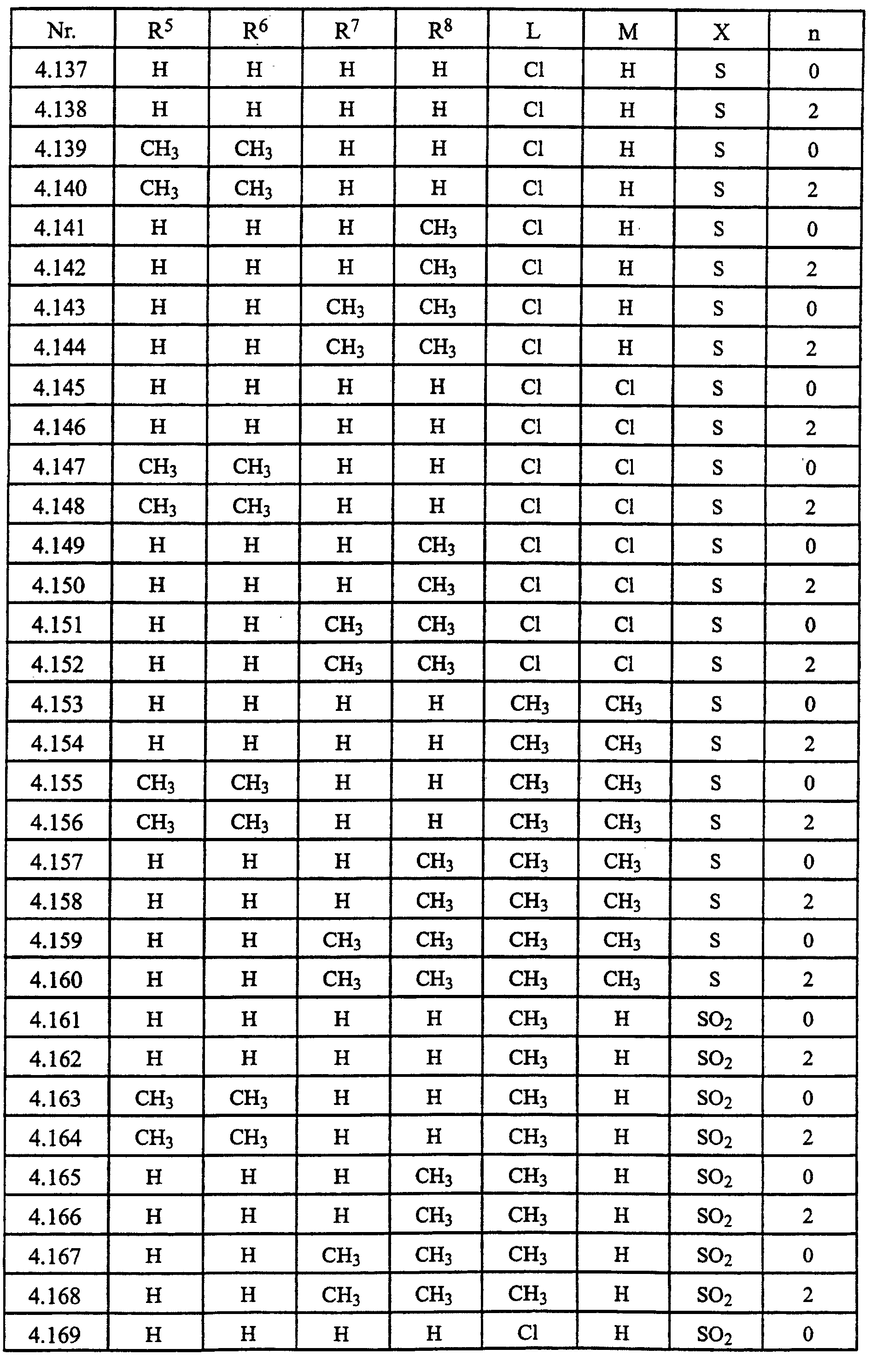

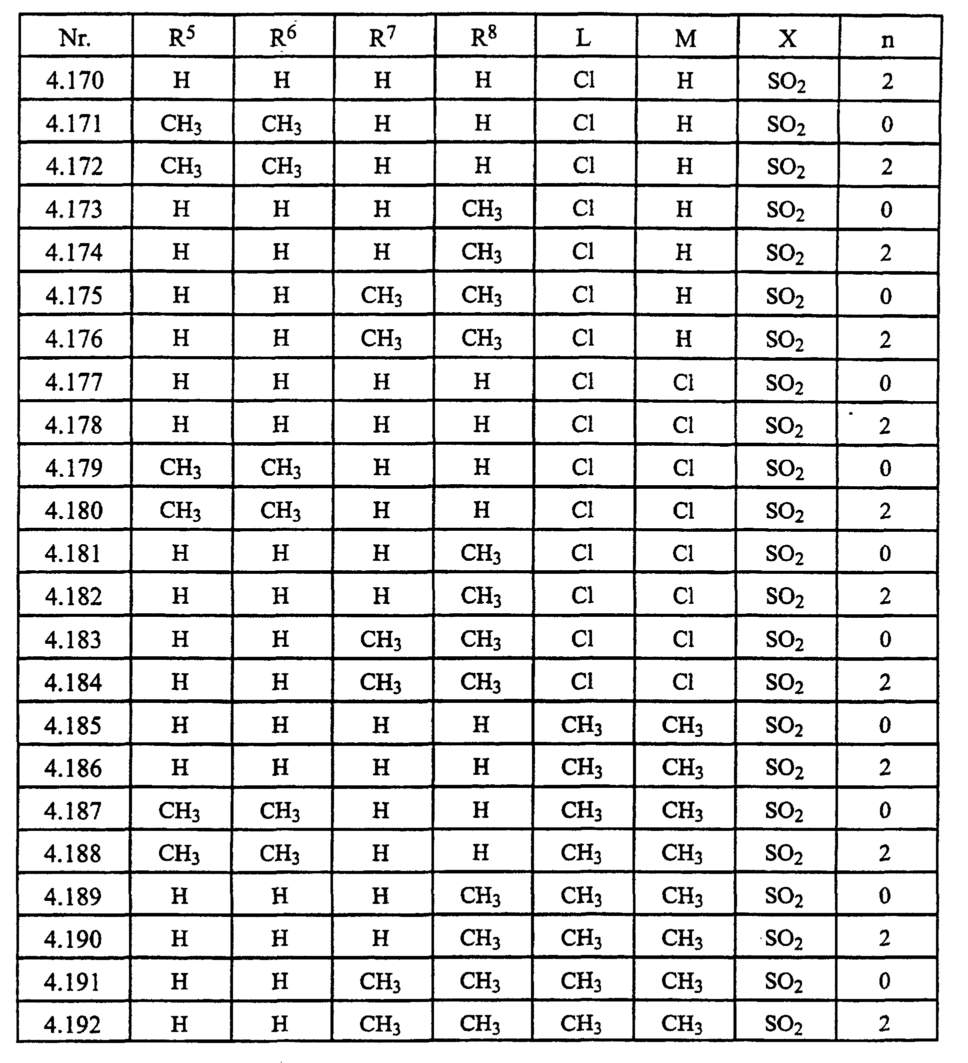

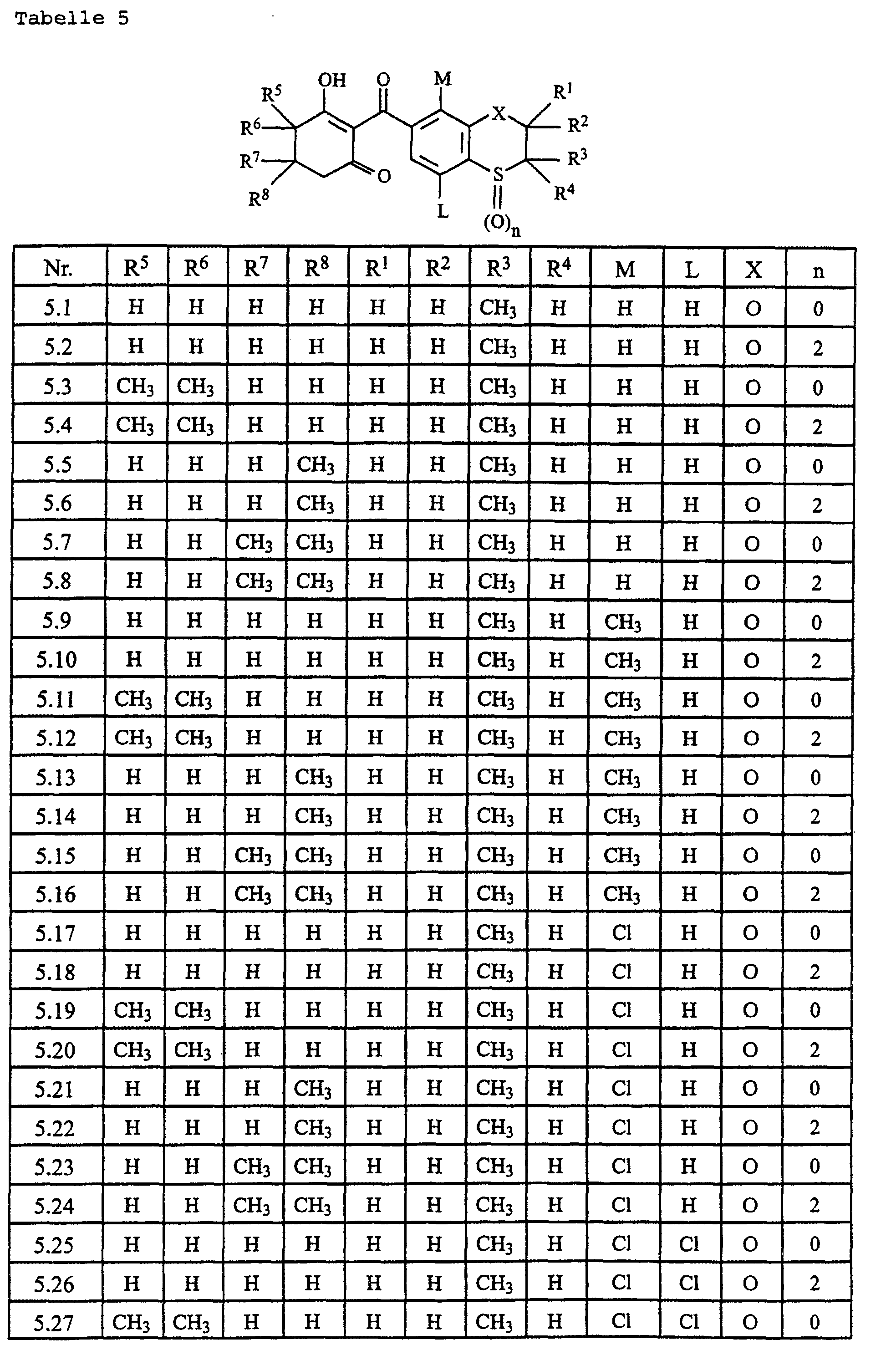

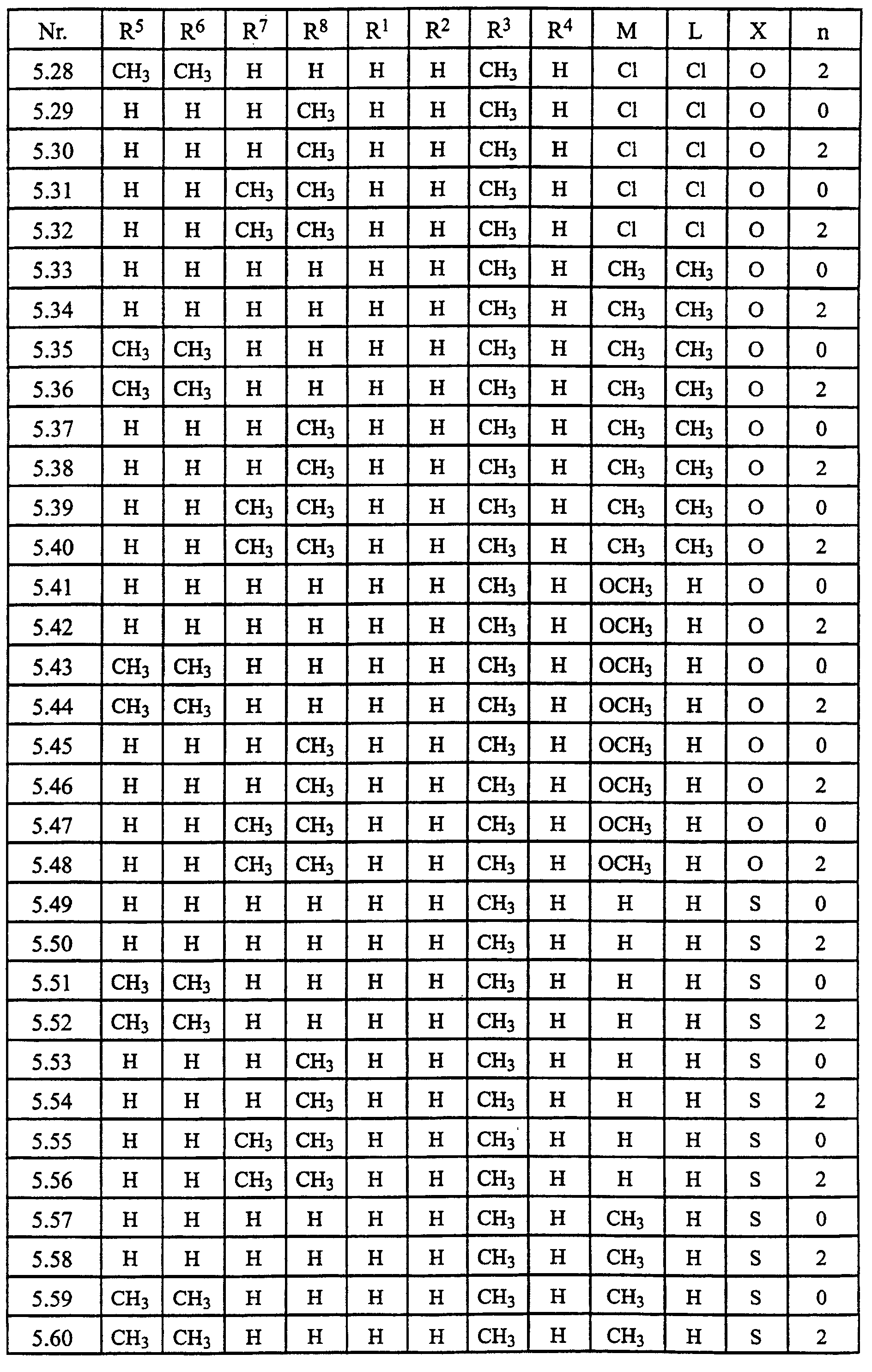

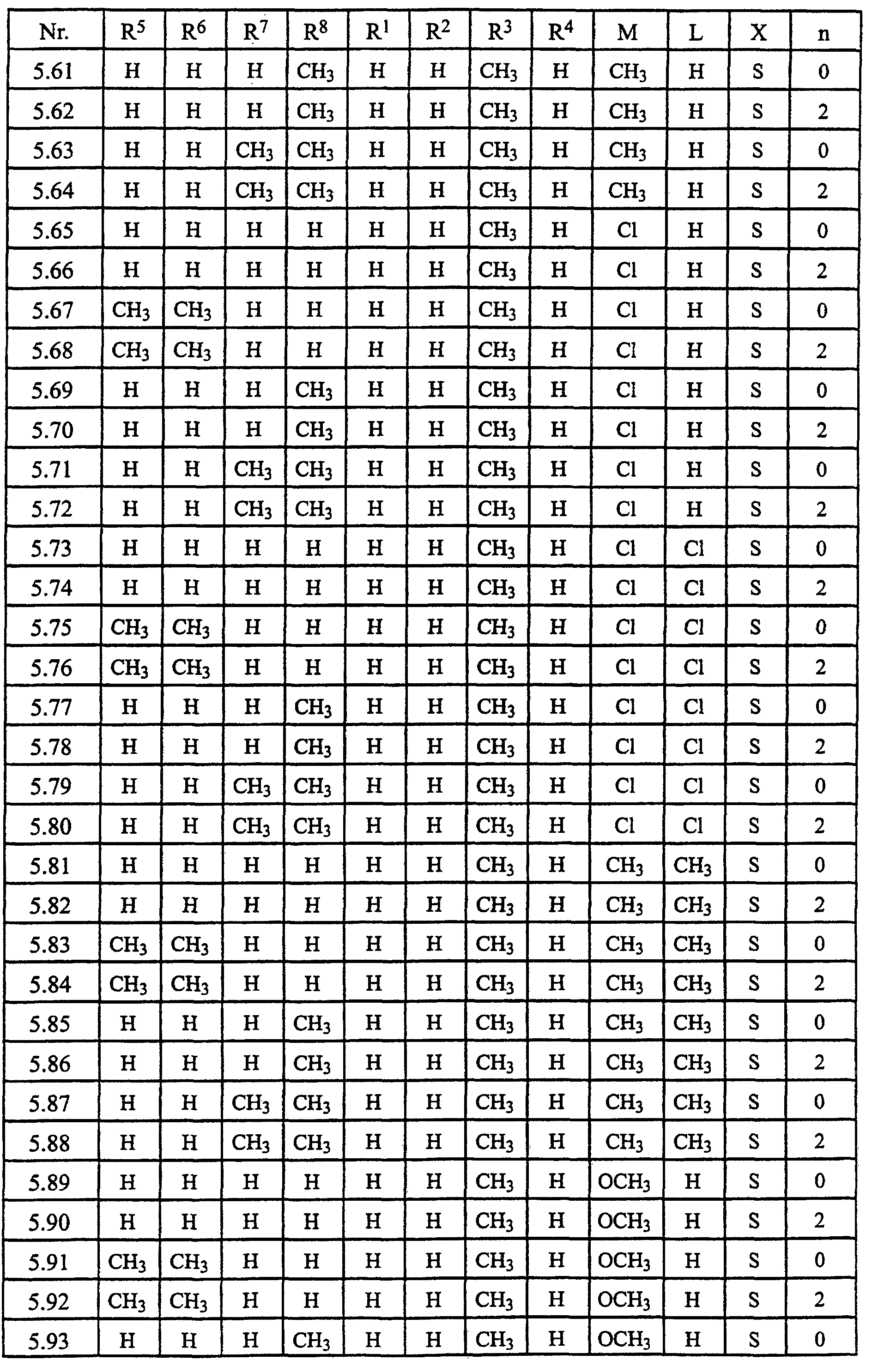

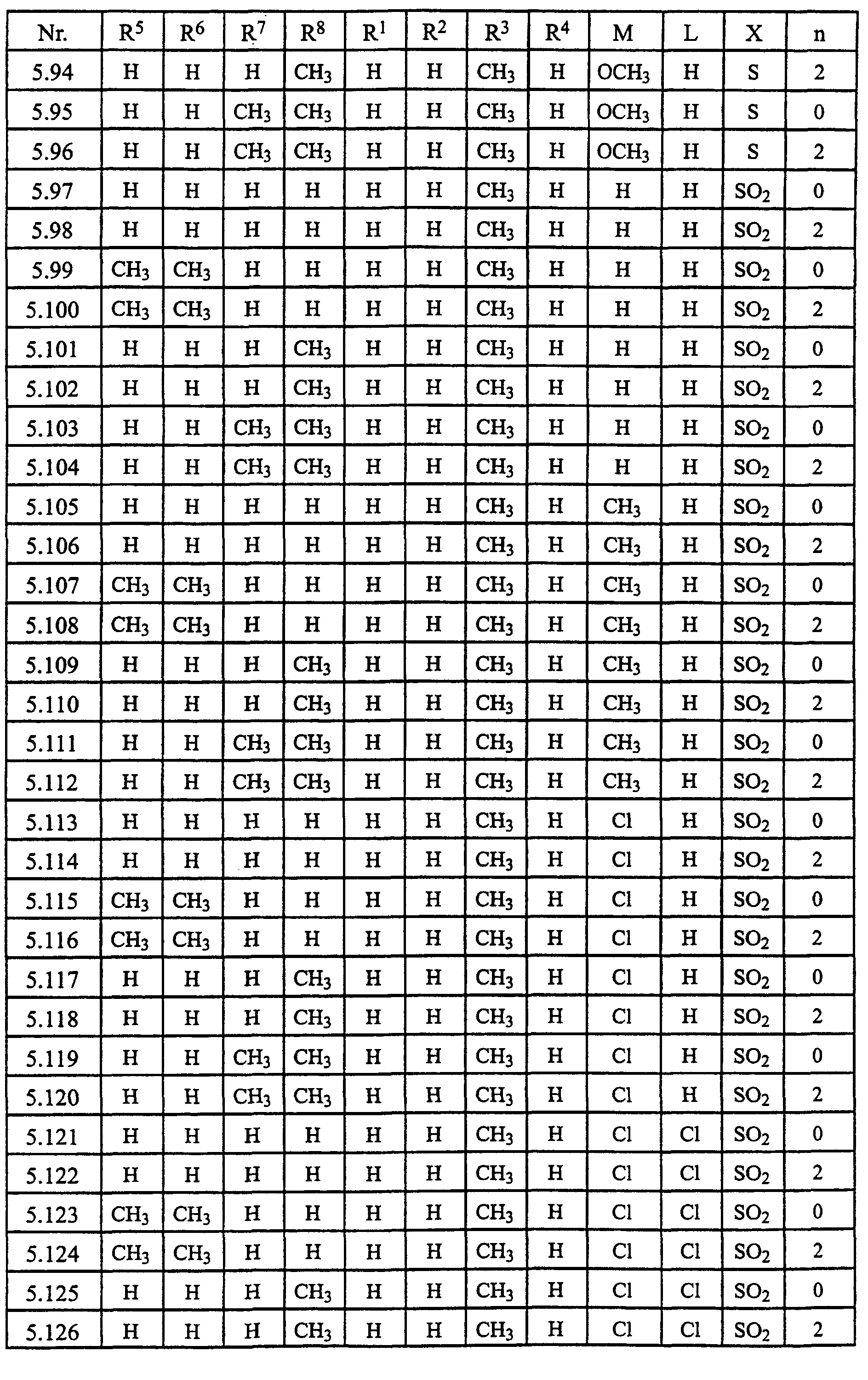

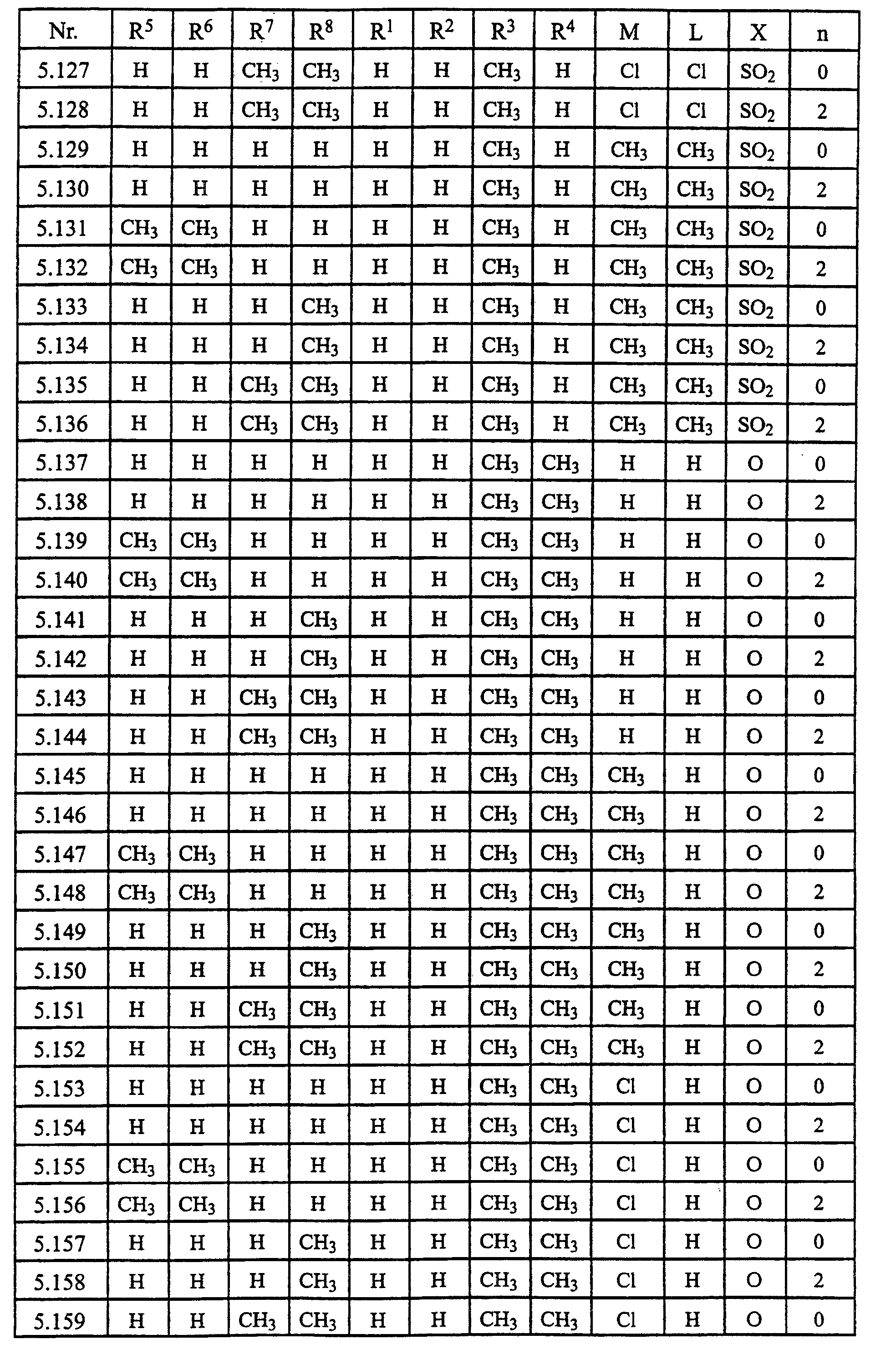

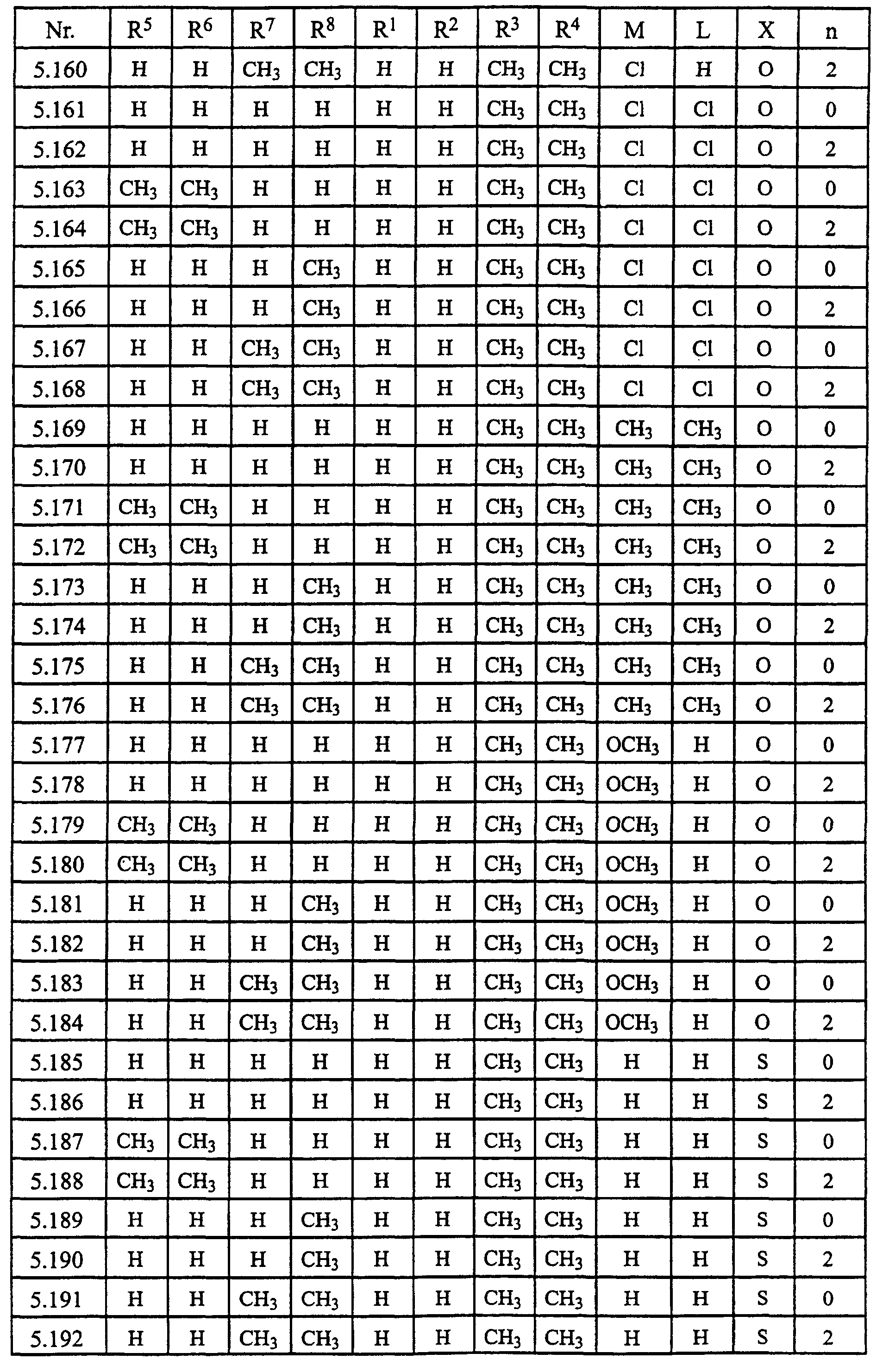

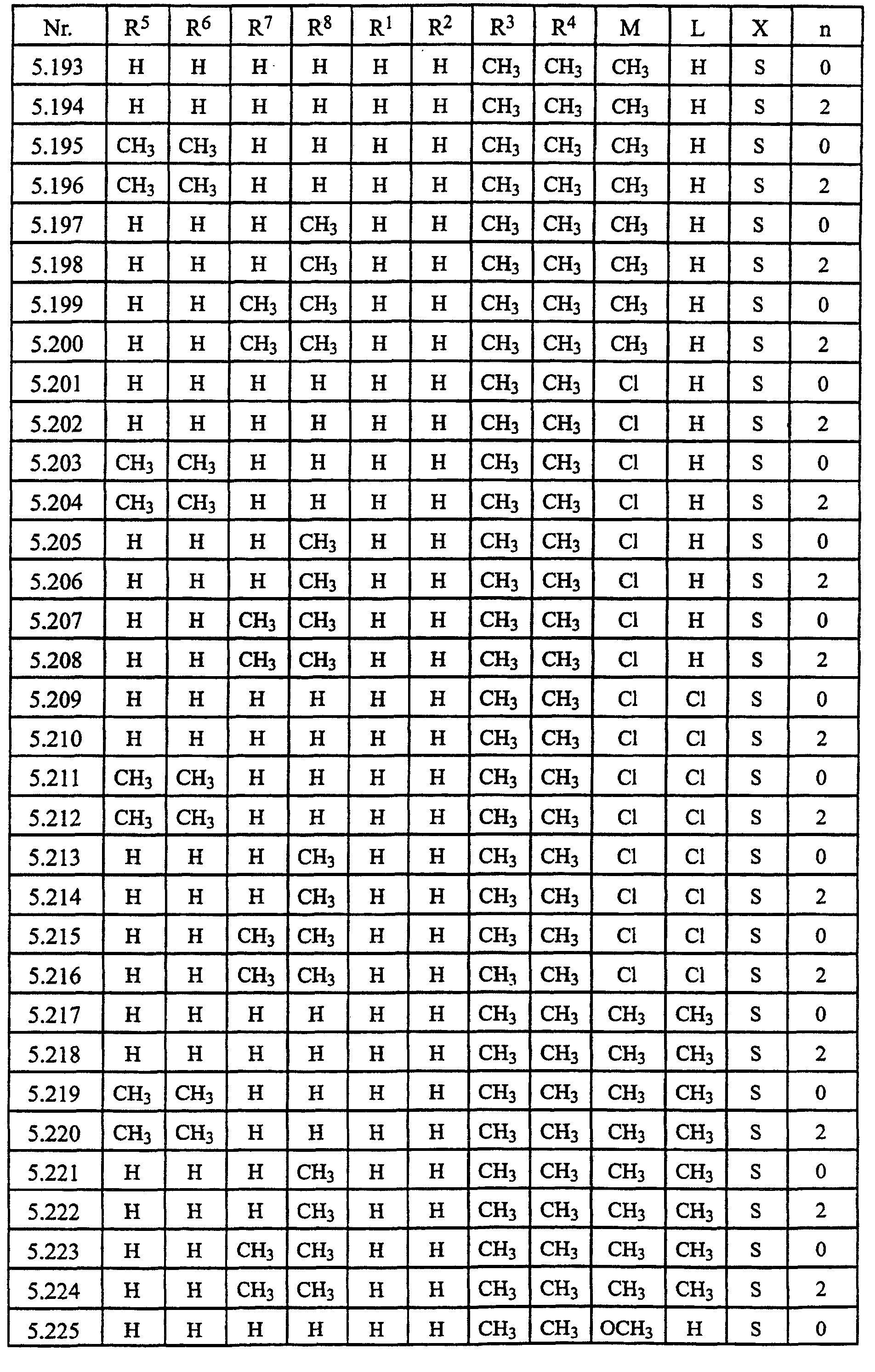

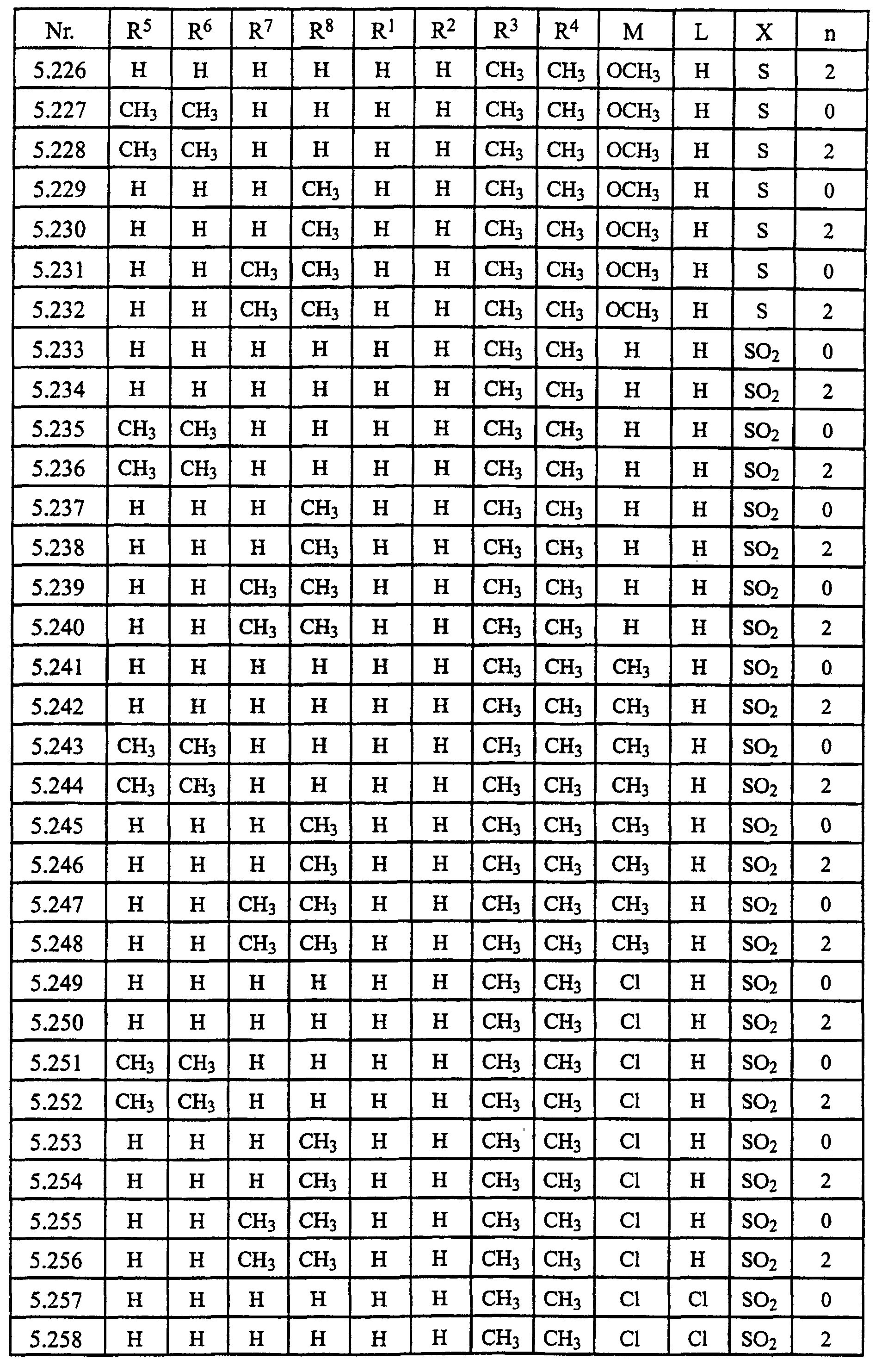

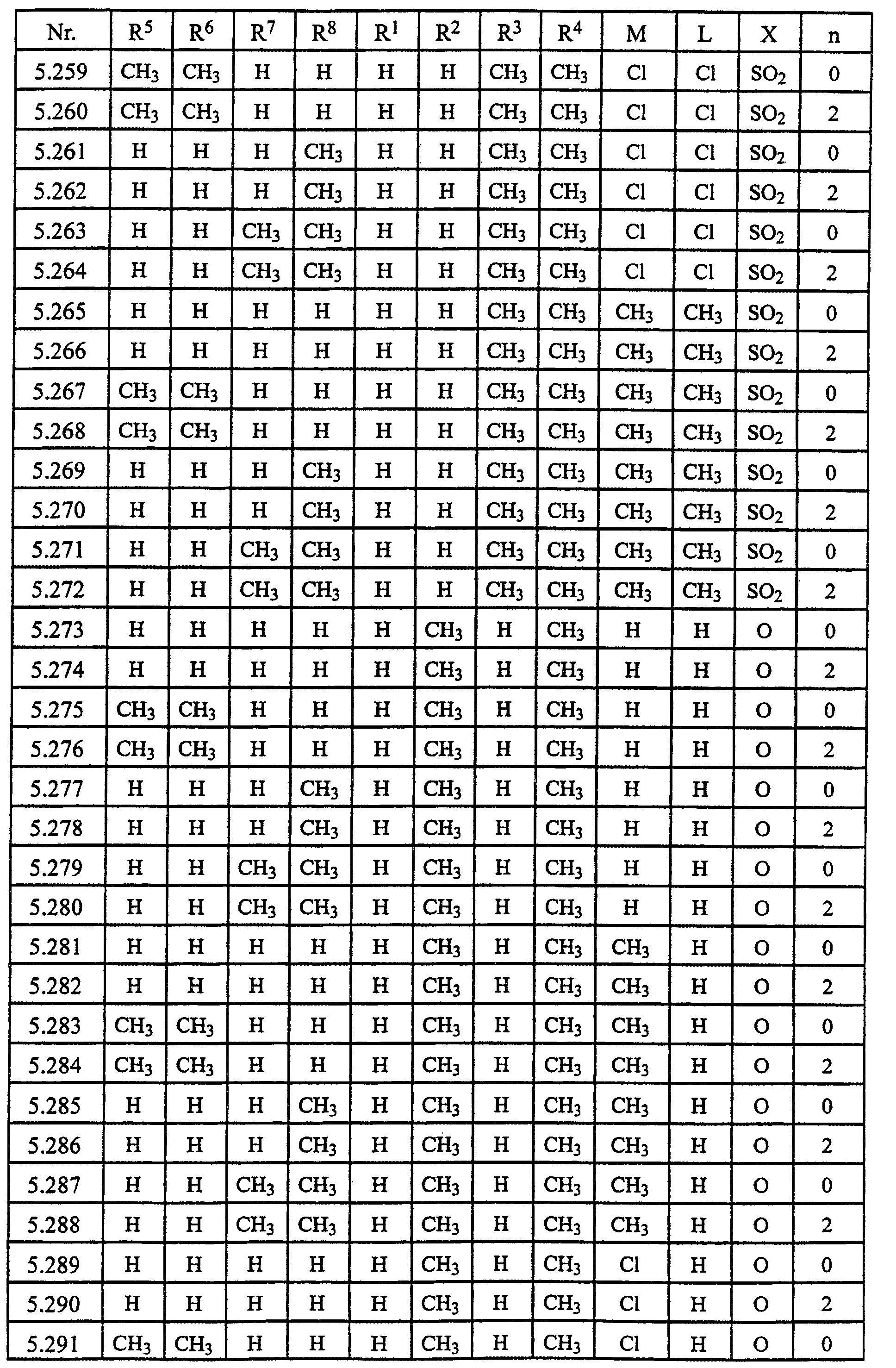

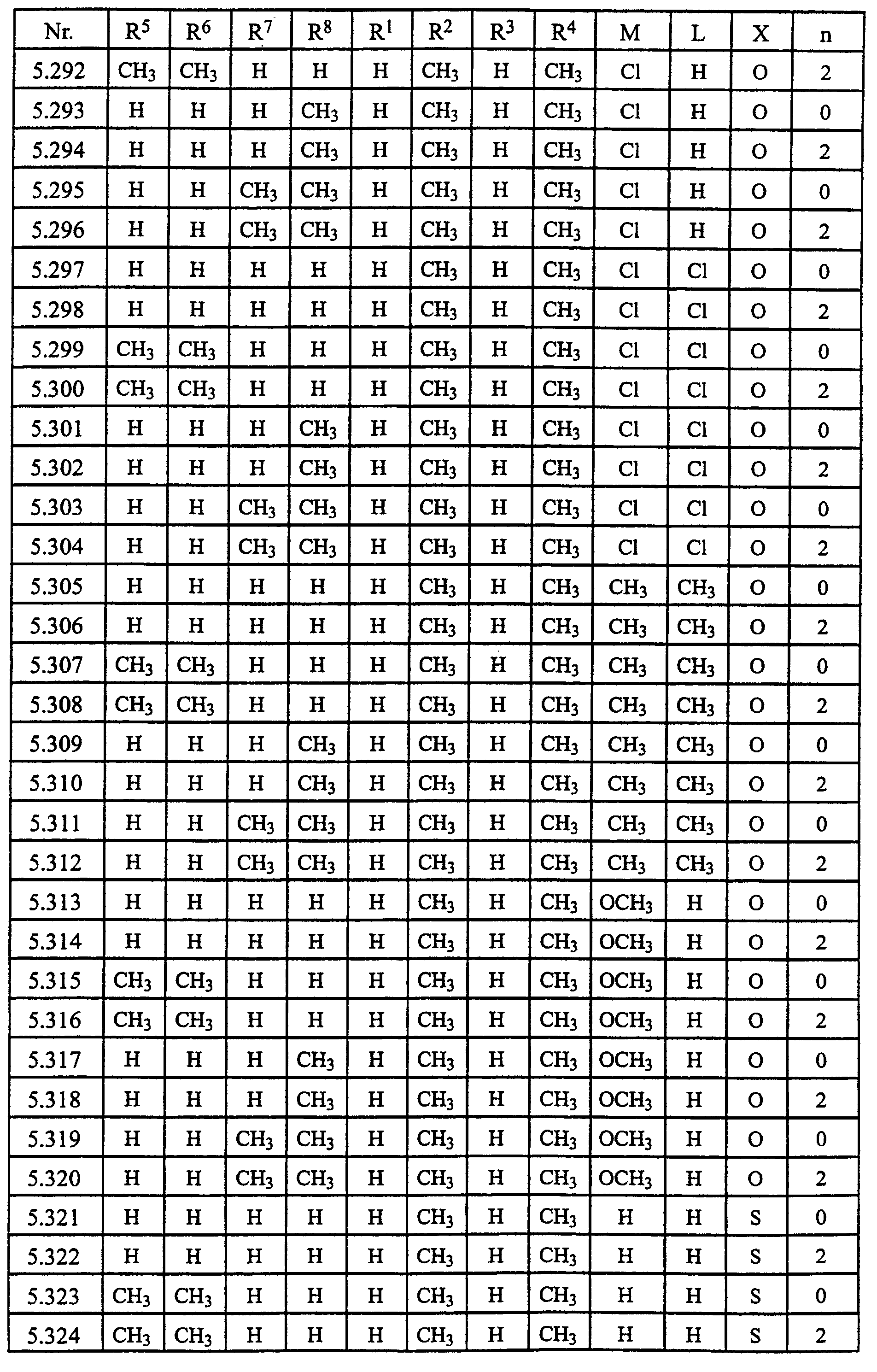

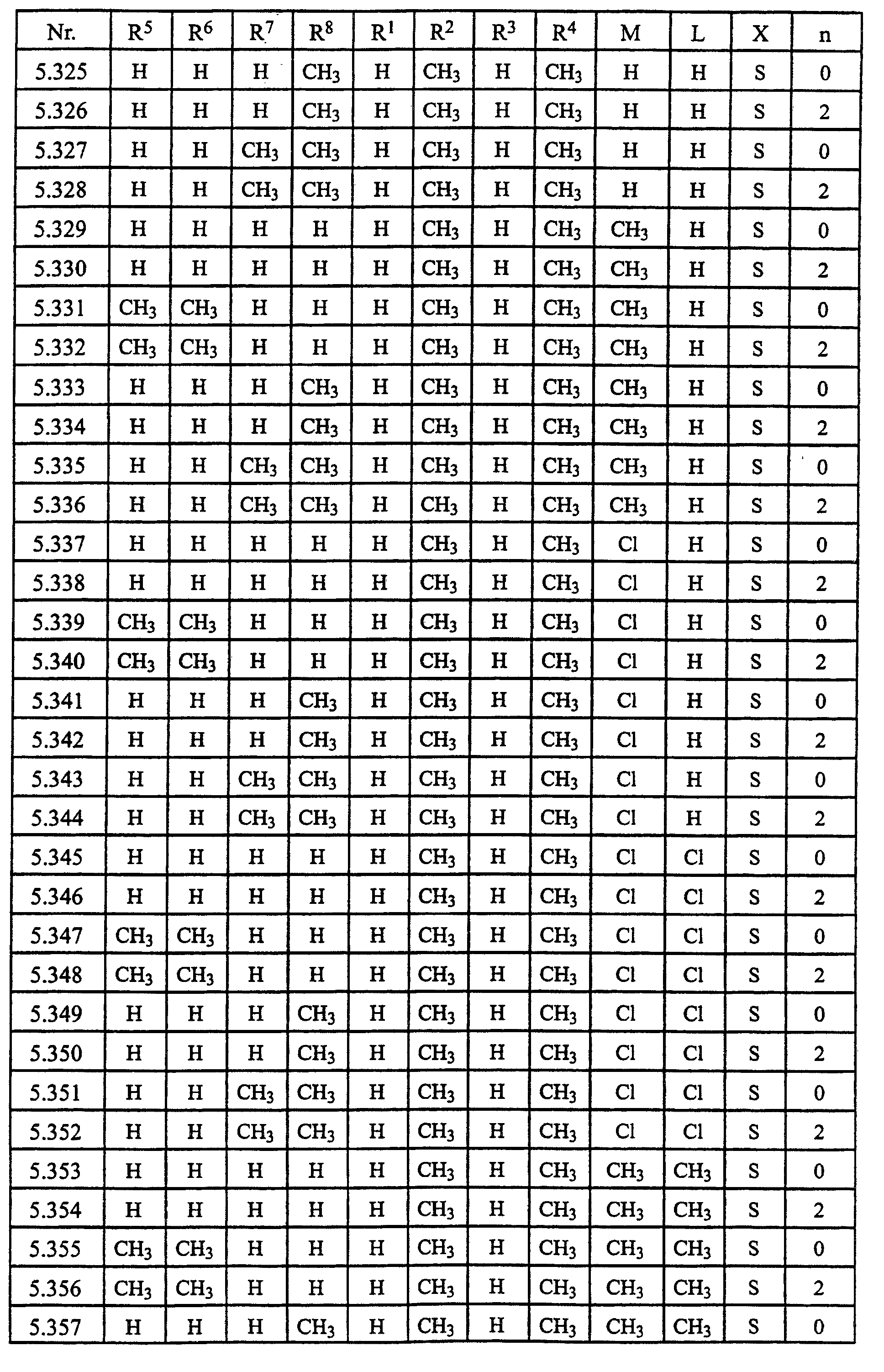

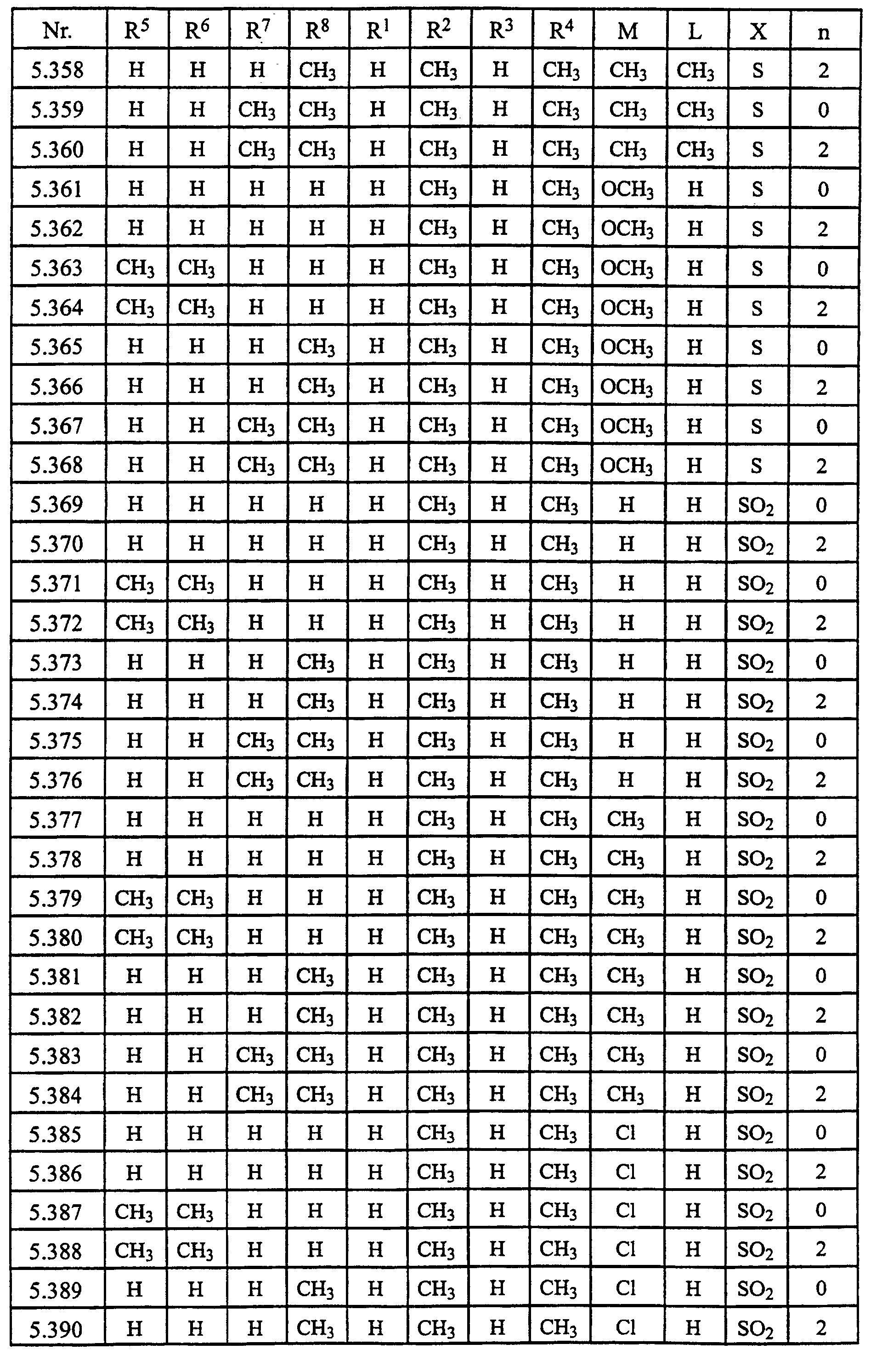

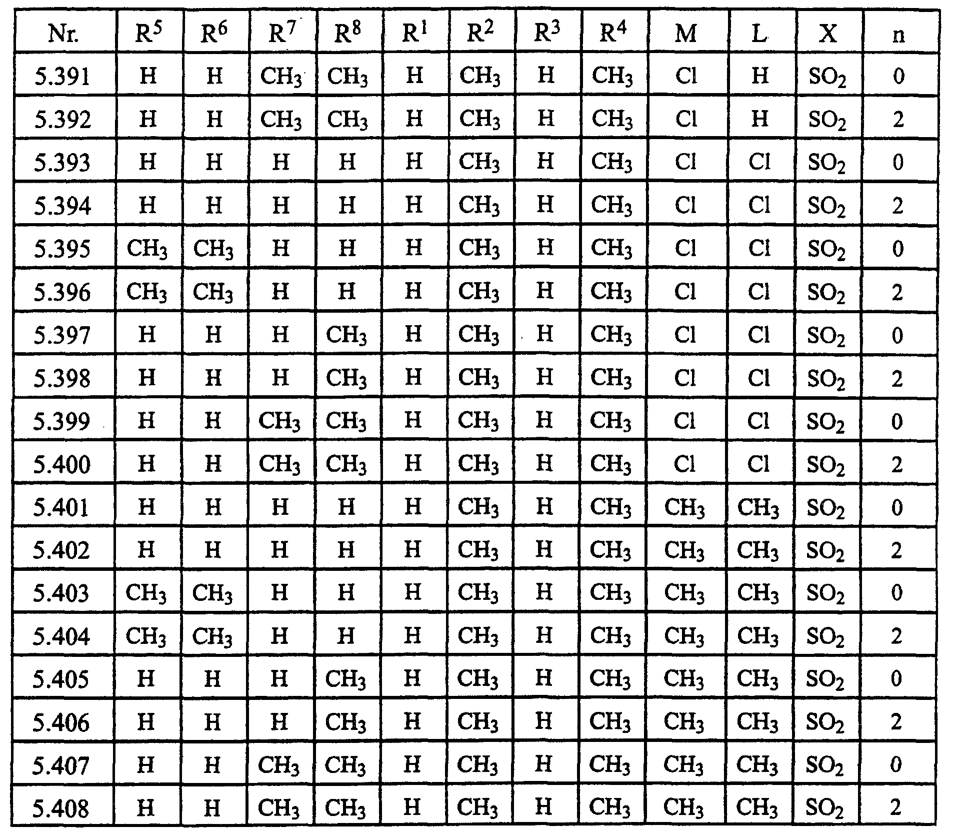

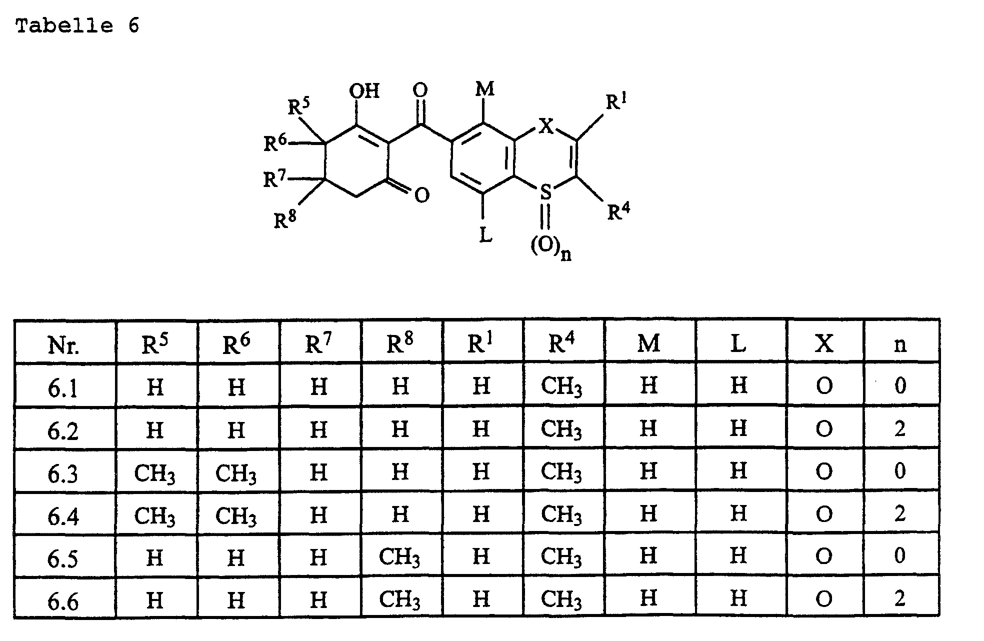

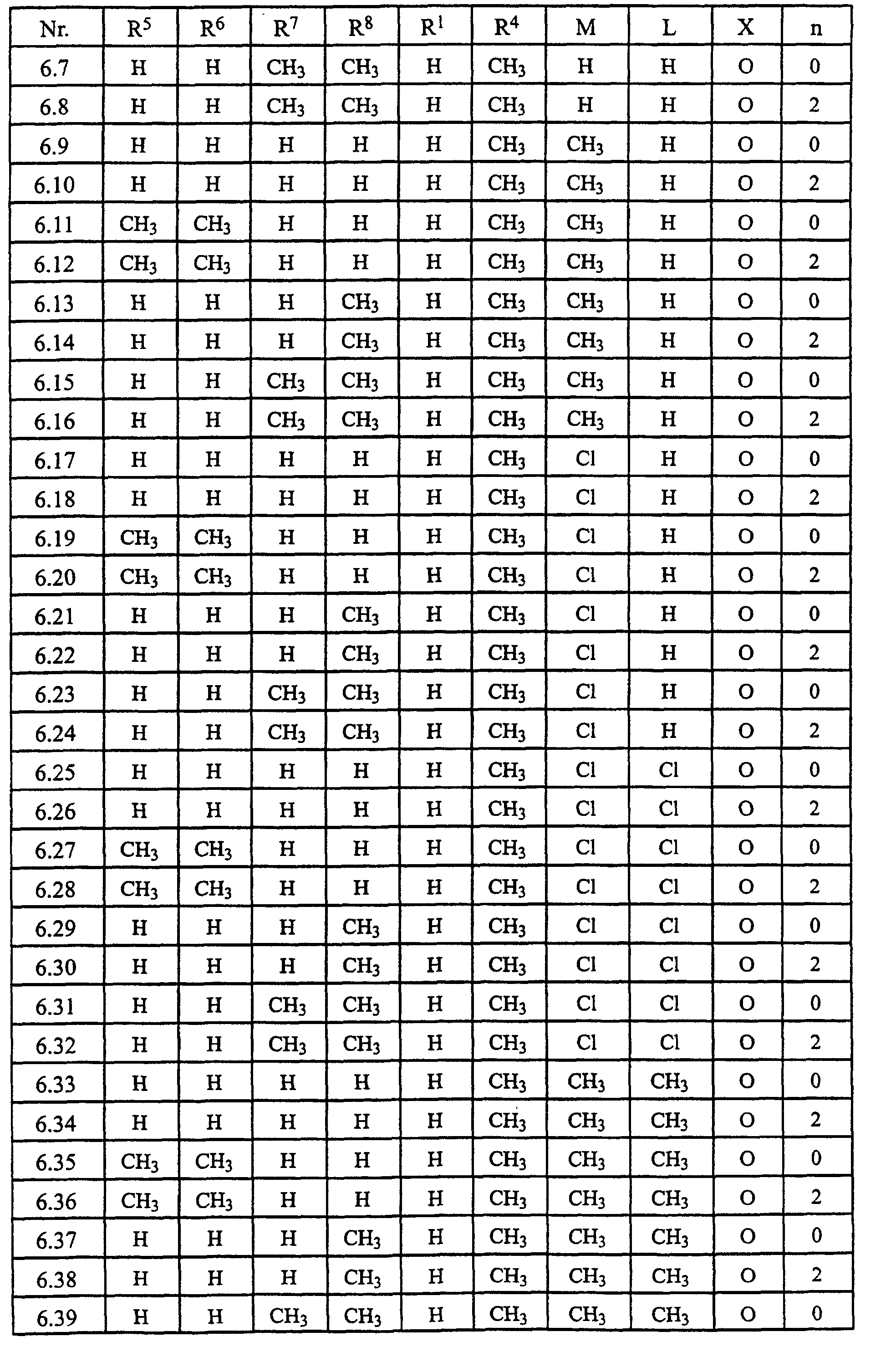

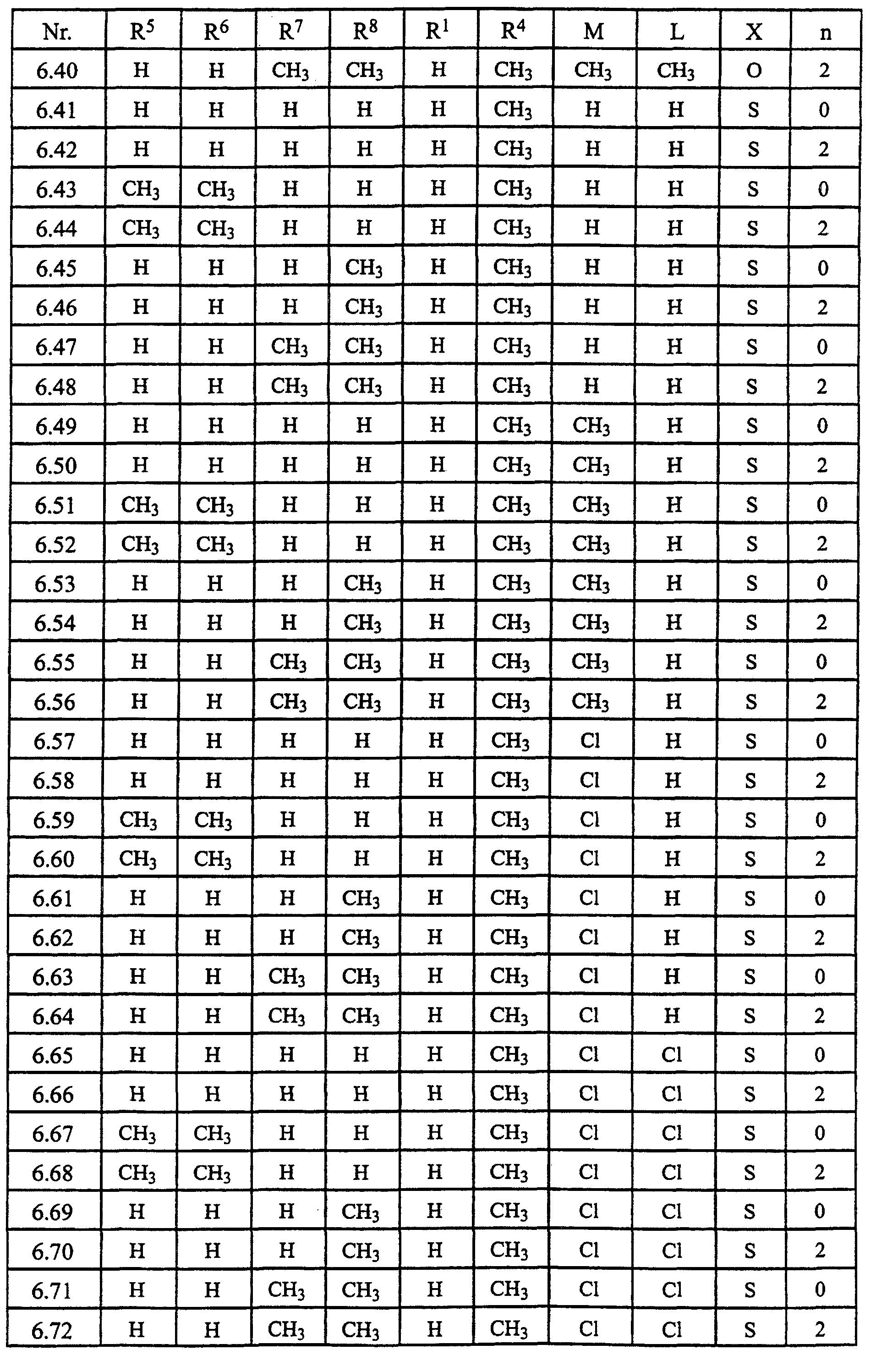

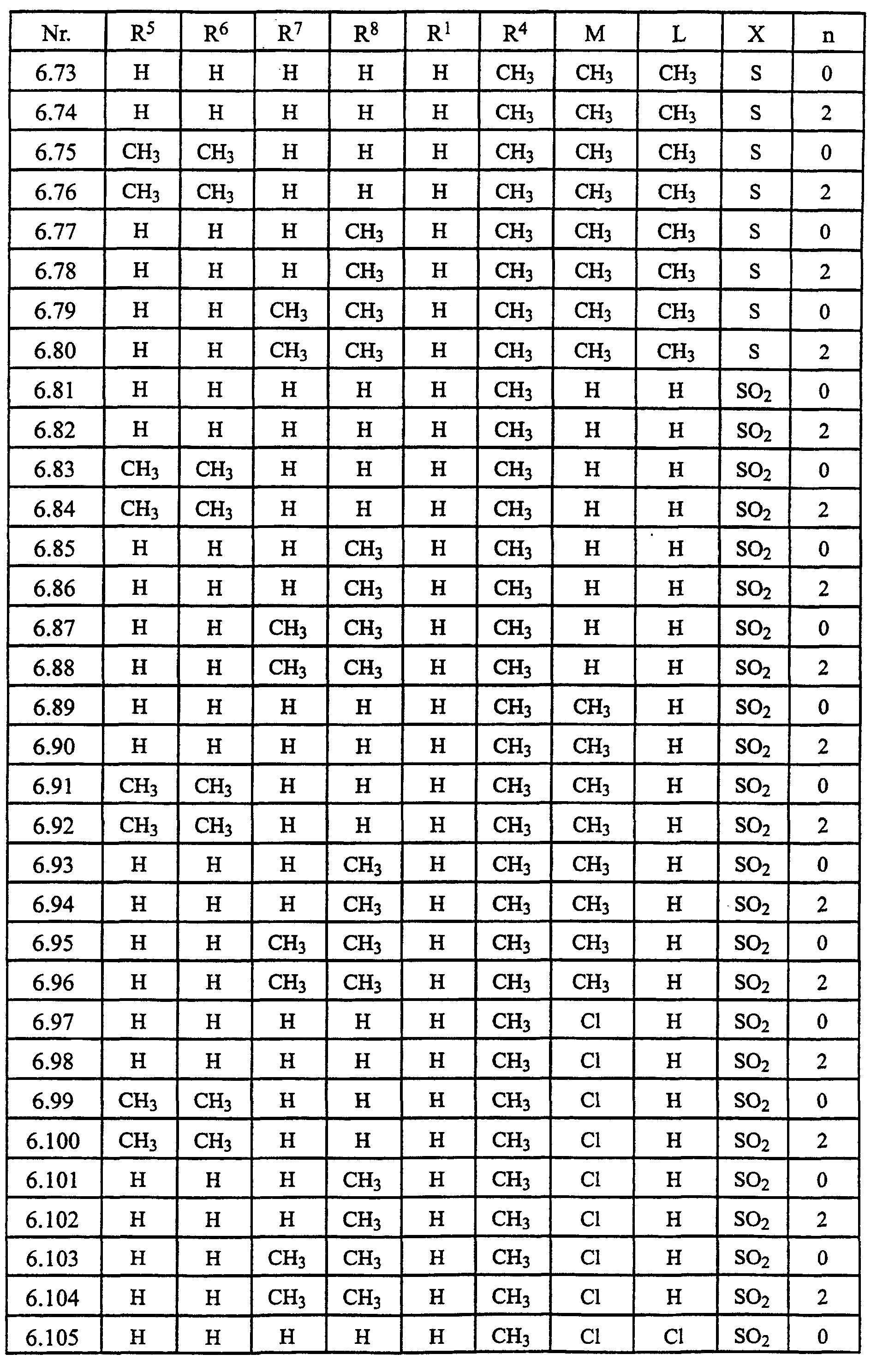

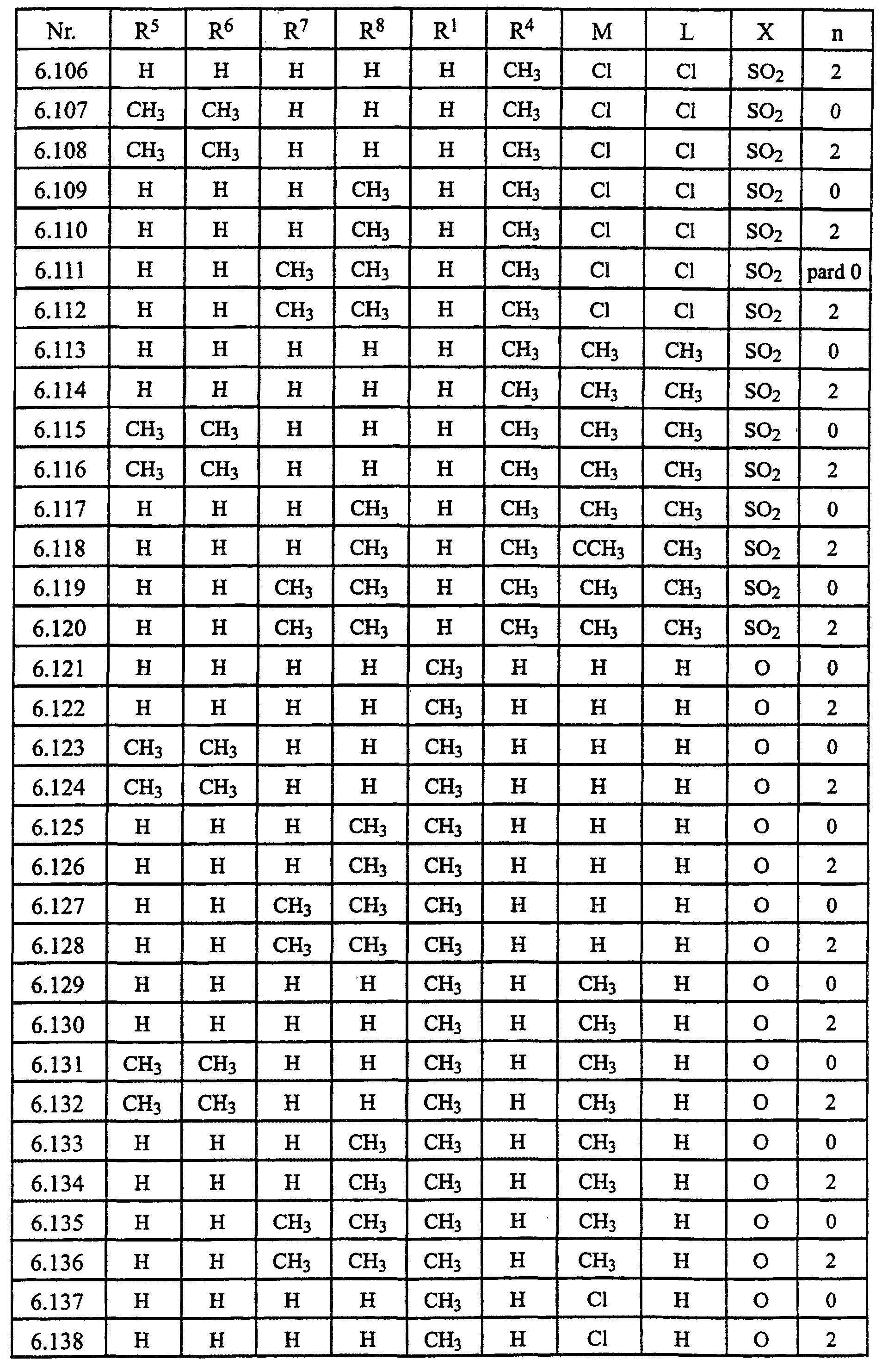

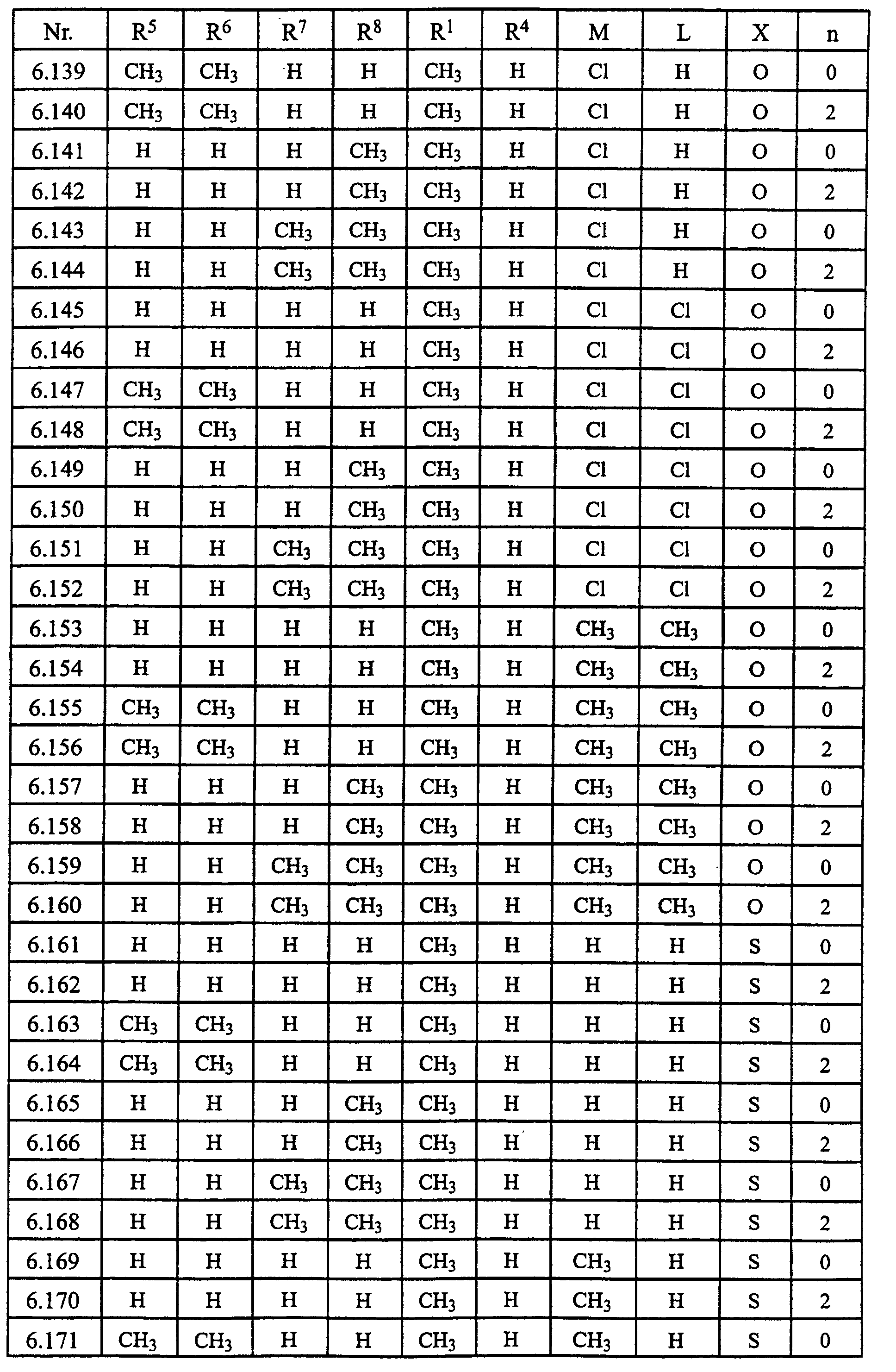

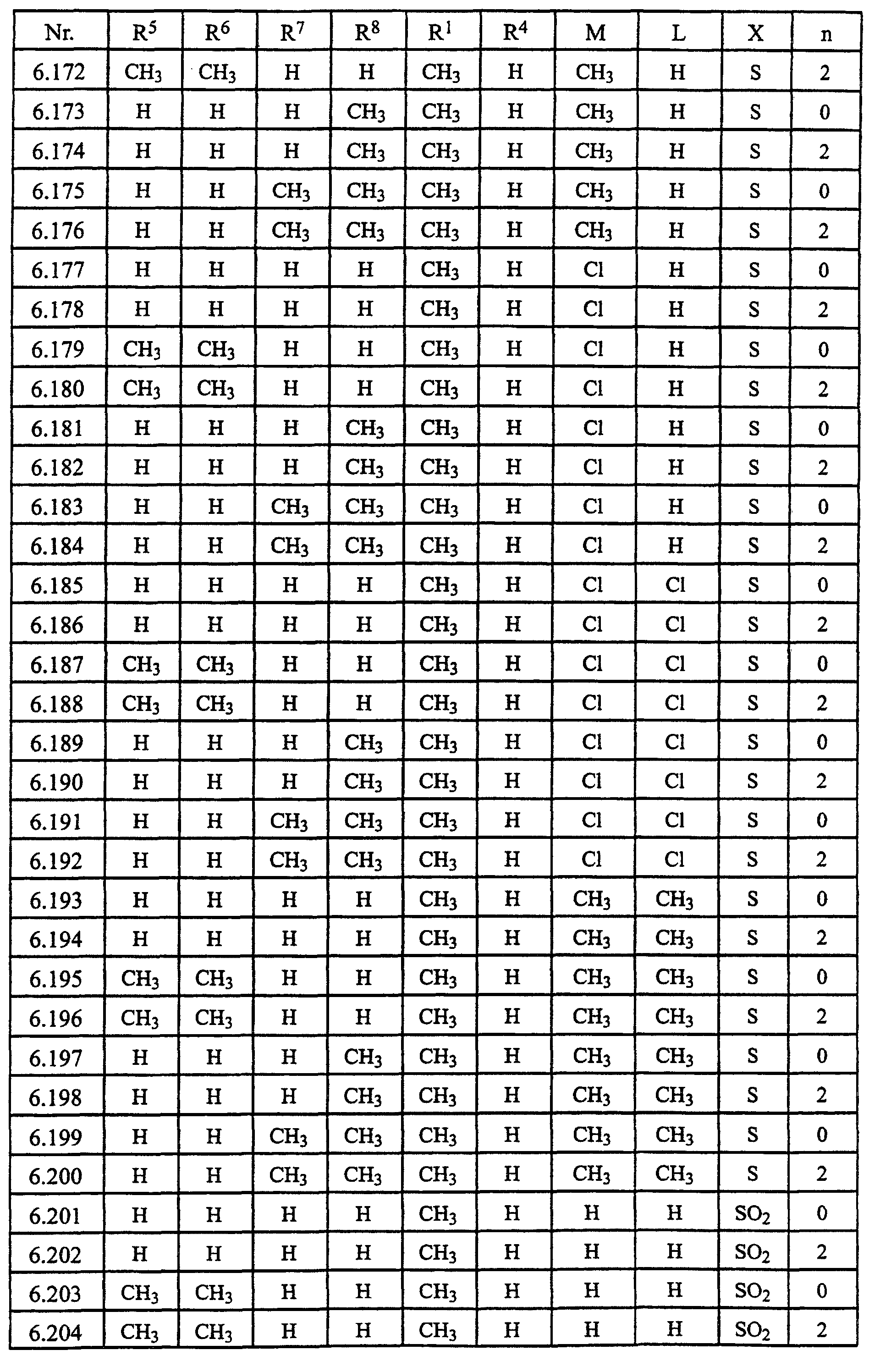

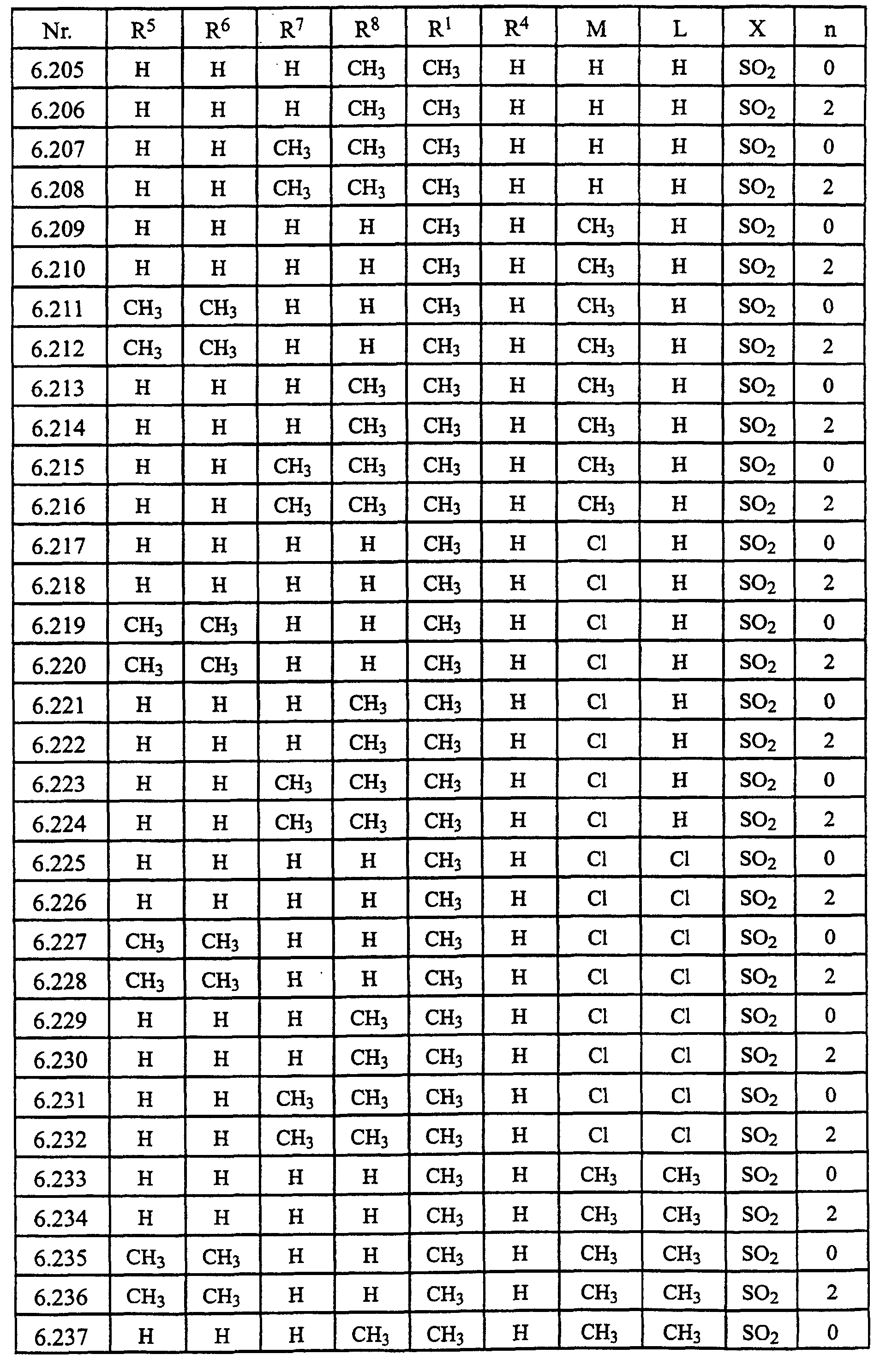

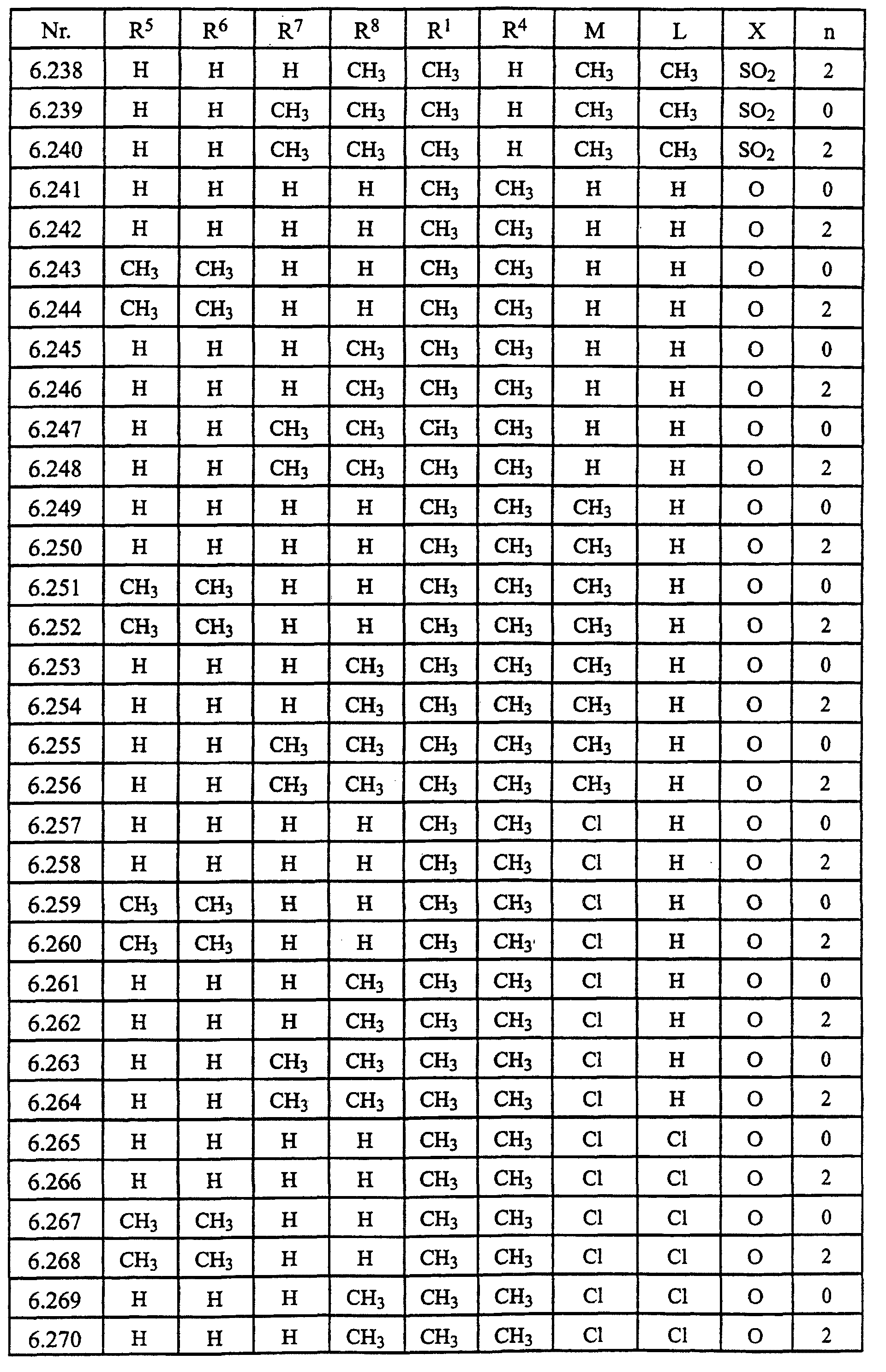

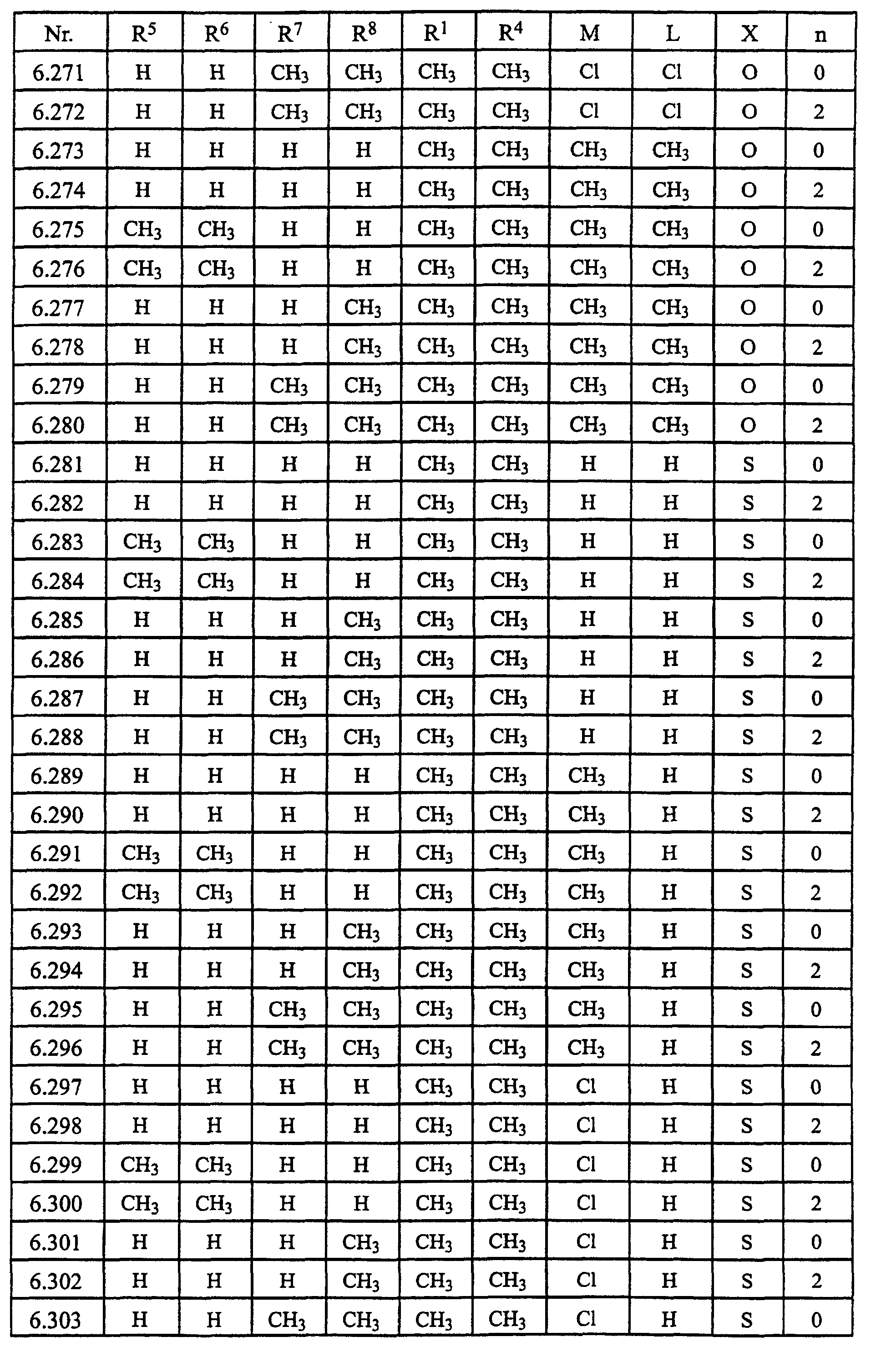

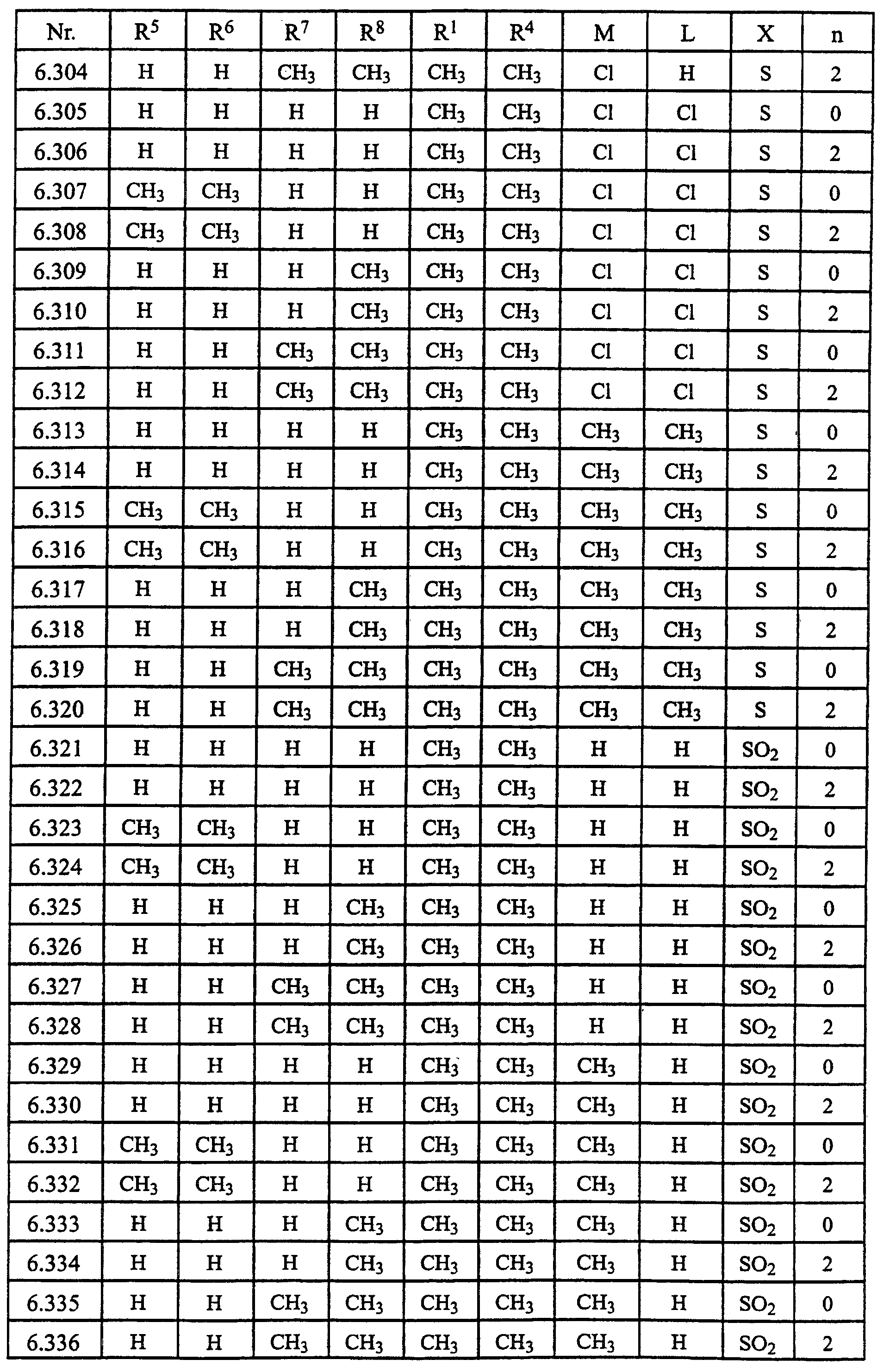

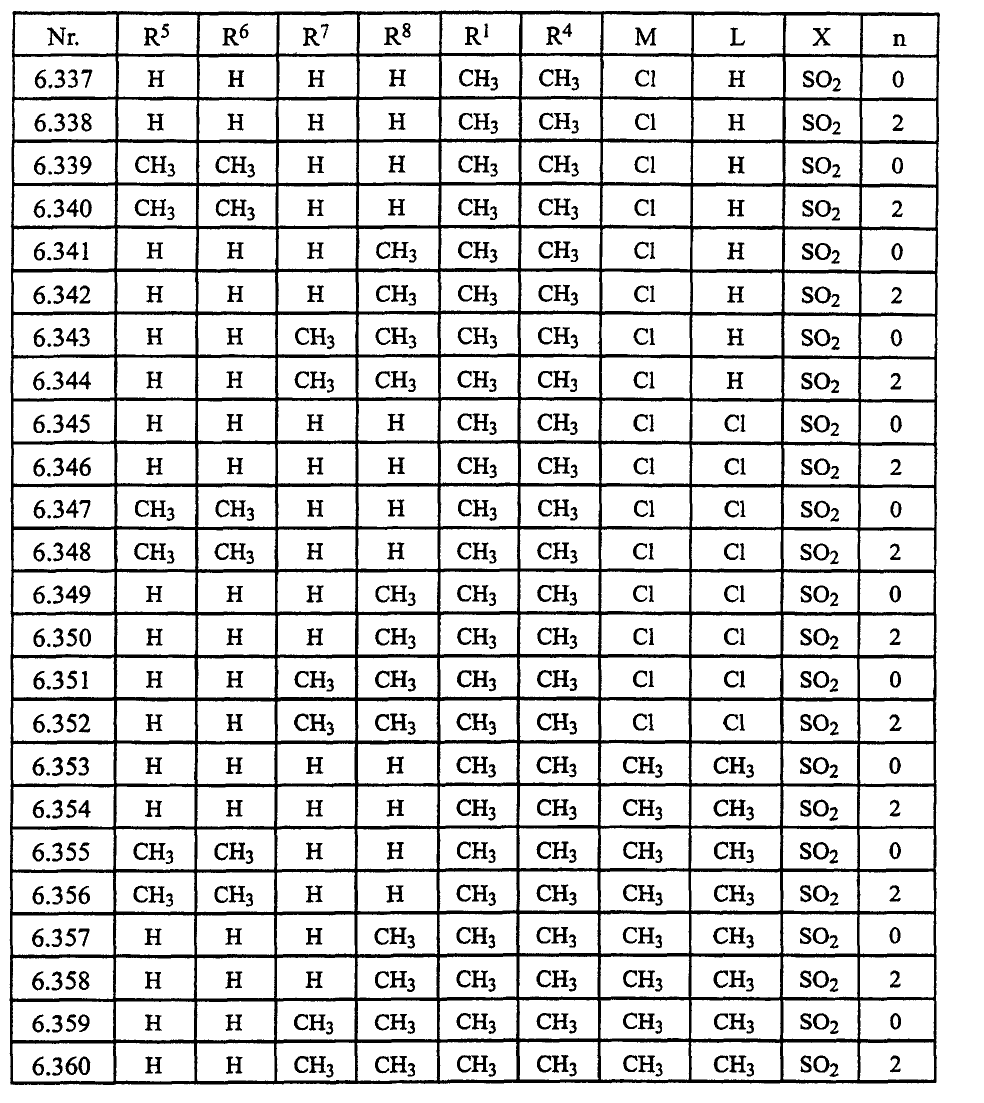

### Herstellungsbeispiele

### A) Herstellungsbeispiele der Ausgangsmaterialien und Zwischenprodukte

### 1. 3-(2-Bromethoxy)-2-methylbenzoesäureethylester

13,6 g (0,2 mol) Natriummethylat werden in 200 ml Ethanol gelöst. Danach werden 36 g (0,2 mol) 3-Hydroxy-2-methylbenzoesäureethylester zugegeben und 2 Stunden unter Rückfluß erhitzt. Anschließend werden 61,4 g (0,32 mol) 1,2-Dibromethan zugetropft und 20 Stunden auf Rückflußtemperatur erwärmt. Die erkaltete Reaktionsmischung wird am Rotationsverdampfer eingeengt. Der Rückstand wird mit Essigsäureethylester aufgenommen und 3 mal mit verdünnter Natronlauge gewaschen. Die organische Phase wird getrocknet und das Lösungsmittel abdestilliert. Das Wertprodukt wird durch Säulenchromatographie gereinigt. Ausbeute: 14,6 g Öl
NMR(270 MHZ; CDCl₃; δ in ppm): 7,4 (d, 1H), 7,2 (tr, 1H), 6,9 (d, 1H), 4,4 (tr, 2H), 4,3 (q, 2H), 3,7 (tr, 2H), 2,4 (s, 3H), 1,5 (tr, 3H)

### 2. 3-(2-Methylsulfonylthioethoxy)-2-methylbenzoesäureethylester

2 g (7 mmol) 3-(2-Bromethoxy)-2-methylbenzoesäureethylester und 1,1 g (7,3 mmol) Kaliumthiomethansulfonat werden in 10 ml abs. Ethanol gelöst. Die Reaktionsmischung wird für 20 Stunden unter Rückfluß erwärmt. Danach wird das Lösungsmittel abdestilliert und der Rückstand in Methylenchlorid aufgenommen und mit Wasser gewaschen. Die organische Phase wird über Natriumsulfat getrocknet und das Solvens entfernt. Das Wertprodukt wird durch Säulenchromatographie gereinigt.
Ausbeute 1.1 g (50 %)
NMR(270 MHZ; CDCl₃; δ in ppm): 7,4 (d, 1H), 7,2 (tr, 1H), 7,0 (d, 1H), 4,5 (tr, 2H), 4,3 (q, 2H), 3,6 (tr, 2H), 3,4 (s, 3H), 2,4 (s, 3H), 1,4 (tr, 3H)

### 3. 8-Methyl-2,3-dihydro-benz-1,4-oxathiin-7-carbonsäureethylester

1.0 g (3,4 mmol) 3-(2-Methylsulfonylthioethoxy)-2-methylbenzoesäureethylester werden in 5 ml Nitromethan gelöst. 0,42 g (3,14 mmol) Aluminiumtrichlorid werden zugegeben. Es wird 45 min bei Raumtemperatur gerührt. Die Aufarbeitung erfolgt durch Zugabe von 10 ml 2N Salzsäure und anschließender Extraktion mit MTB-Ether. Die vereinigten organischen Phasen werden mit Wasser und Natriumcarbonatlösung gewaschen, über Natriumsulfat getrocknet und das Lösungsmittel abdestilliert.
Ausbeute: 0,7 g (93 %)
NMR(270 MHZ; CDCl₃; δ in ppm): 7,5 (d, 1H), 6,9 (d, 1H), 4,5 (tr, 2H), 4,3 (q, 2H), 3,2 (tr, 2H), 2,4 (s, 3H), 1,4 (tr, 3H),

### 4. 8-Methyl-2,3-dihydro-benz-1,4-oxathiin-7-carbonsäure

4,0 g (0,0168 mol) 8-Methyl-2,3-dihydro-benz-1,4-oxathiin-7-carbonsäureethylester werden zusammen mit 1.0 g (0,0252 mol) Natriumhydroxid in 40 ml Methanol/Wasser auf Rückflußtemperatur geheizt. Man rührt 2 Stunden bei der Temperatur und destilliert anschließend das Solvens ab. Der Rückstand wird mit Wasser aufgenommen. Man extrahiert mit Ether und säuert danach die wäßrige Phase mit 2N Salzsäure an. Das Wertprodukt fällt aus, wird abgesaugt und mit wenig Wasser gewaschen. Man trocknet das Produkt im Vakuumtrockenschrank bei 40°C.
Ausbeute: 2,9 g (82 %)
NMR(270 MHZ; d⁶-DMSO; δ in ppm): 12,3 (bs, 1H), 7,3 (d, 1H) 6,9 (d, 1H), 4,4 (tr, 2H), 3,2 (tr, 2H), 2,4 (s, 3H),

### 5. 8-Methyl-2,3-dihydro-4,4-dioxo-benz-1,4-oxythiin-7-carbonsäure

2,8 g (0,013 mol) 8-Methyl-2,3-dihydro-benz-1,4-oxathiin-7-carbonsäure werden zusammen mit einer Spatelspitze Natriumwolframat in 30 ml Essigsäure vorgelegt. Es wird auf 50°C aufgeheizt. 3,3 g (0,029 mol) Wasserstoffperoxid (30 %ig) werden zugetropft. Man hält die Reaktionslösung noch 4 Stunden bei 50-60°C. Die Lösung wird auf Eiswasser gegeben. Der Niederschlag wird abgesaugt, mit Wasser gewaschen und im Vakuumtrockenschrank bei 40°C getrocknet.
Ausbeute: 2,7 g
Schmelzpunkt: 234°C

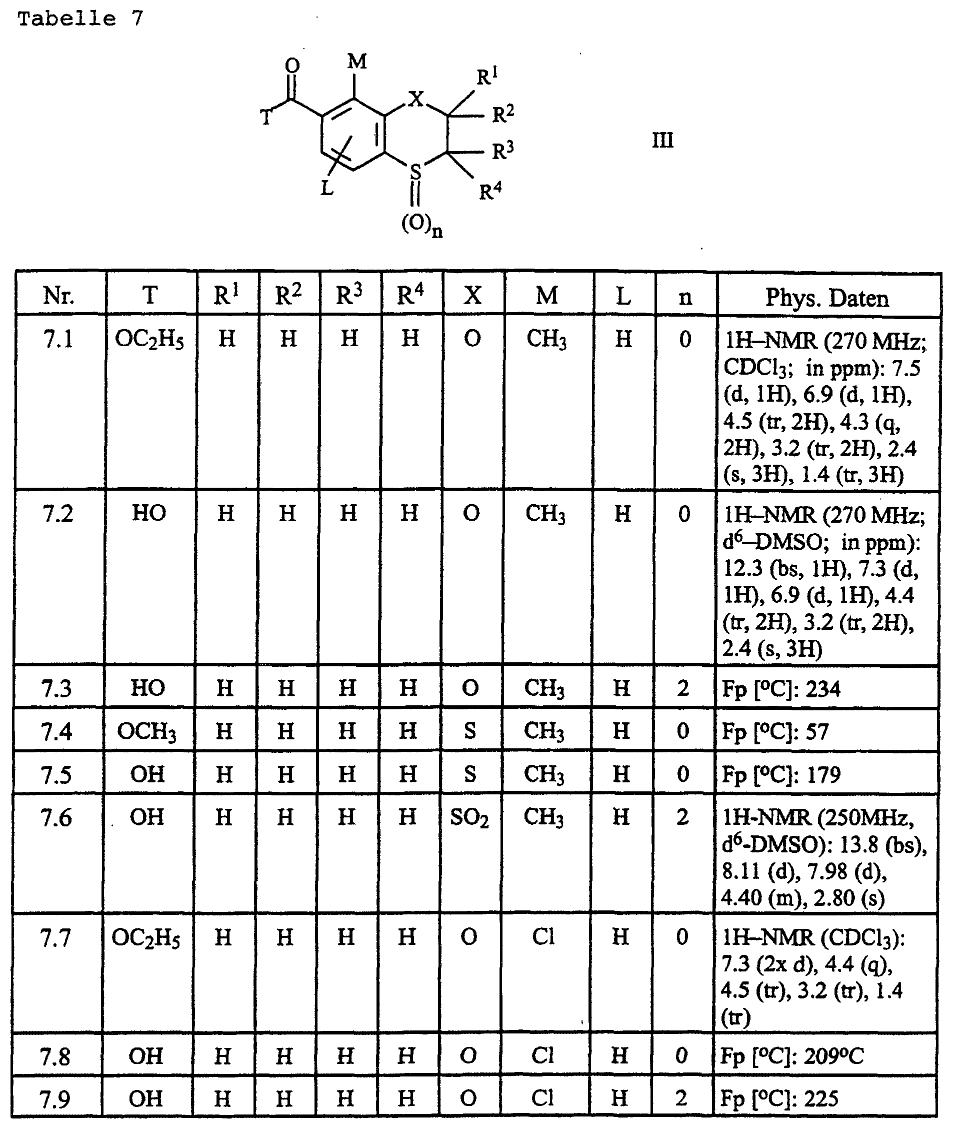

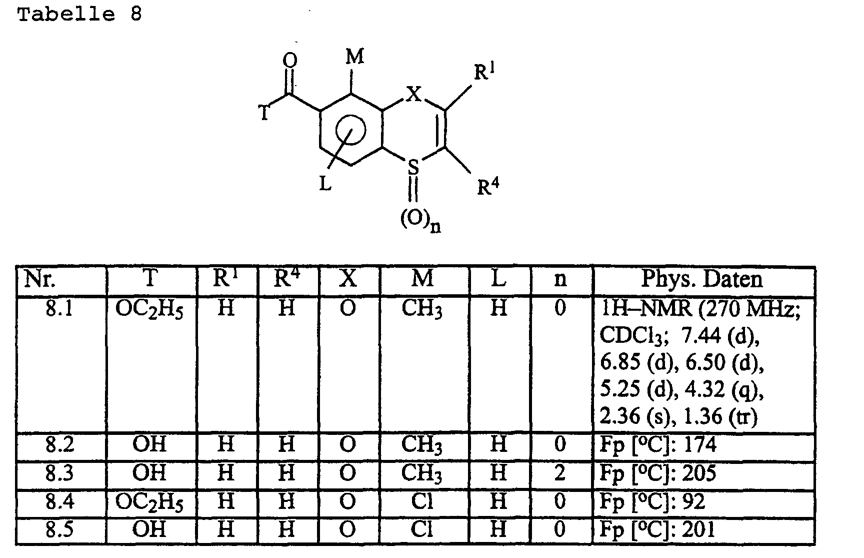

### Herstellung der Endprodukte

### 1. 2-(8-Methyl-2,3-dihydro-4,4-dioxo-benz-1,4-oxathiin-7-carbonyl)-1,3-cyclohexandion

0,9 g (3,72 mmol) 8-Methyl-2,3-dihydro-4,4-dioxobenz-1,4-oxathiin-7-carbonsäure und 0,42 g (3,72 mmol) 1,3-Cyclohexadion werden in 20 ml Acetonitril gelöst. Anschließend gibt man 0,81 g (3,9 mmol) DCC (Dicyclohexylcarbodiimid) zu und rührt mehrere Stunden bis zur vollständigen Umsetzung. Danach werden 0,75 g (7,44 mmol) Triethylamin und 0,2 ml Trimethylsilylcyanid zugetropft. Es wird 3 Stunden bei RT gerührt. Zur Aufarbeitung wird die Reaktionsmischung in 100 ml 2 %ige Natriumcarbonatlösung gegossen. Der Niederschlag wird abgesaugt und die wäßrige Phase mit Essigsäureethylester gewaschen. Die wäßrige Phase wird dann mit 2N Salzsäure angesäuert. Das Wertprodukt fällt aus. Es wird abgesaugt, mit Wasser gewaschen und im Vakuumtrockenschrank getrocknet.
Ausbeute: 0,6 g
Schmelzpunkt: 201°C

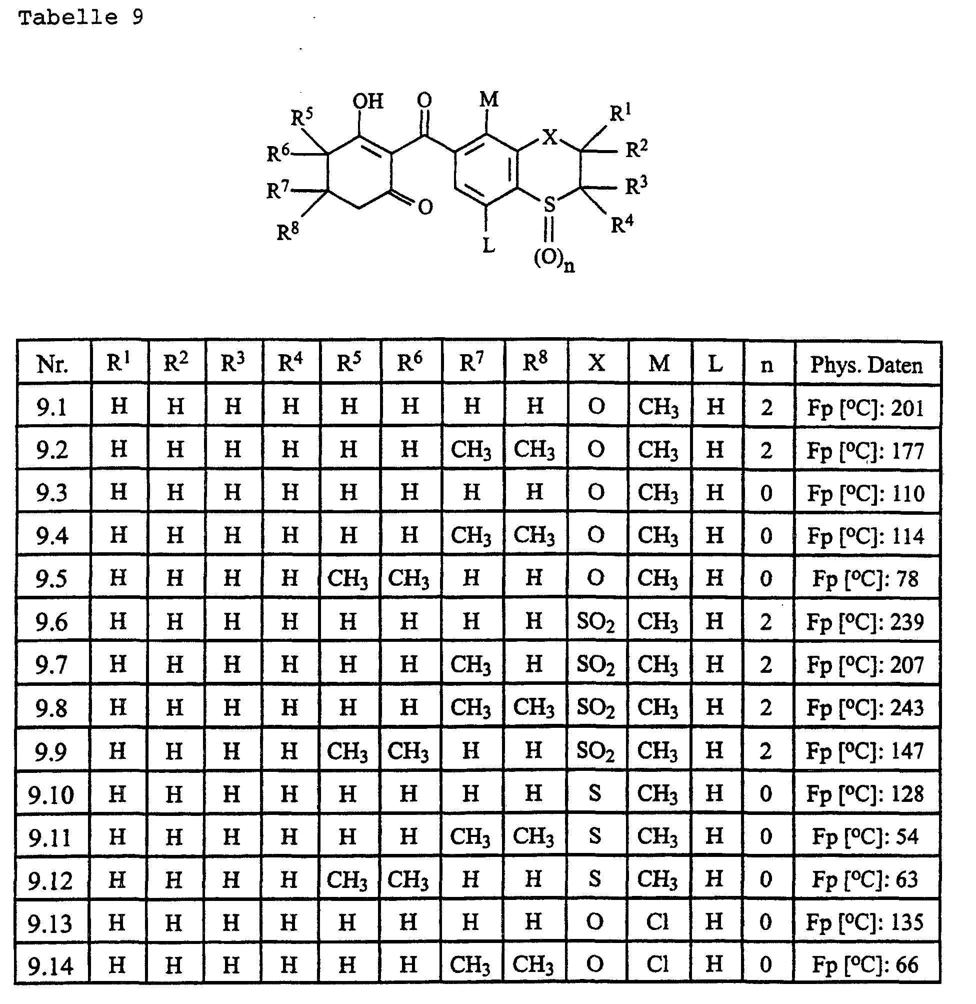

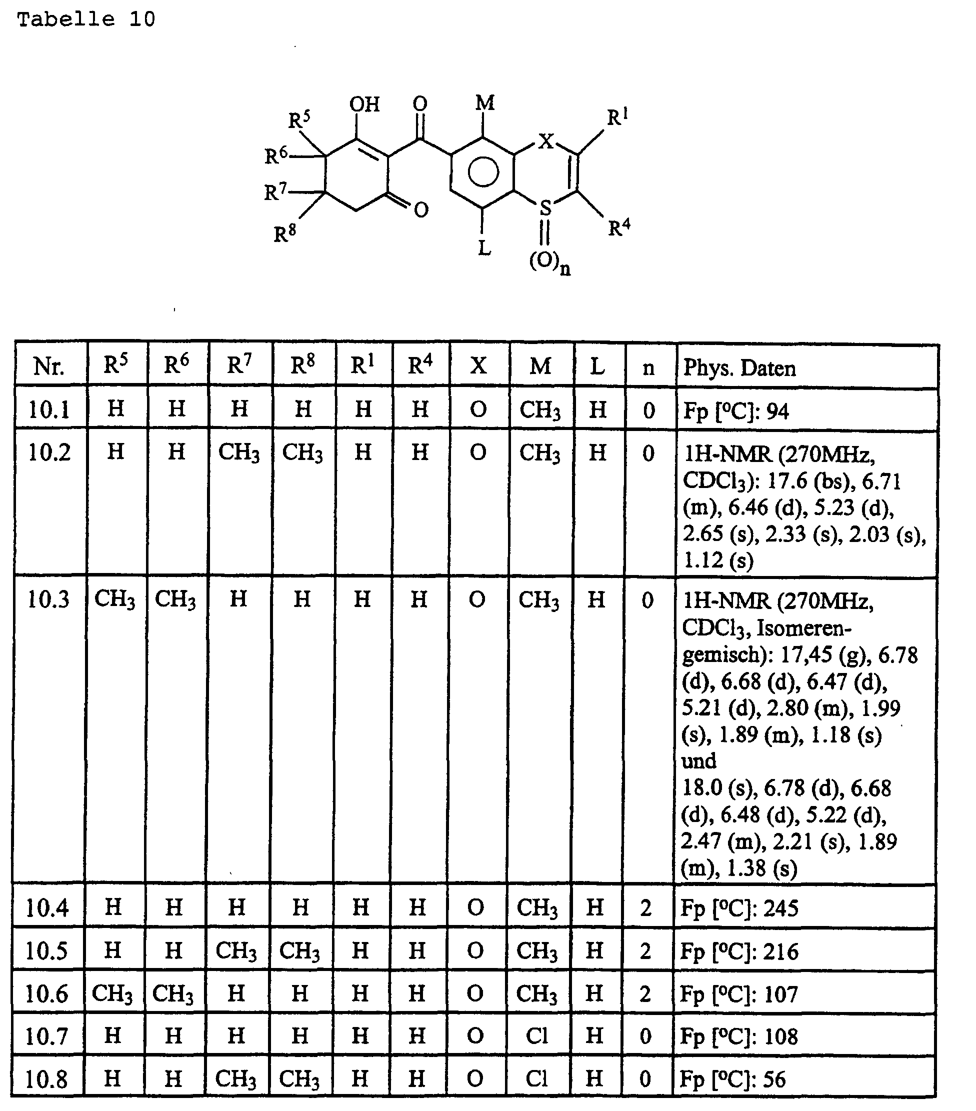

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spp. altissima, Beta vulagris spp. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spp., Manihot esculenta, Medicago sativa, Musa spp., Nicotiana tabacum (N.rustica), Olea europaea, Oryza sativa, Phaseolus lunatus, Phaseolus vulgaris, Picea abies, Pinus spp., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.- %, Wirkstoff. Die Wirkstoffe werden dabei in einer Reinheit von 90 % bis 100 %, vorzugsweise 95 % bis 100 % (nach NMR-Sektrum) eingesetzt.

Die erfindungsgemäßen Verbindungen I können beispielsweise wie folgt formuliert werden:
I. 20 Gewichtsteile der Verbindung Nr. 9.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II. 20 Gewichtsteile der Verbindung Nr. 9.1 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 9.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 9.1 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 9.1 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 9.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
VII. 1 Gewichtsteil der Verbindung 9.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung 9.1 wird in einer Mischung gelöst, die aus 80 Gewichtsteilen Cyclohexanon und 20 Gewichtsteilen Emulphor EL (ethoxyliertes Rizinusöl/casteroil) besteht. Man erhält ein stabiles Emulsionskonzentrat.

Zur Verbreiterung des Wirkungsspektrums und zur Erzielung synergistischer Effekte können die 2-Hetaroylcyclohexan-1,3-dione I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 2-Hetaroylcyclohexan-1,3-dione der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurdenleicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufbehandlung betrug 0,0625 bzw. 0,0313 kg/ha a. S.

Zum Zweck der Nachauflaufbehandlung werden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen werden dafür entweder direkt gesät und in den gleichen Gefäßen aufgezogen oder sie werden erst als Keimpflanzen getrennt angezogen und einige Tage vor der Behandlung in die Versuchsgefäße verpflanzt.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englicher Name |
|---|---|---|
| Chenopodium album | Weißer Gänsefuß | lambsquarters |
| (CHEAL) | | (gossefoot) |
| Echinochloa crus-galli | Hühnerhirse | barnyardgrass |
| (ECHCG) | | |
| Solanum nigrum | Schwarzer Nacht- | black nightshade |
| (SOLNI) | schatten | |
| Triticum aestivum | Winterweizen | winter wheat |
| (TRZAW) | | |
| Zea mays | Mais | Indian corn |
| (ZEAMX) | | |

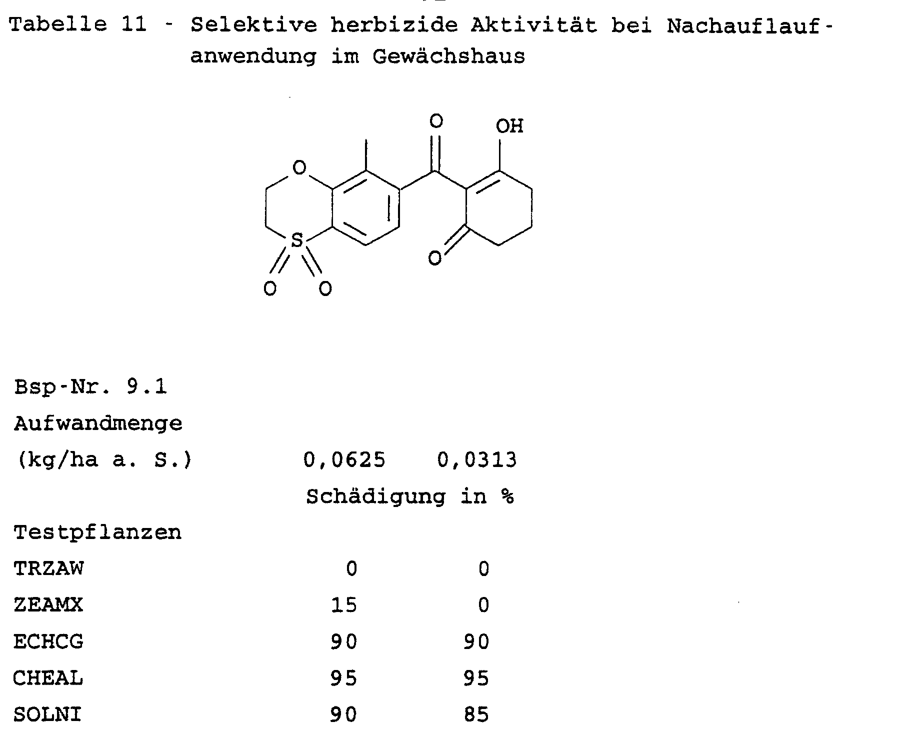

## Patentansprüche

1. Hetaroylderivate der Formel I 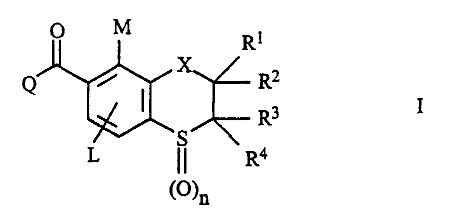 in der die Substituenten folgende Bedeutung haben:
L,M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
X Sauerstoff oder Schwefel, der mit einem oder zwei Sauerstoffen substituiert sein kann;
n null, eins, zwei;
R¹ Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl;
R² Wasserstoff, C₁-C₆-Alkyl, C₂-C₆-Alkenyl. C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl; R² und R³ können eine Bindung bilden;
R³ Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl; R³ und R² können eine Bindung bilden;
R⁴ Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen;
Phenyl, das einfach oder mehrfach durch folgende Gruppen substituiert sein kann: C₁-C₄-Alkyl, Wasserstoff, C₁-C₄-Alkoxy, C₁-C₄-Haloalkyl, C₁-C₄-Haloalkoxy, Halogen, Nitro, Cyano, C₁-C₄-Alkyloxycarbonyl;
Q ein in 2-Stellung verknüpfter Cyclohexan-1,3-dionring der Formel II 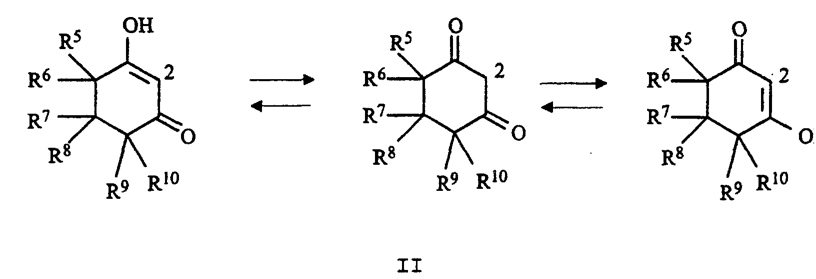
in welcher die Substituenten folgende Bedeutung haben:
R⁵, R⁶ und R¹⁰ Wasserstoff, C₁-C₄-Alkyl,
R⁷ Wasserstoff, C₁-C₄-Alkyl, C₃-C₄-Cycloalkyl, wobei diese Gruppen gegebenenfalls einen bis drei der folgenden Substituenten tragen können: Halogen, C₁-C₄-Thioalkyl oder C₁-C₄-Alkoxy; oder
R⁷ C₁-C₄-Alkoxy; C₁-C₆-Alkoxyalkyl, Tetrahydropyranyl-3, Tetrahydropyranyl-4; oder
R⁷ und R⁹ können gemeinsam eine Bindung oder einen drei bis sechsgliedrigen carbocyclischen Ring bilden;
R⁸ Wasserstoff, C₁-C₄-Alkyl;
R⁹ Wasserstoff, C₁-C₄-Alkyl oder eine Gruppe COOR¹¹;
R¹¹ C₁-C₄-Alkyl;
sowie landwirtschaftlich brauchbare Salze.

2. Hetaroylderivate der Formel Ia 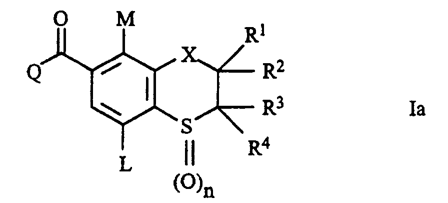 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, X, R¹ bis R⁴ und n die in Anspruch 1 angegebenen Bedeutungen haben.

3. Hetaroylderivate der Formel Ib 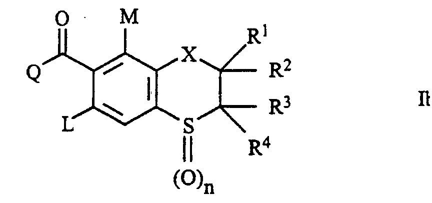 in der L für C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, X, R¹ bis R⁴ und n die in Anspruch 1 angegebenen Bedeutungen haben.

4. Hetaroylderivate der Formel I gemäß Anspruch 1 in der die Reste L und M für Wasserstoff, Methyl, Methoxy, Chlor, Cyano, Nitro und Trifluormethyl stehen.

5. Hetaroylderivate der Formel Ic 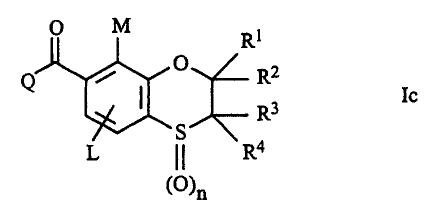 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, R¹ bis R⁴ und n die in Anspruch 1 angegebenen Bedeutungen haben.

6. Hetaroylderivate der Formel Id 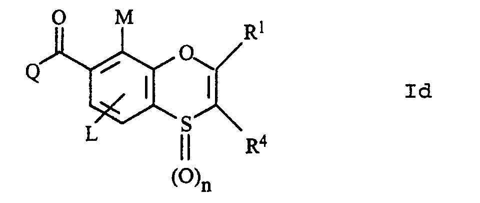 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro steht und Q, R¹, R⁴ und n die in Anspruch 1 angegebenen Bedeutungen haben.

7. Hetaroylderivate der Formel Ie 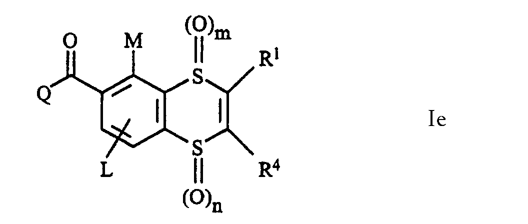 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro sowie m für null, eins oder zwei steht und Q, R¹, R⁴ und n die in Anspruch 1 angegebenen Bedeutungen haben.

8. Hetaroylderivate der Formel If 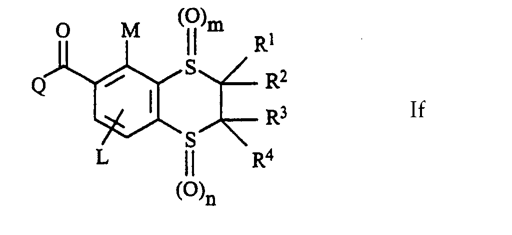 in der L für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro und M für Wasserstoff, C₁-C₆-Alkyl, C₂-C₆-Alkenyl, C₂-C₆-Alkinyl, C₁-C₄-Alkoxy, C₁-C₄-Halogenalkyl, C₁-C₄-Halogenalkoxy, Halogen, Cyano oder Nitro sowie m für null, eins oder zwei steht und Q, R¹ bis R⁴ und n die in Anspruch 1 angegebenen Bedeutungen haben.

9. Verfahren zur Herstellung der Verbindung der Formel I gemäß Anspruch 1 **dadurch gekennzeichnet, daß** man die jeweiligen Ausgangsstoffe der Formel II 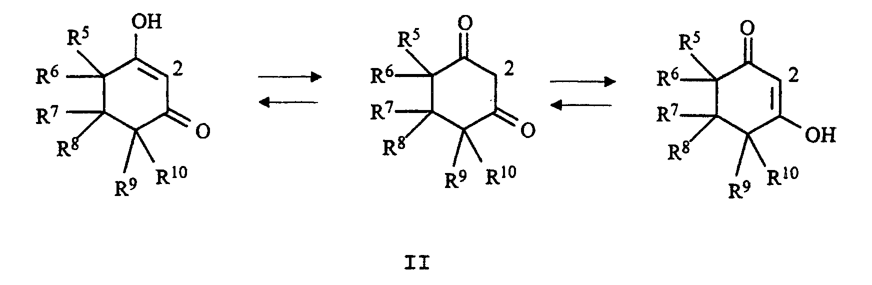 mit einem Säurechlorid der Formel IIIa oder einer Säure der Formel IIIb 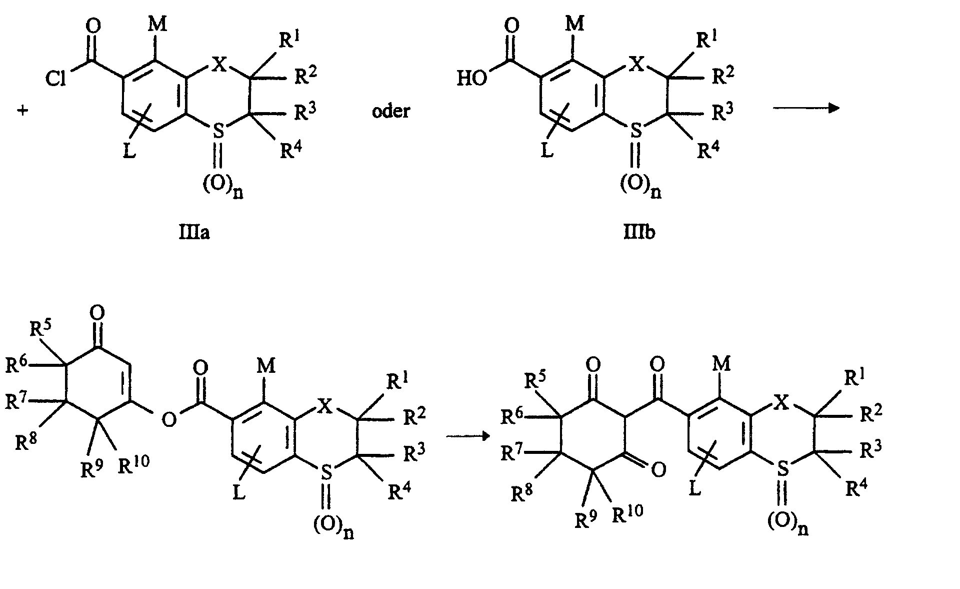 wobei L, M, X, R¹ bis R⁴ und n die in Anspruch 1 genannte Bedeutung haben, acyliert und das Acylierungsprodukt in Gegenwart eines Katalysators zu den Verbindungen I umlagert.

10. Hetaroylderivate der Formel IIIc 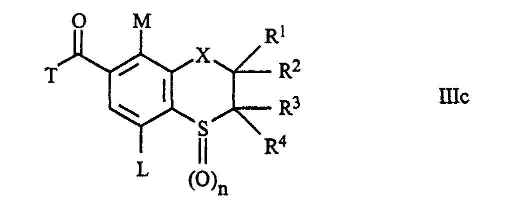 in der T, L, M, X, R¹ bis R⁴ und n die folgende Bedeutung haben:
T Chlor, OH oder C₁-C₄-Alkoxy;
L Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
X, R¹, R², R³, R⁴ und n wie in Anspruch 1 angegeben.

11. Hetaroylderivate der Formel IIId 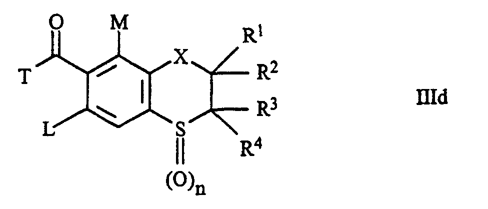 in der T, L, M, X, R¹ bis R⁴ und n die folgende Bedeutung haben:
T Chlor, OH oder C₁-C₄-Alkoxy;
L C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
M Wasserstoff, C₁-C₆-Alkyl, C₂-C₆-Alkenyl, C₂-C₆-Alkinyl, C₁-C₄-Alkoxy, wobei diese Gruppen gegebenenfalls durch ein bis fünf Halogenatome oder C₁-C₄-Alkoxy substituiert sein können; Halogen, Cyano, Nitro;
X, R¹, R², R³, R⁴ und n wie in Anspruch 1 angegeben.

12. Herbizides Mittel, enthaltend mindestens ein Hetaroylderivat der Formel I gemäß Anspruch 1 und Zusatzstoffe.

13. Verfahren zur Bekämpfung unerwünschten Pflanzenwuchses, **dadurch gekennzeichnet, daß** man eine herbizid wirksame Menge eines Hetaroylderivates der Formel I gemäß Anspruch 1 auf die Pflanzen oder deren Lebensraum einwirken läßt.

## Claims

1. A hetaroyl derivative of the formula I 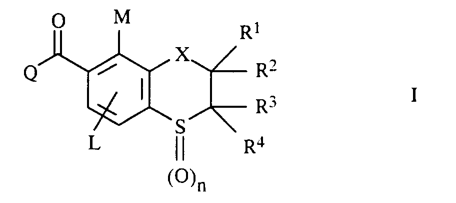 where the substituents have the following meanings:
L and M hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro;
X oxygen or sulfur which can be substituted by one or two oxygens;
n zero, one, two;
R¹ hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen;
phenyl which can be mono- or polysubstituted by the following groups: C₁-C₄-alkyl, hydrogen, C₁-C₄-alkoxy, C₁-C₄-haloalkyl, C₁-C₄-haloalkoxy, halogen, nitro, cyano, C₁-C₄-alkyloxycarbonyl;
R² hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen;
phenyl which can be mono- or polysubstituted by the following groups: C₁-C₄-alkyl, hydrogen, C₁-C₄-alkoxy, C₁-C₄-haloalkyl, C₁-C₄-haloalkoxy, halogen, nitro, cyano, C₁-C₄-alkyloxycarbonyl; R² and R³ can form a bond;
R³ hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen;
phenyl which can be mono- or polysubstituted by the following groups: C₁-C₄-alkyl, hydrogen, C₁-C₄-alkoxy, C₁-C₄-haloalkyl, C₁-C₄-haloalkoxy, halogen, nitro, cyano, C₁-C₄-alkyloxycarbonyl; R³ and R² can form a bond;
R⁴ hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen;
phenyl which can be mono- or polysubstituted by the following groups: C₁-C₄-alkyl, hydrogen, C₁-C₄-alkoxy, C₁-C₄-haloalkyl, C₁-C₄-haloalkoxy, halogen, nitro, cyano, C₁-C₄-alkyloxycarbonyl;
Q a cyclohexane-1,3-dione ring, linked in the 2-position, of the formula II 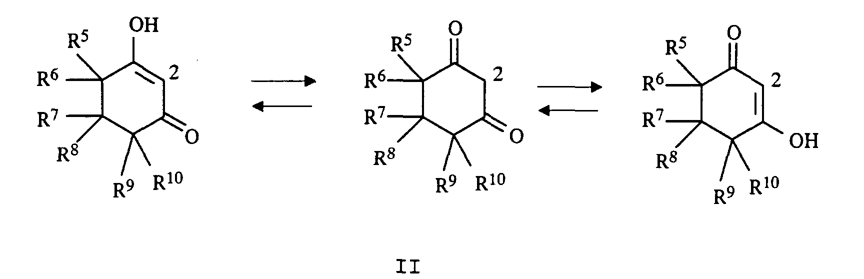
where the substituents have the following meanings:
R⁵, R⁶ and R¹⁰ hydrogen, C₁-C₄-alkyl;
R⁷ hydrogen, C₁-C₄-alkyl, C₃-C₄-cycloalkyl, it being possible for these groups, if desired, to carry one to three of the following substituents: halogen, C₁-C₄-thioalkyl or C₁-C₄-alkoxy; or
R⁷ C₁-C₄-alkoxy; C₁-C₆-alkoxyalkyl, tetrahydropyran-3-yl, tetrahydropyran-4-yl; or
R⁷ and R⁹ can together form a bond or a three- to six-membered carbocyclic ring;
R⁸ hydrogen, C₁-C₄-alkyl;
R⁹ hydrogen, C₁-C₄-alkyl or a group COOR¹¹;
R¹¹ C₁-C₄-alkyl;
or agriculturally utilizable salts.

2. A hetaroyl derivative of the formula Ia 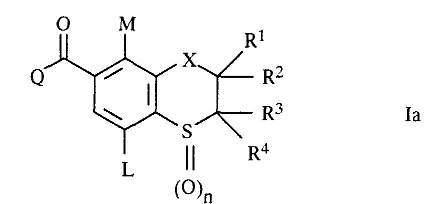 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and Q, X, R¹ to R⁴ and n have the meanings given in claim 1.

3. A hetaroyl derivative of the formula Ib 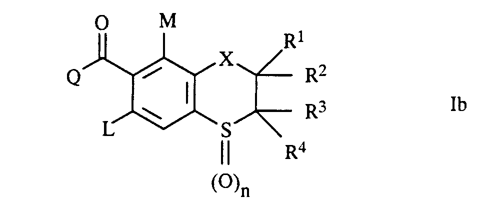 where L is C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and Q, X, R¹ to R⁴ and n have the meanings given in claim 1.

4. A hetaroyl derivative of the formula I as claimed in claim 1 where the radicals L and M are hydrogen, methyl, methoxy, chlorine, cyano, nitro and trifluoromethyl.

5. A hetaroyl derivative of the formula Ic 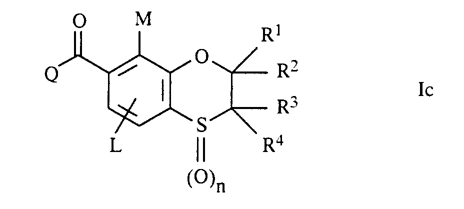 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and Q, R¹, R⁴ and n have the meanings given in claim 1.

6. A hetaroyl derivative of the formula Id 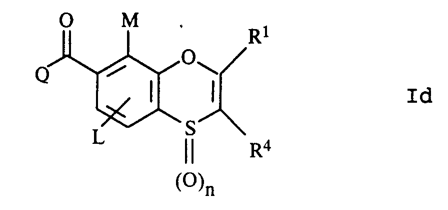 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and Q, R¹, R⁴ and n have the meanings given in claim 1.

7. A hetaroyl derivative of the formula Ie 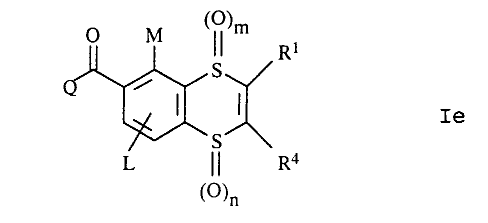 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro, and m is zero, one or two and Q, R¹, R⁴ and n have the meanings given in claim 1.

8. A hetaroyl derivative of the formula If 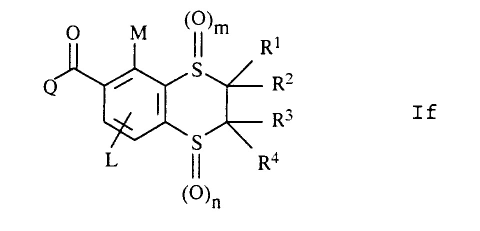 where L is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro and M is hydrogen, C₁-C₆-alkyl, C₂-C₆-alkenyl, C₂-C₆-alkynyl, C₁-C₄-alkoxy, C₁-C₄-haloalkyl, C₁-C₄-haloalkoxy, halogen, cyano or nitro, and m is zero, one or two and Q, R¹ to R⁴ and n have the meanings given in claim 1.

9. A process for preparing the compound of the formula I as claimed in claim 1, which comprises acylating the respective starting substances of the formula II 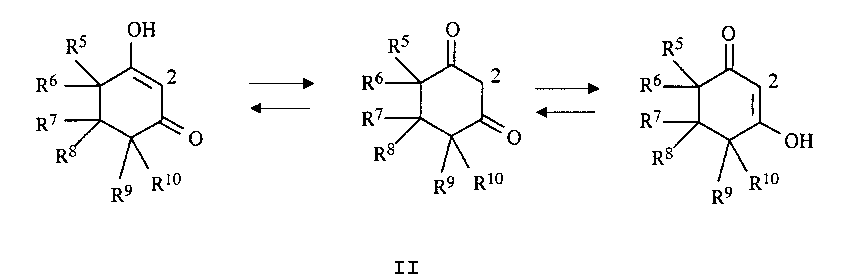 using an acid chloride of the formula IIIa or an acid of the formula IIIb 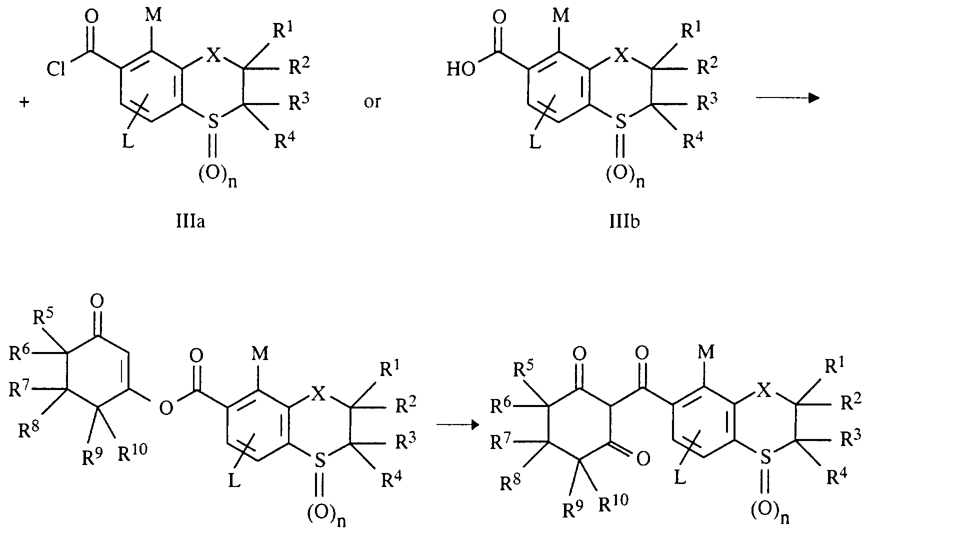 where L, M, X, R¹ to R⁴ and n have the meanings mentioned in claim 1, and rearranging the acylation product to the compounds I in the presence of a catalyst.

10. A hetaroyl derivative of the formula IIIc 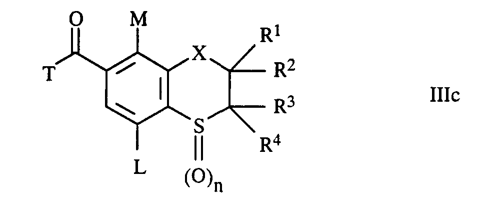 where T, L, M, X, R¹ to R⁴ and n have the following meanings:
T chlorine, OH or C₁-C₄-alkoxy;
L hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro;
M hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro;
X, R¹, R², R³, R⁴ and n are as given in claim 1.

11. A hetaroyl derivative of the formula IIId 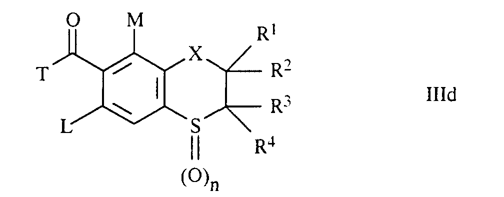 where T, L, M, X, R¹ to R⁴ and n have the following meanings:
T chlorine, OH or C₁-C₄-alkoxy;
L C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro;
M hydrogen, C₁-C₆-alkyl, C₂-C₆-alkenyl, C₂-C₆-alkynyl, C₁-C₄-alkoxy, it being possible for these groups to be unsubstituted or substituted by one to five halogen atoms or C₁-C₄-alkoxy; halogen, cyano, nitro;
X, R¹, R², R³, R⁴ and n are as given in claim 1.

12. A herbicidal composition, comprising at least one hetaroyl derivative of the formula I as claimed in claim 1 and additives.

13. A method of controlling undesired plant growth, which comprises allowing a herbicidally active amount of a hetaroyl derivative of the formula I as claimed in claim 1 to act on the plants or their habitat.

## Revendications

1. Dérivés hétéroaroylique de formule I 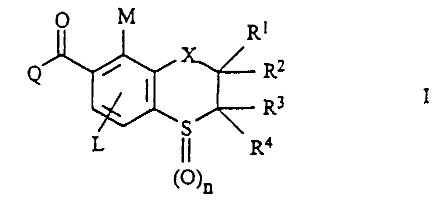 dans laquelle les symboles ont les significations suivantes :
L, M : l'hydrogène, un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène, un groupe cyano, nitro ;
X : l'oxygène ou le soufre, qui peut être substitué par un ou deux atomes d'hydrogène ;
n : 0, 1, 2;
R¹ : l'hydrogène, un groupe alkyle en C1-C6, alcényle en C2-C6, alcynyle en C2-C6, alcoxy en C1-C4, ces groupes pouvant éventuellement être substitués par un à cinq atomes d'halogènes ou groupes alcoxy en C1-C4 ; un halogène ;
un groupe phényle qui peut être substitué une ou plusieurs fois par les groupes suivants :
alkyle en C1-C4, hydrogène, alcoxy en C1-C4, halogénoalkyle en C1-C4, halogénoalcoxy en C1-C4, halogène, nitro, cyano, (alcoxy en C1-C4)carbonyle ;
R² : l'hydrogène, un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène ;
un groupe phényle qui peut être substitué une ou plusieurs fois par les groupes suivants :
alkyle en C1-C4, l'hydrogène, alcoxy en C1-C4, halogénoalkyle en C1-C4, halogénoalcoxy en C1-C4, halogène, nitro, cyano, (alcoxy en C1-C4)carbonyle ;
R² et R³ peuvent également former une liaison ;
R³ : l'hydrogène, un groupe alkyle en C1-C6, alcényle en C2-C6, alcynyle en C2-C6, alcoxy en C1-C4, ces groupes pouvant éventuellement être substitués par un à cinq atomes d'halogènes ou groupes alcoxy en C1-C4 ; un halogène ;
un groupe phényle qui peut être substitué une ou plusieurs fois par les groupes suivants :
alkyle en C1-C4, hydrogène, alcoxy en C1-C4, halogénoalkyle en C1-C4, halogénoalcoxy en C1-C4, halogène, nitro, cyano, (alcoxy en C1-C4)carbonyle ; R³ et R² peuvent également former une liaison ;
R⁴ : l'hydrogène, un groupe alkyle en C1-C6, alcényle en C2-C6, alcynyle en C2-C6, alcoxy en C1-C4, ces groupes pouvant éventuellement être substitués par un à cinq atomes d'halogènes ou groupes alcoxy en C1-C4 ; un halogène ;
un groupe phényle qui peut être substitué une ou plusieurs fois par les groupes suivants :
alkyle en C1-C4, l'hydrogène, alcoxy en C1-C4, halogénoalkyle en C1-C4, halogénoalcoxy en C1-C4, halogène, nitro, cyano, (alcoxy en Cl-C4)carbonyle ;
Q : un cycle cyclohexane-1,3-dione de formule II relié en position 2 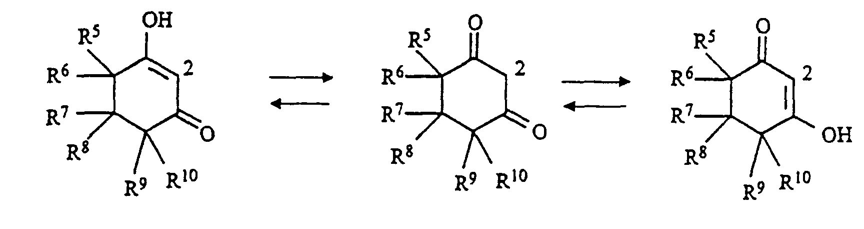 dans laquelle les symboles ont les significations suivantes :
R⁵, R⁶ et R¹⁰ : l'hydrogène, des groupes alkyle en C1-C4,
R⁷ : l'hydrogène, un groupe alkyle en C1-C4, cycloalkyle en C3-C4, ces groupes pouvant le cas échéant porter un à trois des substituants suivants : halogéno, thioalkyle en C1-C4 ou alcoxy en C1-C4 ; ou bien
R⁷ : un groupe alcoxy en C1-C4 ; (alcoxy en C1-C6)alkyle, tétrahydropyranyle-3, tétrahydropyranyle-4 ; ou bien
R⁷ et R⁹ peuvent former ensemble une liaison ou un cycle homogène de trois à six chaînons ;
R⁸ : l'hydrogène, un groupe alkyle en C1-C4 ;
R⁹ : l'hydrogène, un groupe alkyle en C1-C4 ou COOR¹¹ ;
R¹¹ : un groupe alkyle en C1-C4 ;
et leurs sels aptes aux applications agricoles.

2. Dérivés hétéroaroyliques de formule Ia 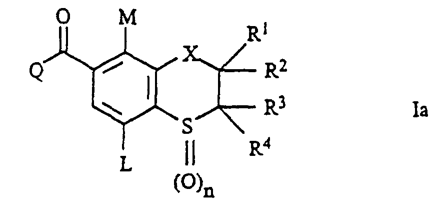 dans laquelle L représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et Q, X, R¹ à R⁴ et n ont les significations indiquées dans la revendication 1.

3. Dérivés hétéroaroyliques de formule Ib 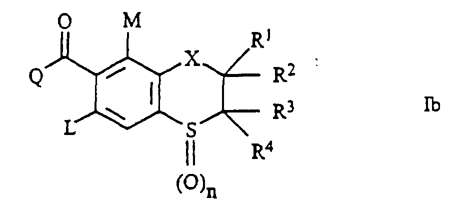 dans laquelle L représente un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et Q, X, R¹ à R⁴ et n ont les significations indiquées dans la revendication 1.

4. Dérivés hétéroaroyliques de formule I de la revendication 1 dans laquelle les symboles L et M représentent l'hydrogène, des groupes méthyle, méthoxy, le chlore, des groupes cyano, nitro ou trifluorométhyle.

5. Dérivés hétéroaroyliques de formule Ic 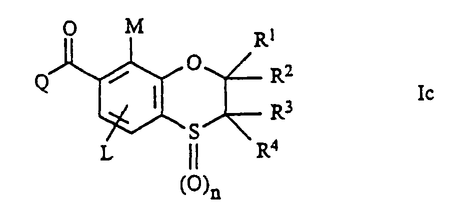 dans laquelle L représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et Q, R¹ à R⁴ et n ont les significations indiquées dans la revendication 1.

6. Dérivés hétéroaroyliques de formule Id 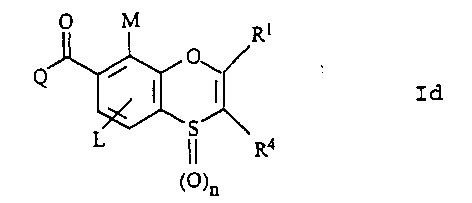 dans laquelle L représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et Q, R¹, R⁴ et n ont les significations indiquées dans la revendication 1.

7. Dérivés hétéroaroyliques de formule Ie 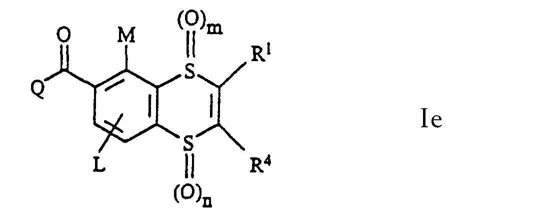 dans laquelle L représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro, m est égal à 0, 1 ou 2 et Q, R¹, R⁴ et n ont les significations indiquées dans la revendication 1.

8. Dérivés hétéroaroyliques de formule If 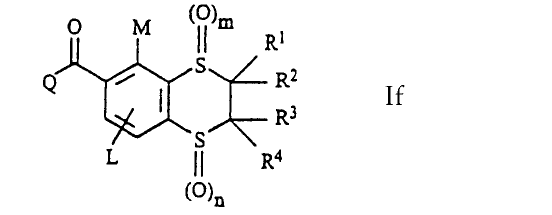 dans laquelle L représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro et M représente l'hydrogène, un groupe alkyle en C1-C6, alcényle en C2-C6, alcynyle en C2-C6, alcoxy en C1-C4, halogénoalkyle en C1-C4, halogénoalcoxy en C1-C4, un halogène, un groupe cyano ou nitro, m est égal à 0, 1 ou 2 et Q, R¹ à R⁴ et n ont les significations indiquées dans la revendication 1.

9. Procédé de préparation des composés de formule I de la revendication 1, **caractérisé par le fait que** l'on acyle l'un des composants de départ de formule II 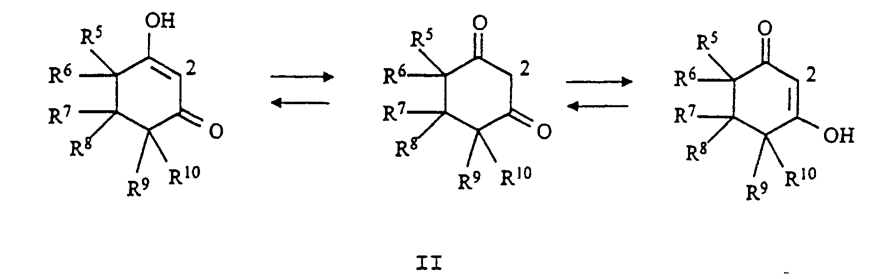 à l'aide d'un chlorure d'acide de formule IIIa ou d'un acide de formule IIIb 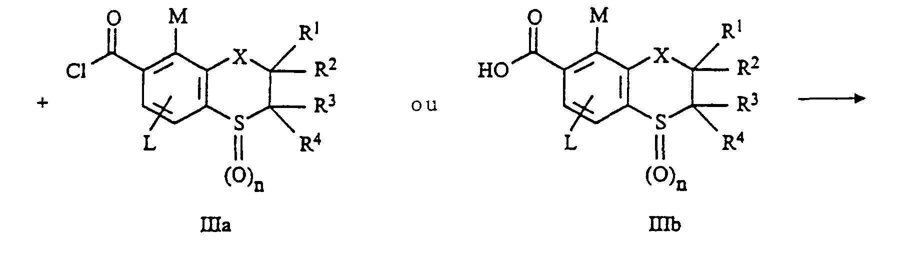 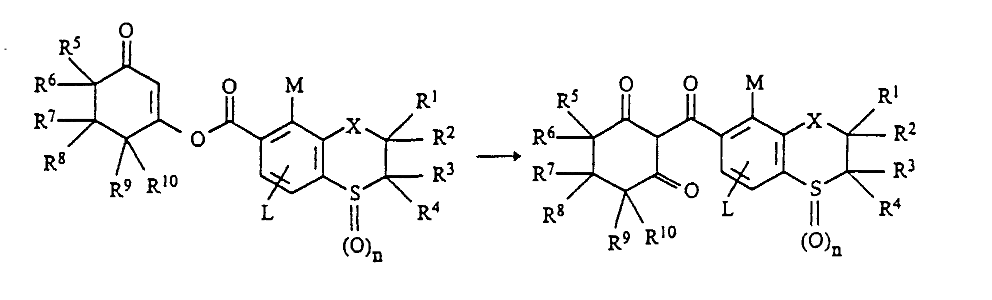 dans lesquelles L, M, X, R¹ à R⁴ et n ont les significations indiquées dans la revendication 1, et l'on soumet le produit d'acylation à transposition en les composés I en présence d'un catalyseur.

10. Dérivés hétéroaroyliques de formule IIIc 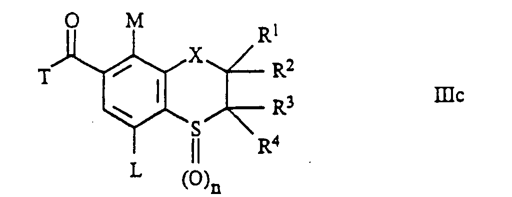 dans laquelle T, L, M, X, R¹ à R⁴ et n ont les significations suivantes :
T : le chlore, un groupe OH ou alcoxy en C1-C4 ;
L : l'hydrogène, un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène, un groupe cyano, nitro ;
M : l'hydrogène, un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène, un groupe cyano, nitro ;
X, R¹, R², R³, R⁴ et n ont les significations indiquées dans la revendication 1.

11. Dérivés hétéroaroyliques de formule IIId 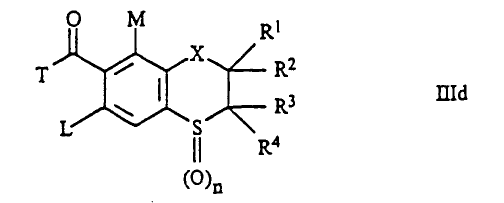 dans laquelle T, L, M, X, R¹ à R⁴ et n ont les significations suivantes :
T : le chlore, un groupe OH ou alcoxy en C1-C4 ;
L : un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène, un groupe cyano, nitro ;
M : l'hydrogène, un groupe alkyle en C1-C6, alcényle en C2-C6, alcynyle en C2-C6, alcoxy en C1-C4, ces groupes pouvant le cas échéant être substitués par un à cinq atomes d'halogènes ou groupes alcoxy en C1-C4 ; un halogène, un groupe cyano, nitro ;
X, R¹, R², R³, R⁴ et n ont les significations indiquées dans la revendication 1.

12. Produit herbicide contenant au moins un dérivé hétéroaroyliques de formule I de la revendication 1 et des additifs.

13. Procédé pour combattre les croissances de végétaux indésirables, **caractérisé par le fait que** l'on fait agir une quantité herbicide efficace d'un dérivé hétéroaroylique de formule I de la revendication 1 sur les végétaux ou leur habitat.
